# EUROPEAN PATENT SPECIFICATION

(11) **EP 4 629 891 B1**
(45) Date of publication and mention of the grant of the patent: **28.10.2026**
(21) Application number: 23837903.6
(22) Date of filing: 05.12.2023
(51) Int. Cl.: A61B 5/1455, A61B 5/28, A61B 5/25, A61B 5/024, A61B 5/00, A61B 5/256

(54) **WEARABLE DEVICE WITH PHYSIOLOGICAL PARAMETERS MONITORING**
TRAGBARE VORRICHTUNG MIT ÜBERWACHUNG PHYSIOLOGISCHER PARAMETER
DISPOSITIF PORTATIF AVEC SURVEILLANCE DE PARAMÈTRE PHYSIOLOGIQUE

(30) Priority: 07.12.2022 US 202263386474 P
(43) Date of publication of application: 15.10.2025
(73) Proprietor: Masimo Corporation, Irvine, CA 92618 (US)
(72) Inventor: AL-ALI, Ammar, Irvine, California 92618 (US); SCRUGGS, Stephen, Irvine, California 92618 (US); KOTCHERHA, Kyla Scott, Irvine, California 92618 (US)
(74) Representative: Michalski Hüttermann & Partner mbB
(86) International application number: PCT/US2023/082505
(87) International publication number: WO 2024/123768

(56) References cited:
- WO-A1-2021/196598
- CN-U- 217 548 018

## Description

### FIELD

The present disclosure relates to the field on noninvasive health monitoring. More specifically, the disclosure relates to a wearable health monitoring device incorporating a plurality of sensors, including electrodes for measuring electrical signals originating from a user's body.

### BACKGROUND

Wearable devices can be worn by a subject and can include physiological sensors to monitor physiological data and/or a health status of the subject. Physiological sensors can include electrodes that contact a subject's skin and that measure electrical signals originating from the subject. Electrical signals may result from the subject's cardiac activity. Electrocardiography is a technique for measuring the electrical activity of a heart. Cardiac electrical activity may be captured by electrodes, processed and/or analyzed by a hardware processor, and represented with an ECG waveform. WO 2021/196598 A1 discloses a smartwatch with electrodes that are fixated to the back shell of the smartwatch via fixing screws, while CN 217 548 018 U discloses a smartwatch that comprises two separate electrodes fixated to the back side of the cover plate of the smartwatch via protruding portions.

Spectroscopy is a common technique for measuring the concentration of organic and some inorganic constituents of a solution. The theoretical basis of this technique is the Beer-Lambert law, which states that the concentration *cᵢ* of an absorbent in solution can be determined by the intensity of light transmitted through the solution, knowing the pathlength *d_{λ}*, the intensity of the incident light *I*_{*0*,*λ*}, and the extinction coefficient *ε*_{*i*,*λ*} at a particular wavelength λ.

In generalized form, the Beer-Lambert law is expressed as: ${I}_{λ} = {I}_{0 , λ} {e}^{- {d}_{λ} ⋅ {μ}_{a , λ}}$ (1) ${μ}_{a , λ} = {\sum }_{\overline{i = 1}}^{n} {ε}_{i , λ} ⋅ {c}_{i}$ (2) where *µ_{α,λ}* is the bulk absorption coefficient and represents the probability of absorption per unit length. The minimum number of discrete wavelengths that are required to solve equations 1 and 2 is the number of significant absorbers that are present in the solution.

A practical application of this technique is pulse oximetry or plethysmography, which utilizes a noninvasive sensor to measure oxygen saturation and pulse rate, among other physiological parameters. Pulse oximetry or plethysmography relies on a sensor attached externally to the patient (typically for example, at the fingertip, foot, ear, forehead, or other measurement sites) to output signals indicative of various physiological parameters, such as a patient's blood constituents and/or analytes, including for example a percent value for arterial oxygen saturation, among other physiological parameters. The sensor has at least one emitter that transmits optical radiation of one or more wavelengths into a tissue site and at least one detector that responds to the intensity of the optical radiation (which can be reflected from or transmitted through the tissue site) after absorption by pulsatile arterial blood flowing within the tissue site. Based upon this response, a processor determines the relative concentrations of oxygenated hemoglobin (HbO₂) and deoxygenated hemoglobin (Hb) in the blood so as to derive oxygen saturation, which can provide early detection of potentially hazardous decreases in a patient's oxygen supply, and other physiological parameters.

A patient monitoring device can include a plethysmograph sensor. The plethysmograph sensor can calculate oxygen saturation (SpO₂), pulse rate, a plethysmograph waveform, perfusion index (PI), pleth variability index (PVI), methemoglobin (MetHb), carboxyhemoglobin (CoHb), total hemoglobin (tHb), respiration rate, glucose, and/or otherwise. The parameters measured by the plethysmograph sensor can display on one or more monitors the foregoing parameters individually, in groups, in trends, as combinations, or as an overall wellness or other index.

### SUMMARY

The invention is defined in the appended claims. Without limiting the scope of the appended claims, the description below describes some prominent features.

Details of one or more implementations of the subject matter described in this specification are set forth in the accompanying drawings and the description below. Other features, aspects, and advantages will become apparent from the description and drawings. Note that relative dimensions of the following figures may not be drawn to scale.

A wearable device can perform physiological measurements and can comprise a frame, an electrode secured to the frame, and a substrate in electrical connection with the electrode and responsive to the electrical signals originating from the user. The electrode can conduct electrical signals originating from a user of the wearable device and can comprise a first portion a second portion, a third portion, and an end portion. The first portion can have a surface configured to contact a skin of the user. The second portion can have a surface configured to contact the skin of the user. The third portion can be disposed between the first portion and the second portion and can comprise a surface that extends away from the surface of the first portion and the surface of the second portion and is separated from the skin of the user when the first portion or the second portion contacts the skin of the user. The third portion can further comprise a through-hole extending through the electrode and configured to receive at least a portion of the frame and a cover portion of the frame can occlude the third portion from contacting the skin of the user. The end portion can be adjacent to the first portion and can extend away from the first portion at an angle.

In some implementations, the end portion is enclosed by the frame.

In some implementations, the frame occludes the end portion from contacting the skin of the user.

In some implementations, a surface of the end portion does not contact the skin of the user.

In some implementations, the end portion is substantially orthogonal to the first portion.

In some implementations, the end portion comprises a through-opening extending through the end portion, the through-opening configured to receive a protrusion of the frame to secure the electrode to the frame.

In some implementations, the wearable device can further comprise a fourth portion that can comprise: a surface that is continuous with the surface of the second portion, wherein the surface of the fourth portion extends away from the surface of the second portion and is separated from the skin of the user when the second portion contacts the skin of the user; and a second through-hole extending through the electrode and configured to receive an electrically conductive material configured to conduct the electrical signals originating from the user to the substrate.

In some implementations, a second cover portion of the frame occludes the fourth portion from contacting the skin of the user.

In some implementations, the wearable device can further comprise a fifth portion comprising a surface that is continuous with the surface of the fourth portion and configured to contact the skin of the user; and another end portion adjacent to the fifth portion, the another end portion having a surface that is continuous with the surface of the fifth portion, the another end portion extending from the fifth portion at an angle.

In some implementations, the another end portion comprises another through-opening extending through the another end portion, the another through-opening configured to receive another protrusion of the frame to secure the electrode to the frame.

In some implementations, the first portion is substantially semi-annular.

In some implementations, the first portion and the second portion form at least a portion of a semi-annulus.

In some implementations, the surface of the third portion is continuous with the surface of the first portion and the surface of the second portion.

In some implementations, the end portion comprises a surface that is continuous with the surface of the first portion.

In some implementations, the wearable device can further comprise a hardware processor coupled to the substrate and configured to access the electrical signals conducted via the electrode.

In some implementations, the hardware processor is configured to perform one or more electrocardiography techniques with the electrical signals conducted via the electrode.

In some implementations, the hardware processor is configured to generate an electrocardiography (ECG) waveform from the electrical signals conducted via the electrode.

In some implementations, the hardware processor is configured to determine one or more cardiac conditions of the user based on at least the electrical signals conducted via the electrode.

In some implementations, the electrode is configured to secure to the frame without an adhesive.

A wearable device can perform physiological measurements and can comprise an electrode configured to conduct electrical signals originating from a user of the wearable device; a hardware processor in electrical communication with the electrode and responsive to the electrical signals conducted by the electrode; and a frame configured to hold the electrode. The frame can comprise a first receptable configured to hold a first portion of the electrode adjacent to a skin of the user to contact the skin of the user; a second receptable configured to hold a second portion of the electrode adjacent to the skin of the user to contact the skin of the user; and a cover disposed between the first receptacle and the second receptacle and configured to cover a third portion of the electrode to secure the electrode to the frame, wherein the third portion of the electrode is occluded by the cover from contacting the skin of the user.

In some implementations, the electrode comprises a through-hole disposed at the third portion of the electrode, the through-hole extending from a first surface of the electrode to a second surface of the electrode, the through-hole configured to receive a portion of the frame extending from the cover to secure the electrode to the frame.

In some implementations, the third portion comprises an edge that is continuous with an edge of the first portion and an edge of the second portion, the edge of the third portion forming a curve that is non-coincident with a curve formed by the edge of the first portion.

In some implementations, the electrode further comprises an end portion adjacent to the first portion, the end portion extending away from the first portion at an angle.

In some implementations, the end portion is substantially orthogonal to the first portion.

In some implementations, the end portion is enclosed by the frame of the wearable device.

In some implementations, the end portion comprises a through-hole extending through the end portion and configured to receive a portion of the frame to secure the electrode to the frame.

A wearable device can perform physiological measurements and can comprise: a frame comprising a protrusion; an electrode configured to conduct electrical signals originating from a user of the wearable device; and a substrate in electrical connection with the electrode and responsive to the electrical signals originating from the user. The electrode can comprise: an outer surface configured to at least partially contact a skin of the user; an inner surface opposite the outer surface; and a through-hole extending through the electrode between the outer surface and the inner surface, wherein the through-hole is configured to receive the protrusion to secure the electrode to the frame.

In some implementations, at least a portion of the outer surface of the electrode is occluded by the frame from contacting the skin of the user.

In some implementations, the electrode further comprises a substantially semi-cylindrical portion having a surface that forms at least a portion of the outer surface of the electrode, the through-hole extending through the substantially semi-cylindrical portion between the outer surface and the inner surface.

In some implementations, the substantially semi-cylindrical portion extends away from the skin of the user such that the surface of the substantially semi-cylindrical portion does not contact the skin of the user.

In some implementations, the electrode further comprises an end portion extending from the electrode at an angle with respect to a portion of the electrode adjacent to the end portion, the end portion being enclosed by the frame of the wearable device.

A wearable device can perform physiological measurements and can comprise: an electrode configured to conduct electrical signals originating from a user of the wearable device. The electrode can comprise: an outer surface configured to at least partially contact a skin of the user; an inner surface opposite the outer surface; and a through-hole extending through the electrode from the outer surface to the inner surface, wherein the through-hole is configured to receive an electrically conductive material configured to contact the through-hole to receive the electrical signals conducted by the electrode. The wearable device can further comprise a frame configured to hold the electrode; and a substrate in electrical connection with the electrode via the electrically conductive material and configured to receive the electrical signals from the electrode via the electrically conductive material.

In some implementations, at least a portion of the outer surface of the electrode is occluded by the frame from contacting the skin of the user.

In some implementations, the electrode further comprises a substantially semi-cylindrical portion having a surface that forms at least a portion of the outer surface of the electrode, the through-hole extending through the substantially semi-cylindrical portion between the outer surface and the inner surface.

In some implementations, the substantially semi-cylindrical portion extends away from the skin of the user such that the surface of the substantially semi-cylindrical portion does not contact the skin of the user.

In some implementations, the electrode further comprises an end portion extending from the electrode at an angle with respect to a portion of the electrode adjacent to the end portion, the end portion being enclosed by the frame of the wearable device.

A wearable device can perform physiological measurements and can comprise an electrode configured to conduct electrical signals originating from a user of the wearable device. The electrode can comprise: a first portion configured to contact a skin of the user, the first portion having a substantially semi-circular edge; a second portion configured to contact the skin of the user, the second portion having a substantially semi-circular edge defining at least a portion of a circle that is coincident with the substantially semi-circular edge of the first portion; and a third portion disposed between the first portion and the second portion, the third portion having an edge that is continuous with the substantially semi-circular edge of the first portion and the substantially semi-circular edge of the second portion, the edge of the third portion being non-coincident with the circle. The wearable device can further comprise a frame configured to secure to the electrode; and a substrate in electrical connection with the electrode and responsive to the electrical signals originating from the user.

In some implementations, at least a portion of the frame covers the third portion.

In some implementations, the third portion is separated from the skin of the user when the first portion or the second portion contacts the skin of the user.

In some implementations, the electrode further comprises an end portion adjacent to the first portion, the end portion having a surface that is continuous with a surface of the first portion, the end portion extending from the first portion at an angle.

In some implementations, the third portion comprises a through-hole extending through the electrode, wherein the through-hole is configured to receive at least a portion of the frame.

In some implementations, edge of the third portion intersects the circle.

A wearable device can perform physiological measurements and can comprise: an electrode configured to conduct electrical signals originating from a user of the wearable device. The electrode can comprise a first portion having a substantially semi-conical surface configured to contact a skin of the user; a second portion having a substantially semi-conical surface configured to contact the skin of the user; and a third portion disposed between the first portion and the second portion, the third portion having a substantially semi-cylindrical surface occluded from contacting the skin of the user by at least a portion of a frame of the wearable device. The wearable device can further comprise a substrate in electrical connection with the electrode and responsive to the electrical signals originating from the user.

A wearable device can perform physiological measurements and can comprise: an electrode configured to conduct electrical signals originating from a user of the wearable device. The electrode can comprise: a first portion having a surface configured to contact a skin of the user; and an end portion adjacent to the first portion and extending from the first portion at an angle, the end portion being enclosed by a frame of the wearable device. The wearable device can further comprise a substrate in electrical connection with the electrode and responsive to the electrical signals originating from the user.

In some implementations, the end portion extends orthogonally from the first portion.

In some implementations, the surface of the end portion is occluded by the frame from contacting the skin of the user.

In some implementations, the end portion comprises a through-hole configured to receive at least a portion of the frame to secure the electrode to the frame.

In some implementations, the end portion comprises a surface that is continuous with the surface of the first portion.

In some implementations, the first portion comprises an edge defining at least a portion of a curve, wherein the surface of the end portion is parallel with a plane intersected by the curve.

In some implementations, the edge of the first portion is substantially semi-circular and the curve is substantially circular.

A wearable device can perform physiological measurements and can comprise: a first strap secured to a first end of the wearable device; a second strap secured to a second end of the wearable device opposite the first end, wherein the first and second straps are configured to secure the wearable device to a user; a first electrode configured to conduct electrical signals originating from the user, wherein the first electrode intersects a first axis of the wearable device that is substantially parallel to a line extending along a length of the first strap and second strap between the first and second ends of the wearable device, wherein the first electrode intersects a second axis of the wearable device that is orthogonal to the first axis; and a second electrode configured to conduct electrical signals originating from the user, wherein a center of mass of the second electrode is displaced from a center of mass of the first electrode.

In some implementations, a center of mass of the first electrode is displaced from the first axis.

In some implementations, a center of mass of the first electrode is displaced from the second axis.

In some implementations, the first electrode is symmetrical about a line extending through a center of mass of the first electrode.

In some implementations, a third axis of the wearable device bisects the first electrode, the third axis intersecting the first axis and the second axis.

In some implementations, the first axis of the wearable device is substantially orthogonal to a line extending along a length of a forearm of the user to which the wearable device is secured.

In some implementations, the second axis of the wearable device is substantially parallel to a line extending along a length of a forearm of the user to which the wearable device is secured.

In some implementations, the first electrode is substantially semi-annular.

In some implementations, the second electrode is substantially semi-annular.

In some implementations, the first electrode is a half-annulus.

A wearable device may be configured to perform physiological measurements. The wearable device may comprise: a first strap, a second strap, a first electrode, and a second electrode. The first strap may be secured to a first end of the wearable device. The second strap may be secured to a second end of the wearable device. The first and second straps may be configured to secure the wearable device to a user. The first electrode may be configured to measure cardiac electrical signals via contact with skin of the user. A center of mass of the first electrode may be displaced from a first axis of the wearable device that is substantially parallel to a line extending along a length of the first strap and second strap between the first and second ends of the wearable device. The center of mass of the first electrode may be displaced from a second axis of the wearable device that is substantially orthogonal to the first axis. The second electrode may be configured to measure cardiac electrical signals via contact with the skin of the user.

In some implementations, the first axis of the wearable device is substantially orthogonal to a line extending along a length of a forearm of the user to which the wearable device is secured.

In some implementations, the wearable device has a lower moment of inertia about the first axis than about any other axis within a same plane as the first axis.

In some implementations, the wearable device is more likely to rotate about the first axis than about any other axis within a same plane as the first axis.

In some implementations, the second axis of the wearable device is substantially parallel to a line extending along a length of a forearm of the user to which the wearable device is secured.

In some implementations, the center of mass of the first electrode is displaced from a center of mass of the second electrode.

In some implementations, the center of mass of the first electrode is displaced from a center of mass of the wearable device.

In some implementations, a center of mass of the second electrode is displaced from a center of mass of the wearable device.

In some implementations, the first electrode is substantially semi-annular.

In some implementations, the second electrode is substantially semi-annular.

In some implementations, the first electrode is a half-annulus.

A wearable device may be configured to perform physiological measurements. The wearable device may comprise: a first strap, a second strap, a first electrode, and a second electrode. The first strap may be secured to a first end of the wearable device. The second strap secured to a second end of the wearable device. The first and second straps may be configured to secure the wearable device to a user. The first electrode may be configured to measure cardiac electrical signals via contact with skin of the user. A center of mass of the first electrode may lie on a first axis of the wearable device. The first axis may be non-parallel to a line extending along a length of the first strap and second strap between the first and second ends of the wearable device. The first axis may not be orthogonal to the line extending along the length of the first strap and second strap between the first and second ends of the wearable device. The second electrode may be configured to measure cardiac electrical signals via contact with the skin of the user. The center of mass of the first electrode may be displaced from a center of mass of the second electrode.

In some implementations, the first axis of the wearable device is non-parallel to a line extending along a length of a forearm of the user to which the wearable device is secured.

In some implementations, the wearable device has a greater moment of inertia about the first axis than about any other axis within a same plane as the first axis.

In some implementations, the wearable device is less likely to rotate about the first axis than about any other axis within a same plane as the first axis.

In some implementations, the center of mass of the first electrode is displaced from a center of mass of the wearable device.

In some implementations, a center of mass of the second electrode is displaced from a center of mass of the wearable device.

In some implementations, the first electrode is substantially semi-annular.

In some implementations, the second electrode is substantially semi-annular.

In some implementations, the first electrode is a half-annulus.

A wearable device may be configured to perform physiological measurements. The wearable device may comprise: a first strap, a second strap, a first electrode, and a second electrode. The first strap may be secured to a first end of the wearable device. The second strap secured to a second end of the wearable device. The first and second straps may be configured to secure the wearable device to a user. The first electrode may be configured to measure cardiac electrical signals via contact with skin of the user. The first electrode may intersect a first axis of the wearable device that is orthogonal to a line extending along a length of the first strap and second strap between the first and second ends of the wearable device. The first electrode may intersect a second axis of the wearable device that is orthogonal to the first axis. The second electrode may be configured to measure cardiac electrical signals via contact with the skin of the user. A center of mass of the first electrode may be displaced from a center of mass of the second electrode.

In some implementations, the wearable device has a lower moment of inertia about the first axis than about any other axis in a same plane. In some implementations, the wearable device is more likely to tilt or rotate about the first axis than about any other axis in a same plane.

In some implementations, the wearable device is more likely to rotate about the first axis than about any other axis of the wearable device in a same plane as the first axis.

In some implementations, the wearable device has a lower moment of inertia about the second axis than about any other axis in a same plane. In some implementations, the wearable device is more likely to tilt or rotate about the second axis than about any other axis in a same plane.

In some implementations, the wearable device is more likely to rotate about the second axis than about any other axis of the wearable device in a same plane as the second axis.

In some implementations, the first axis of the wearable device is substantially parallel to a line extending along a length of a forearm of the user to which the wearable device is secured.

In some implementations, the center of mass of the first electrode is displaced from a center of mass of the wearable device.

In some implementations, a center of mass of the second electrode is displaced from a center of mass of the wearable device.

In some implementations, the first electrode is substantially semi-annular.

In some implementations, the second electrode is substantially semi-annular.

In some implementations, the first electrode is a half-annulus.

A wearable device may be configured to perform physiological measurements. The wearable device may comprise: a first strap, a second strap, a first electrode, and a second electrode. The first strap may be secured to a first end of the wearable device. The second strap secured to a second end of the wearable device. The first and second straps may be configured to secure the wearable device to a user. The first electrode may be configured to measure cardiac electrical signals via contact with skin of the user. An axis of the wearable device that is non-parallel to a line extending along a length of the first strap and second strap between the first and second ends of the wearable device may bisect the first electrode. The second electrode may be configured to measure cardiac electrical signals via contact with the skin of the user. A center of mass of the first electrode may be displaced from a center of mass of the second electrode.

In some implementations, the first axis of the wearable device is non-parallel to a line extending along a length of a forearm of the user to which the wearable device is secured.

In some implementations, the wearable device has a greater moment of inertia about the first axis than about any other axis within a same plane as the first axis. In some implementations, the wearable device is less likely to tilt or rotate about the first axis than about any other axis in a same plane.

In some implementations, the wearable device is less likely to rotate about the first axis than about any other axis within a same plane as the first axis.

In some implementations, the line extending along the length of the first strap and second strap between the first and second ends of the wearable device coincides with a second axis of the wearable device about which the wearable device has a lower moment of inertia than any other axis of the wearable device in a same plane as the second axis.

In some implementations, the line extending along the length of the first strap and second strap between the first and second ends of the wearable device coincides with a second axis of the wearable device about which the wearable device is more likely to rotate than any other axis of the wearable device in a same plane as the second axis.

In some implementations, the center of mass of the first electrode is displaced from a center of mass of the wearable device.

In some implementations, a center of mass of the second electrode is displaced from a center of mass of the wearable device.

In some implementations, the first electrode is substantially semi-annular.

In some implementations, the second electrode is substantially semi-annular.

In some implementations, the first electrode is a half-annulus.

A wearable device may be configured to perform physiological measurements. The wearable device may comprise a sensor or module. The sensor or module may comprise a frame, an electrode, and a substrate. The frame may comprise one or more receptacles. The electrode may be disposed within the frame. The electrode my comprise an outer surface. The outer surface may be configured to contact skin of a user of the wearable device via the one or more receptacles. Less than all portions of the outer surface may be configured to contact the skin of the user. The substrate may be in electrical connection with the electrode.

In some implementations, the outer surface further comprises recess portions occluded from contacting the skin of the user, wherein the recess portions are disposed between portions of the outer surface that are configured to contact the skin of the user.

A wearable device configured to perform physiological measurements. The wearable device may comprise a sensor or module. The sensor or module may comprise an electrode, a frame, and a substrate. The electrode may comprise a first portion configured to contact skin of a user, a second portion configured to contact the skin of the user, and a recess portion disposed between the first and second portions. The frame may comprise a first receptacle configured to expose the first portion of the electrode to the skin of the user, a second receptacle configured to expose the second portion of the electrode to the skin of the user; and a cover portion disposed between the first and second receptacles and configured to envelope the recess portion. The substrate may be electrical connection with the electrode.

In some implementations, the cover portion is further configured to occlude the recess portion from contacting the skin of the user.

In some implementations, the cover portion is further configured to secure the recess portion to the frame to prevent the electrode from moving relative to the frame.

A wearable device may be configured to perform physiological measurements. The wearable device may comprise a sensor module. The sensor module may comprise a frame, an electrode, and a substrate. The frame may comprise a protrusion. The electrode may be secured to the frame. The electrode may comprise an outer surface configured to contact skin of a user, an inner surface opposite the outer surface, and an opening through the electrode between the outer surface and the inner surface. The opening may be configured to receive the protrusion to secure the electrode to the frame. The substrate may be in electrical connection with the electrode.

In some implementations, the opening is disposed within a plane that is substantially parallel to a plane in which portions of the outer surface adjacent to the opening are disposed.

A wearable device may be configured to perform physiological measurements. The wearable device may comprise a sensor or module. The sensor or module may comprise a frame, an electrode, and a substrate. The electrode may be disposed within the frame. The electrode may comprise an outer surface configured to contact skin of a user. The outer surface may be substantially non-planar. The substrate may be in electrical connection with the electrode.

In some implementations, the outer surface of the electrode forms a partial, substantially conical surface.

In some implementations, the outer surface of the electrode forms a partial, substantially spherical surface.

In some implementations, the outer surface of the electrode is substantially convex.

In some implementations, the frame comprises an outer surface configured to contact the skin of the user, wherein the outer surface of the frame is substantially convex, wherein the outer surface of the electrode is flush with the outer surface of the frame.

In some implementations, the outer surface of the electrode is non-parallel with a substantially planar surface of the substrate.

A wearable device may be configured to perform physiological measurements. The wearable device may comprise a sensor or module. The sensor or module may comprise a frame, a substrate, and an electrode. The electrode may be disposed within the frame. The electrode may be in electrical communication with the substrate. The electrode may comprise an outer surface configured to contact skin of a user. A portion of the outer surface may be non-parallel with a surface of the substrate.

In some implementations, a majority of the outer surface is non-parallel with the surface of the substrate.

For purposes of summarization, certain aspects, advantages and novel features are described herein. Of course, it is to be understood that not necessarily all such aspects, advantages or features need to be present in any particular aspect.

Various combinations of the above and below recited features, implementations, and aspects are also disclosed and contemplated by the present disclosure.

### BRIEF DESCRIPTION OF THE DRAWINGS

The drawings and the associated descriptions are provided to illustrate aspects of the disclosure and not to limit the scope of the claims. In the present disclosure, "bottom" refers to the side facing a wearer's wrist when an example wearable device disclosed herein is worn on the wearer's wrist and "top" refers to the side facing away from the wearer's wrist.
Figures 1A-1C illustrate example wearable devices including a physiological parameter measurement sensor or module worn on a wrist using straps.
Figure 2 is a diagram illustrating schematically a network of non-limiting examples of devices that can communicate with the wearable device disclosed herein.
Figure 3 illustrates a schematic system diagram of a wearable device including a physiological parameter measurement module.
Figure 4A illustrates a schematic system diagram of an example wearable device including a physiological parameter measurement module.
Figure 4B illustrate a schematic diagram of an example device processor shown in Figure 4A.
Figure 4C illustrates a schematic system diagram of an example sensor or module processor shown in Figure 4A.
Figure 4D illustrates a block diagram of an example front end circuitry of the sensor or module processor of Figure 4C.
Figure 5A illustrates a front view of an example aspect of a physiological parameter measurement sensor or module.
Figure 5B illustrates an exploded view of an example aspect of a physiological parameter measurement sensor or module.
Figure 6A illustrates a perspective view of PCB substrate of a physiological parameter measurement sensor or module with example plethysmograph sensor arrangement.
Figures 6B-6C illustrate an example physiological parameter measurement sensor or module and example light paths between emitters and detectors of the module.
Figures 6D-6G illustrate an example physiological parameter measurement sensor or module and example light barriers or light blocks between emitter and detector chambers of the module.
Figure 6H illustrates an example physiological parameter measurement sensor or module and example light diffusing material and light transmissive lens(es) or cover(s).
Figure 7A illustrates an example wearable device with electrodes.
Figure 7B illustrates an example wearable device.
Figure 7C illustrates an example wearable device with electrodes.
Figure 8 illustrates an example sensor or module with electrodes.
Figure 9A is an exploded perspective view of an example sensor or module of a wearable device.
Figure 9B is an exploded perspective view of an example sensor or module of a wearable device.
Figure 10A is a cutaway view of an example sensor or module.
Figure 10B is a cutaway view of an example frame of a sensor or module.
Figure 11 is a cutaway view of electrodes and a substrate of a sensor or module.
Figures 12A-12B are side views of an example electrode.
Figures 13A-13B are side views of example electrodes.
Figure 13C is a perspective view of an example electrode.
Figure 14 is a perspective cutaway view of an example frame of a sensor or module.
Figure 15 is a cutaway view of an example frame of a sensor or module.
Figure 16 shows a block diagram illustrating an example aspect of the wearable device in communication with an external device via a network.
Figure 17 illustrates an example user interface of the health application.

### DETAILED DESCRIPTION

Although certain aspects and examples are described below, those of skill in the art will appreciate that the disclosure extends beyond the specifically disclosed aspects and/or uses and obvious modifications and equivalents thereof based on the disclosure herein. Thus, it is intended that the scope of the disclosure herein disclosed should not be limited by any particular aspects described below.

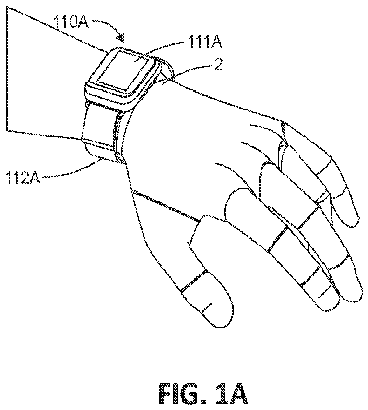

Use of a wearable healthcare monitoring device, which can include oximetry- or plethysmograph-based and/or ECG physiological parameters, can be beneficial to the wearer. Figure 1A illustrates an example implementation of a wearable device 110A. The device 110A can be a wristwatch (also referred to as a "watch") which can incorporate one or more sensors, including physiological sensors, and time-indicating functions. The device 110 can include a strap 112A to releasably secure the device 110A around the wrist 2 of the wearer. The strap 112A may be adjustable to accommodate various sizes of wrists or other body parts to which the device 110A is secured. Of course, the present specification is not limited solely to a watch but can include other implementations. For example, the device 110 can be worn on the wrist without a watch, screen, or other smartwatch features. As another example, the device 110 may be worn on another body part of a wearer other than the wrist 2, such as an arm, a leg, an ankle, or the like.

The device 110A can include a display 111A and can display one or more of the measured physiological parameters on the display 111A. The information can be helpful in providing feedback to the wearer and/or a third-party user, for example, a healthcare professional or the wearer's family member, when the wearer is exercising, or otherwise for warning the wearer of possible health-related conditions, including but not limited to changes in the wearer's physiological parameters in response to medication that is being administered to the wearer. The wearer can be informed of physiological parameters, such as vital signs including but not limited to heart rate (or pulse rate), and oxygen saturation by the wearable device 110A.

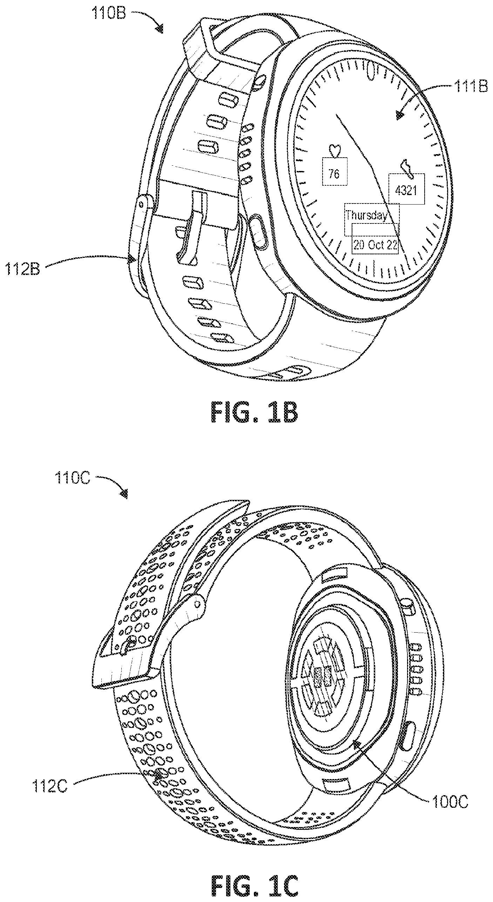

Figure 1B is a perspective view of an example wearable device 110B. The wearable device 110B may be a wearable device such as a smartwatch. The device 110B may include a display 111B. The display 111B may be an LED display. The display 111B may be configured to display day, month, date, year, and/or time. The display 111B may display time as analog or digital. The display 111B may display physiological related data such as physiological parameters, physiological trends, physiological graphs, or the like. For example, the display 111B may display heart rate, respiration rate, ECG data, SpO2, a step count of the number of steps taken by a user of the device 110B, or the like.

The device 110B may include one or more straps 112B. The straps 112B may be adjustable. The straps 112B may be configured to secure the device 110B to a body part of a user, such as a wrist.

Figure 1C is a perspective view of an example wearable device 110C. The device 110C can include one or more straps 112C. The device 110C can include a physiological parameter measurement sensor or module 100C configured to measure an indication of the wearer's physiological parameters, which can include, for example, heart rate, pulse rate, respiration rate, oxygen saturation (SpO₂), Pleth Variability Index (PVI), Perfusion Index (PI), Respiration from the pleth (RRp), hydration, glucose, blood pressure, ECG, and/or other parameters. The sensor or module 100C can perform spectroscopy, plethysmography, oximetry, electrocardiography, etc. The sensor or module 100C can perform intermittent and/or continuous monitoring of the measured parameters. The sensor or module 100C can additionally and/or alternatively perform a spot check of the measured parameters, for example, upon request by the wearer.

The physiological parameter measurement sensor or module 100C can include an optical sensor which can include emitters and/or detectors. Emitters can emit light of various wavelengths which may penetrate into a tissue of the user. Detectors can detect light emitted by the emitters and generate one or more signals based at least in part on the light detected that was emitted by the emitters. The detectors may generate data relating to blood oxygen saturation of a user of the device 110C. The detectors may generate data relating to spectroscopy.

The physiological parameter measurement sensor or module 100C can include one or more electrodes which can contact the skin of a user. The electrodes can measure electrical activity. The electrodes may obtain data related to the cardiac activity of a user to generate ECG data.

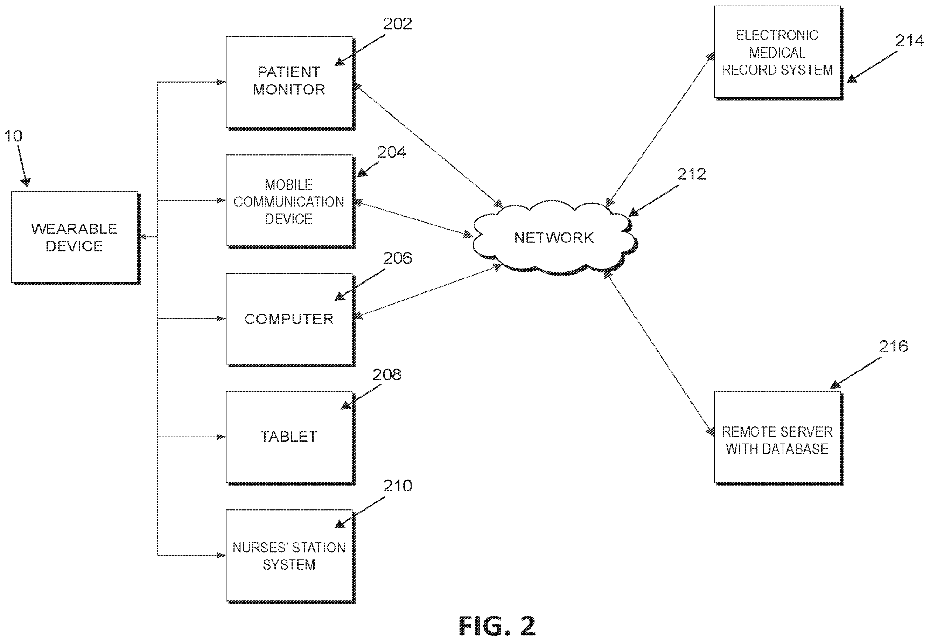

The wearable device 10 can be used in a standalone manner and/or in combination with other devices and/or sensors. As shown in Figure 2, the device 10 can connect (for example, wirelessly) with a plurality of devices, including but not limited to a patient monitor 202 (for example, a bedside monitor such as Masimo's Radical-7^{®}, Rad-97^{®} (optionally with noninvasive blood pressure or NomoLine capnography), and Rad-8^{®} bedside monitors, a patient monitoring and connectivity hub such as Masimo's Root^{®} Platform, any handheld patient monitoring devices, and any other wearable patient monitoring devices), a mobile communication device 204 (for example, a smartphone), a computer 206 (which can be a laptop or a desktop), a tablet 208, a nurses' station system 201, glasses such as smart glasses configured to display images on a surface of the glasses and/or the like. The wireless connection can be based on Bluetooth technology, WiFi, near-field communication (NFC) technology, and/or the like. Additionally, the wearable device 10 can connect to a computing network 212 (for example, via any of the connected devices disclosed herein, or directly). The network 212 may comprise a local area network (LAN), a personal area network (PAN) a metropolitan area network (MAN), a wide area network (WAN) or the like, and may allow geographically dispersed devices, systems, databases, servers and the like to connect (e.g., wirelessly) and to communicate (e.g., transfer data) with each other. The wearable device 10 can establish connection via the network 212 to one or more electronic medical record system 214, a remote server with a database 216, and/or the like.

The device 10 can include open architecture to allow connection of third-party wireless sensor, and/or allow third party access to a plurality of sensors on the wearable device 10 or connected to the wearable device 10. The plurality of sensors can include, for example, a temperature sensor, an altimeter, a gyroscope, an accelerometer, emitters, LEDs, etc. Third party applications can be installed on the wearable device 10 and can use data from one or more of the sensors on the wearable device 10 and/or in electrical communication with the wearable device.

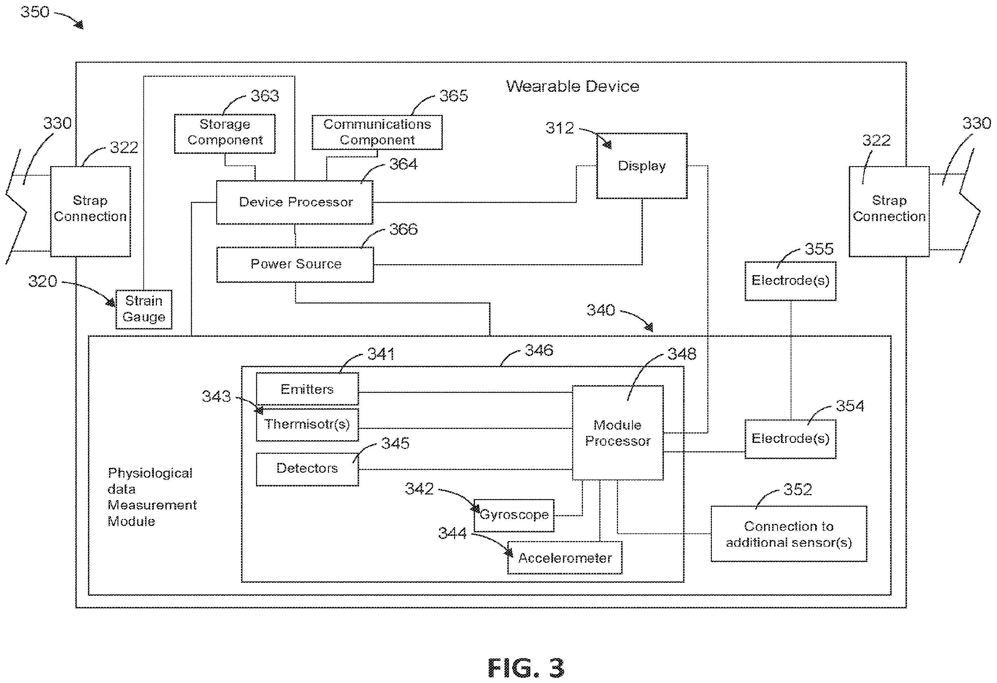

Figure 3 is a schematic diagram of a wearable device 350 illustrating various example components thereof. The device 350 can include a device processor 364, which can be a digital/analog chip or other processor(s), such as a digital watch processor or a smartwatch processor. The device processor 364 can include one or more hardware processors configured to execute program instructions to cause the device processor 364 or other components of the device 350 to perform one or more operations. The device processor 364 can be located on a substrate such as a printed circuit board (PCB). The device processor 364 can generate display data to render a display or user interface on the display 312.

The device 350 can include a power source 366, which can be a battery, for powering the components of the device 350 such as the device processor 364, and/or the physiological data measurement module 340. The power source 366 can include a dual-battery configuration with a main battery and a backup battery. The device 350 can additionally or alternatively be configured to be solar-powered, for example, by including a solar panel on the dial or elsewhere of the wearable device 350.

The display 312 can include an LED display. The display 312 can use e-ink or ULP (ultra low power screen) technology, which draws little amount of current for displaying information. The display 312 may automatically adjust the brightness, being brighter when outdoors and dimmer when indoors to further prolong battery life. The display screen 312 can display physiological parameters, or combinations thereof, monitored by the sensor or module 340.

The device 350 can include a storage component 363. The storage component 363 can include any computer readable storage medium and/or device (or collection of data storage mediums and/or devices), including, but not limited to, one or more memory devices that store data, including without limitation, dynamic and/or static random-access memory (RAM), programmable read-only memory (PROM), erasable programmable read-only memory (EPROM), electrically erasable programmable read-only memory (EEPROM), optical disks (e.g., CD-ROM, DVD-ROM, etc.), magnetic disks (e.g., hard disks, floppy disks, etc.), memory circuits (e.g., solid state drives, random-access memory (RAM), etc.), and/or the like. Such stored data can be processed and/or unprocessed physiological data obtained from physiological sensors.

The device 350 can include a communication component 365 which can facilitate communication (via wired and/or wireless connection) between the device 350 (and/or components thereof) and separate devices, such as separate wearable devices, mobile devices, monitoring devices, monitoring hubs, computing devices, sensors, systems, servers, or the like. For example, the communication component 365 can be configured to allow the device 350 to wirelessly communicate with other devices, systems, and/or networks over any of a variety of communication protocols. The communication component 365 can be configured to use any of a variety of wireless communication protocols, such as Wi-Fi, Bluetooth^{®}, ZigBee^{®}, Z-wave^{®}, cellular telephony, infrared, near-field communications (NFC), radio frequency identification (RFID), satellite transmission, proprietary protocols, combinations of the same, and the like. The communication component 365 can allow data and/or instructions to be transmitted and/or received to and/or from the device 350 and separate computing devices. The communication component 365 can be configured to transmit and/or receive (for example, wirelessly) processed and/or unprocessed physiological data with separate computing devices. The communication component 365 can be embodied in one or more components that are in communication with each other. The communication component 365 can include one or more wireless transceivers, one or more antennas, one or more radios, and/or a near field communication (NFC) component such as a transponder.

The sensor or module 340 of the wearable device 350 can include a sensor or module processor 348. The sensor or module processor 348 can include one or more hardware processors configured to execute program instructions to cause the sensor or module processor 348 to perform one or more operations. The sensor or module processor 348 can include a memory, and/or other electronics. The sensor or module processor 348 can be located on a substrate such as a PCB. The sensor or module processor 348 can be in electrical communication with the emitters 341, thermistor(s) 343, detectors 3245, gyroscope 342, accelerometer 344, and/or electrodes 354, 355.

The physiological data measurement module 340 can be configured to measure an indication of the wearer's physiological parameters. This can include, for example, pulse rate, respiration rate, SpO2, Pleth Variability Index (PVI), Perfusion Index (PI), Respiration from the pleth (RRp), total hemoglobin (SpHb), hydration, glucose, blood pressure, and/or other parameters. The sensor or module 340 can perform intermittent and/or continuous monitoring of the measured parameters. The sensor or module 340 can additionally and/or alternatively perform a spot check of the measured parameters, for example, upon request by the wearer.

The sensor or module processor 348 can determine and output the physiological parameters based on the detected signals for display on the device display 312. The sensor or module processor 348 can generate display data to render a display or user interface on the display 312. Optionally, the sensor or module 340 can send the signals from the detectors 345 (for example, preprocessed signals) to the device processor 364, which can determine and output for display the physiological parameters based on the detected signals.

The sensor or module processor 348 can process signals from one or more of the sensors of the sensor or module 340 (or optionally other sensors in communication with the device 350) to determine a plurality of physiological parameters. The sensor or module processor 348 can be configured to drive the emitters 341 to emit light of different wavelengths and/or to process signals from the detectors 345 of attenuated light after absorption by the body tissue of the wearer. The absorption of light can be via transreflectance by the wearer's body tissue, for example, by the pulsatile arterial blood flowing through the capillaries (and optionally also the arteries) within a tissue site where the device 350 is worn (for example, the wrist).

The sensor or module 340 can include a plurality of light emitters 341. The emitters 341 can include light emitting diodes (LEDs). The emitters 341 can include more than one group or cluster of light emitters 341. In some implementations, each group or cluster of emitters 341 may include five emitters, or less than five emitters. The detectors 345 can include light sensitive photodetectors or photodiodes. The detectors 345 can include more than one group or cluster of detectors 345. In some implementations, each group or cluster of detectors 345 may include a single detector, or more than one detector. Each group of emitters 341 can be configured to emit five different wavelengths such as described herein.

The sensor or module 340 can include one or more thermistors 343 or other types of temperature sensors. The thermistor(s) 343 can be placed near one or more groups of emitters 341. There can be at least one thermistor 343 near each group of emitters 341. Optionally the device 350 can include one or more thermistors 343 located at other places of the sensor or module 340. The thermistor(s) 343 can provide for wavelength correction of the light emitted by the emitters 341. Optionally, the thermistor(s) 343 can additionally measure a temperature of the wearer of the device 350. The emitters 341, the thermistor(s) 343, and/or the detectors 345 can be positioned on a substrate such as a PCB.

The emitters 341 of the module 340 can be configured to emit a plurality of (for example, three, four, five, or more) wavelengths. The emitters 341 can be configured to emit light of a first wavelength providing an intensity signal that can act as a reference signal. The first wavelength can be more absorbent by the human body than light of other wavelengths emitted by the emitters 341. The reference signal can be used by the module processor 348 to extract information from the other signals, for example, information relevant to and/or indicative of the pulsing rate, harmonics, or otherwise. The module processor 348 can focus the analysis on the extracted information for calculating the physiological parameters of the wearer. The first wavelength can include a range of wavelengths, including, for example, from about 530 nm to about 650 nm, or from about 580 nm to about 585 nm, or from about 645 nm to about 650 nm, or about 580 nm, or about 645 nm. The light providing the reference signal can have an orange color or yellow. Alternatively, the light providing the reference signal can have a green color.

The emitters 341 can be configured to emit light of a second wavelength having a red or orange color. The second wavelength can be from about 620 nm to about 660 nm. Light of the second wavelength can be more sensitive to changes in SpO2. The second wavelength is preferably closer to 620 nm (for example, about 625 nm), which results in greater absorption by the body tissue of the wearer, and therefore a stronger signal and/or a steeper curve in the signal, than a wavelength that is closer to 660 nm. The module processor 348 can extract information such as the pleth waveform from signals of the second wavelength.

The emitters 341 can be configured to emit light of a third wavelength of about 900 nm to about 910 nm, or about 905 nm, or about 907 nm. The third wavelength can be in the infrared range. The pulse oximeter processor can use the third wavelength as a normalizing wavelength when calculating ratios of the intensity signals of the other wavelengths, for example, a ratio of the intensity signals of the second wavelength (red) to the third wavelength (infrared).

Additionally or optionally, the emitters 341 can be configured to emit light having a fourth wavelength that is more sensitive to changes in water than the rest of the emitted wavelengths. The fourth wavelength can be in the infrared range or about 970 nm or higher than 970 nm. The module processor 348 can determine physiological parameters such as a hydration status of the wearer based at least in part on a comparison of the intensity signals of the fourth wavelength and a different wavelength detected by certain detectors 345.

The emitters 341 can be configured to emit light of a fifth wavelength. Each of the wavelengths emitted may be different than the others.

In some aspects, drivers may drive the emitters at varying intensities. The intensity at which the drivers drive the emitters may affect the amount of light that is outputted (e.g., lumens), the strength of the light signal that is outputted, and/or the distance that the outputted light travels. The drivers may drive the emitters at varying intensities according to modeling, logic and/or algorithms. The logic and/or algorithms may be based, at least in part, on various inputs. The inputs may include historical data, the amount of light that is attenuated, for example as the light penetrates and travels through the tissue of the wearer, or the amount of blood with which the light is interacting, or the type of blood (e.g., venous, arterial) or type of blood vessel (e.g., capillary, arteriole) with which the light is interacting and/or the heat being generated by the emitters. For example, the drivers may increase the intensity at which they drive the emitters based upon a determination that too much light is being attenuated in the tissue or that the light is not interacting with enough blood. As another example, the drivers may decrease the intensity at which they drive the emitters based upon a determination that the emitters have exceeded a threshold temperature. The threshold temperature may be a temperature which may be uncomfortable for human skin.

In some aspects, each of the drivers may be capable of driving a corresponding emitter at various intensities independently of the other drivers. In some aspects, each of the drivers may drive a corresponding emitter at various intensities in unison with each of the other drivers.

Additionally, various LEDs may be used in various aspects. For example, certain LEDs may be used which are capable of outputting more light with the same amount of power as other LEDs. These LEDs may be more expensive. In some aspects, less expensive LEDs may be used. In some aspects, a combination of various types of LEDs may be used.

The device 350 can include a gyroscope 342, an accelerometer 344, and/or other position and/or posture detection sensor(s) configured to detect motion related data. The gyroscope 342 and/or the accelerometer 344 can be located on a substrate such as a PCB.

The device 350 can include an electrocardiogram (ECG) sensor including a plurality of electrodes 354, 355 configured to make contact with the wearer's skin. One or more electrodes 354 may be located on the sensor or module 340. One or more electrodes 355 may be located elsewhere on the device 350.

Optionally, the sensor or module 340 can be preassembled before being integrated into the device 350. An electrical connection can be established between the sensor module PCB and the circuit of the rest of the device 350, including for example, the device processor 364, the display 312, and the power source 366. The sensor or module 340 can be characterized before being assembled with the rest of the device 350. Alternatively, a housing of the module can be an integral component of a housing of the device.

The device 350 can include a gyroscope 342, an accelerometer 344, and/or other position and/or posture detection sensor(s). The gyroscope 342 and/or the accelerometer 344 can be in electrical communication with the sensor or module processor 348. The sensor or module processor 348 can determine motion information from signals from the gyroscope 342 and/or the accelerometer 344. The motion information can provide noise reference for analysis of the pleth information and other signal processing (for example, processing of ECG signals) performed by the sensor or module processor 348. The gyroscope 342 and/or the accelerometer 344 can be located on a PCB.

The device 350 can include an electrocardiogram (ECG) sensor including a plurality of electrodes 354, 355 configured to make contact with the wearer's skin. In some implementations, the electrode(s) 354 may be located on the sensor or module 340. In some implementations, the electrode(s) 355 may be located elsewhere on the device 350 (for example, an electrode 355 can form a part of the housing of the wearable device 350). In some implementations, the electrode(s) 354 can include a reference electrode and a negative electrode. In some implementations, the electrode(s) 355 can include a positive electrode.

The electrode(s) 354, 355 can comprise an electrically conductive material and can conduct electrical signals originating from a user, such as from a user's muscular activity (e.g., cardiac activity), neural activity, etc. The module processor 348 and/or device processor 364 can receive electrical signals conducted by the electrode(s) 354, 355. The module processor 348 and/or device processor 364 can implement one or more electrocardiography techniques with electrical signals received via the electrodes 354 and/or 355. For example, the module processor 348 and/or device processor 364 can generate an ECG waveform from electrical signals received from the electrodes 354 and/or 355 which can be displayed via the display 312. As another example, the module processor 348 and/or device processor 364 can determine a heart rate from electrical signals received from the electrodes 354 and/or 355. As another example, the module processor 348 and/or device processor 364 can determine one or more cardiac conditions (e.g., tachycardia, fibrillation, arrythmia, arrest, flutter, bradycardia, premature contractions, etc.) based on analyzing electrical signals received from the electrodes 354 and/or 355.

The tightness of the device 350 on the wearer's body (for example, the wrist) can be adjusted by adjusting any suitable strap(s) 330 used to secure the device 350 to the wearer's body. The strap(s) 330 can be connected to the device 350 using any suitable strap connections 322. For example, the strap connections 322 can be compatible with third party watch bands, wearable blood pressure monitors, and/or the like. The adjustment of the strap 30 around the wearer's wrist can reduce and/or eliminate a gap between a tissue-facing surface of the module 340 and the wearer's skin to improve accuracy in the measurements. The device 350 can include an optional strain gauge 320 to measure a pressure of the device 350 on the wearer. The strain gauge 320 can be located in a device housing between the sensor module 340 and other components of the device 350, for example, the power source 366, the device processor 364, or otherwise. When the device 350 is worn on the wearer, for example, on the wrist, the pressure exerted by the module 340 against the tissue can be transmitted to and measured by the strain gauge 320. Readings from the strain gauge 320 can be communicated to the device processor 364, which can process the readings and output an indication of the pressure asserted by the device 350 on the wearer to be displayed on the display 312. Optionally, the device 350 can output a warning that the device 350 is worn too tight or too loose when the device 350 has determined that the wearer's SpO2 readings are decreasing by a certain percentage, at a certain rate, and/or at a certain rate within a predetermined amount of time.

The module 340 disclosed herein can include an optional connector 352 for receiving additional sensor(s) such as a fingertip sensor configured to monitor opioid overdose, or any other suitable noninvasive sensor, such as an acoustic sensor, a blood pressure sensor, or otherwise. The connector 352 can be oriented such that the second sensor can extend from a housing of the device 350 with reduced or no impingement of the tissue at the device/tissue interface, resulting in less or no effect of the connector 352 or the second sensor on the blood flow through the device measurement site.

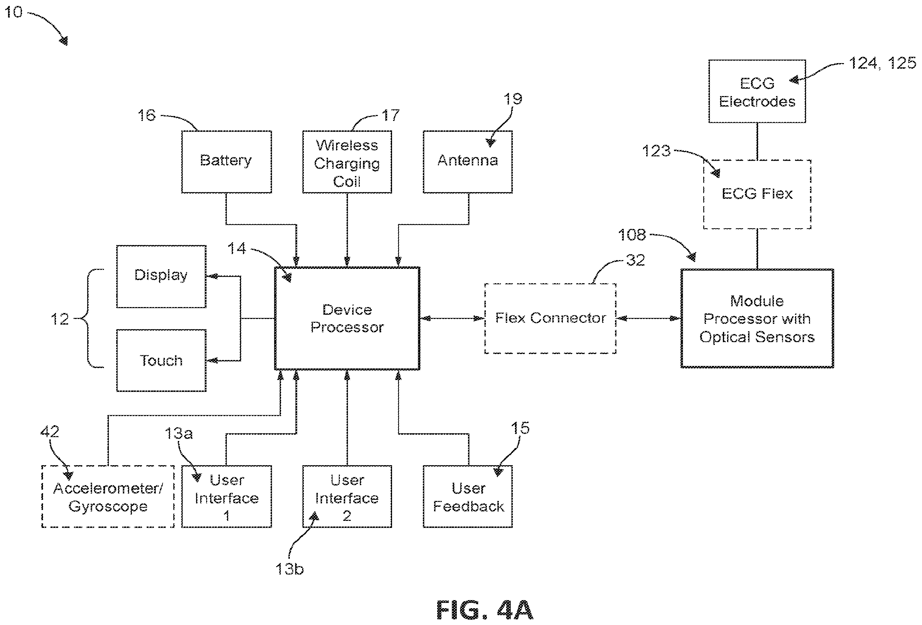

Figure 4A illustrates schematically an example wearable device 10 disclosed herein. As described above, the device processor 14 can be connected to the module sensor 108 of a physiological parameter measurement module, which can include emitters, detectors, thermistors, and other sensors disclosed herein. The electrical connection between the device processor 14 and the sensor or module processor 108 can be establish optionally via a flex connector 32. The sensor or module processor 108 can be coupled to the electrodes 124, 125, optionally via an ECG flex connector 123.

The device processor 14 can be connected to a display 12, which can include the display screen and touch input from the wearer. The device processor 14 can include a battery 16, and optionally one or more wireless charging coils 17 to enable wireless charging of the battery 16. The device processor 14 can be connected to an antenna 19 for extending signals transmitted wirelessly, for example, to an external device as described with reference to Figure 2. The device processor 14 can include connection to a first user interface (UI 1) 13a and a second user interface (UI 2) 13b on the device 10 to receive input from the wearer. First and second user interface 13a, 13b can be in the form of buttons. Additionally or alternatively, the device 10 can include a microphone. The device 10 can receive user inputs via the user interfaces, which can be the buttons, the microphone, and/or the touchscreen. The user inputs can command the device 10 to turn on and/or off certain measurements, and/or to control externally connected devices, such as an insulin pump, a therapeutics delivery device, or otherwise. The device processor 14 can be connected to a user feedback output 15 to provide feedback to the wearer, for example, in the form of vibration, an audio signal, and/or otherwise. The device processor 14 can optionally be connected to an accelerometer and/or a gyroscope 42 located on the device 10 that is different from the accelerometer 114 and gyroscope 112 on the physiological parameter measurement module 100. The accelerometer and/or gyroscope 42 can measure position and/or orientation of the wearer for non-physiological parameter measurement functions, for example, for sensing that the wearer has woken up, rotating the display 12, and/or the like.

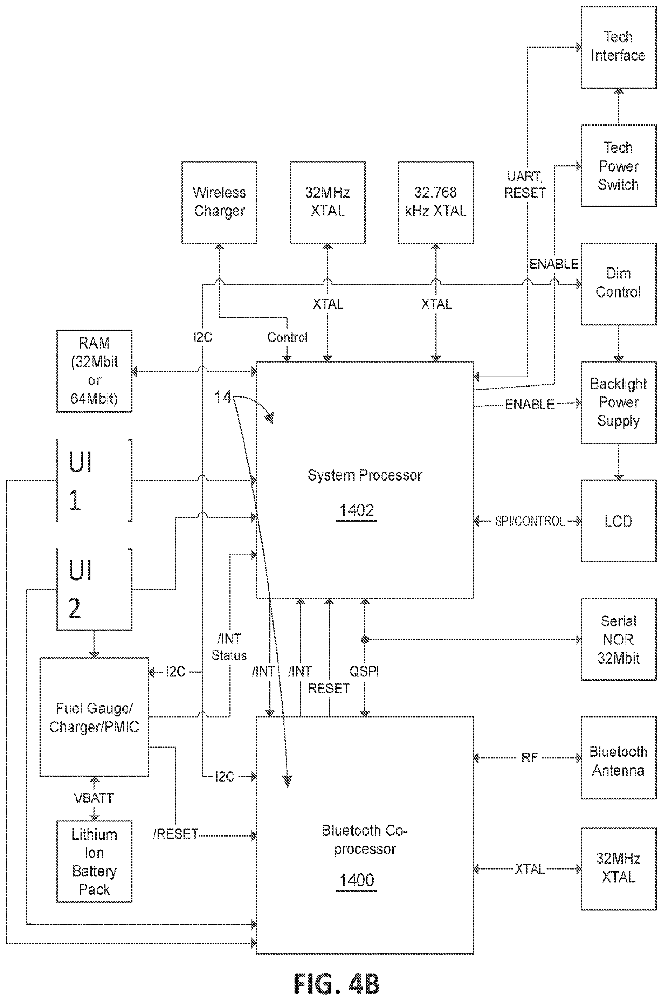

Figure 4B illustrates example components of the device processor 14 PCB board. As shown in Figure 4B, the device processor 14 can include a Bluetooth co-processor 1400 and a system processor 1402. The system processor 1402 can run the peripheral functions of the device 10, receive user (that is, the wearer) input and communicate to the sensor or module processor 108. The Bluetooth co-processor 1400 can focus on managing Bluetooth communication so as to allow the system processor 1402 to focus on the high memory utilization tasks, such as managing the display screen 12. The Bluetooth co-processor 1400 can be activated when there is incoming and/or outgoing Bluetooth communication. Alternatively, the Bluetooth co-processor 1400 can be replaced by a different wireless co-processor configured to manage wireless communication using a different wireless communication protocol.

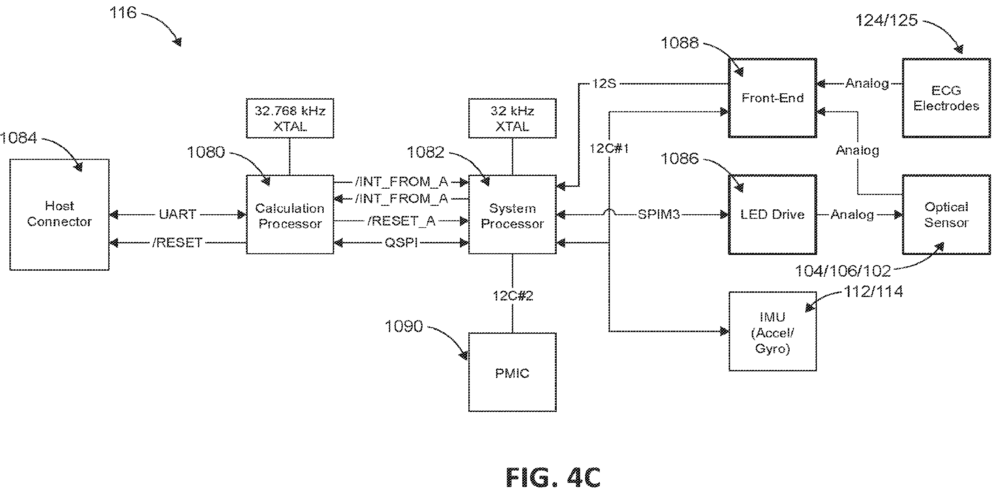

Figure 4C illustrates example components of the module processor PCB board 116. As shown in Figure 4C, the sensor or module processor 108 can include a calculation processor 1080 and a system processor 1082. The calculation processor 1080 can manage host communication with the device processor 14 via a host connector 1084. The calculation processor 1080 can perform algorithm computations to calculate the physiological parameters based on the signals received from the electrodes 124/125 and the optical sensor including the emitters 104, the detectors 106, and the temperature sensors 102, and optionally from other sensors in communication with the sensor or module processor 108. The calculation processor 1080 can have relatively large memory suitable for running algorithm computations. The system processor 1082 can be in communication with a power management integrated circuit (PMIC) 1090. The system processor 1082 can run the physical system of the sensor or module 100 (for example, including turning on and off the emitter LEDs, changing gain, setting current, reading the accelerometer 114 and/or the gyroscope 112, and the like) and decimate data to a lower sampling rate. The system processor 1082 can focus on data processing, taking measurements and diagnostics, and basic functions of the sensor or module processor 108. The system processor 1082 can allow the calculation processor 1080 to sleep (being inactive) most of the time, and only wake up when there is enough measurement data to perform calculations.

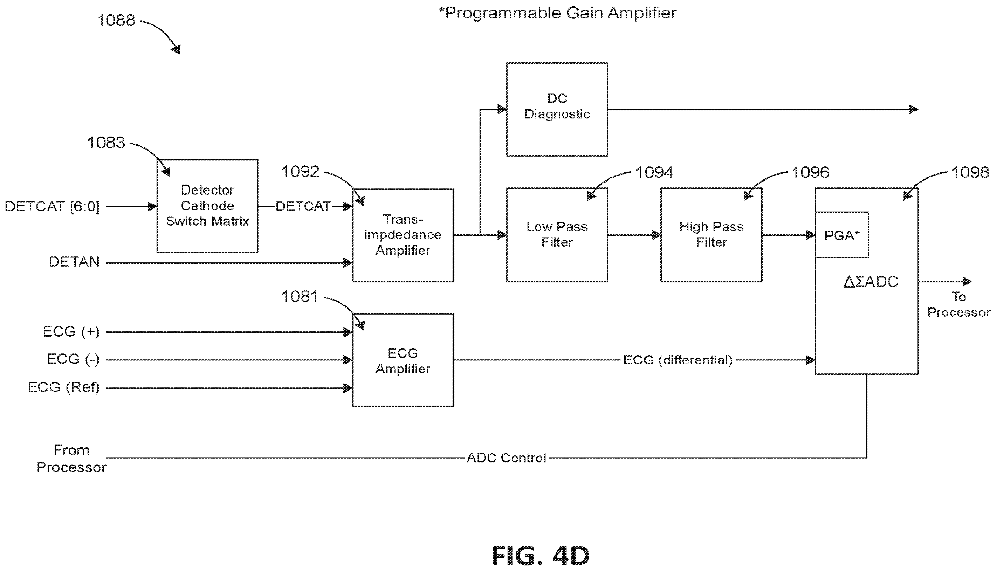

Figure 4D illustrates an example front-end analog signal conditioning circuitry 1088 of the module PCB 116 shown in Figure 4C. The entire front-end circuitry 1088 can be located on a single application-specific integrated circuit (ASIC).

The front-end circuitry 1088 can include a transimpedance amplifier 1092 configured to receive analog signals from the optical sensor including the emitters 104, the detectors 106, and the temperature sensors 102, which can be preprocessed (for example, via a low pass filter 1094 and a high pass filter 1096) before being sent to an analog-digital converter 1098. The analog-digital converter 1098 can output a digital signal based on the analog signals from the optical sensor including the emitters 104, the detectors 106, and the temperature sensors 102 to the system processor 1082 and the calculation processor 1080. The front-end circuitry 1088 can include a detector cathode switch matrix 1083 configured to activate the cathode of the detectors that are selected to be activated. The matrix 1083 can be further configured to deactivate (for example, by short-circuiting) anodes of the detectors that are selected to be deactivated in configurations in which the detectors share a common anode and have different cathodes.

The front-end circuitry 1088 can include an ECG amplifier 1091 configured to receive analog signals from the electrodes 124/125, which can output the amplified analog signals to the analog-digital converter 1098. The amplified analog signals can include an ECG differential between the positive and negative electrodes. The analog-digital converter 1098 can output a digital signal based on the analog signals from the electrodes 124/125 to the system processor 1082 and the calculation processor 1080.

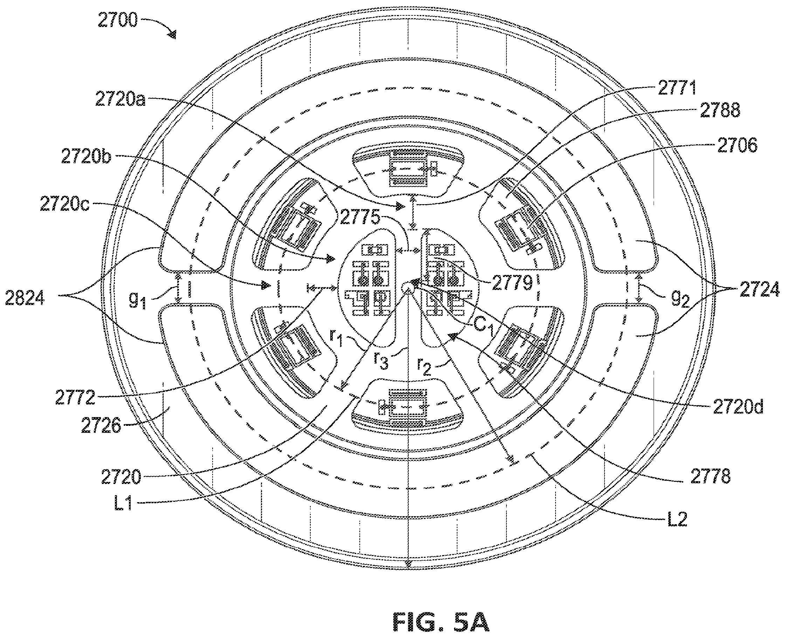

Figure 5A is a front view of an example aspect of a sensor or module 2700. The sensor or module 2700 includes an opaque frame 2726, one or more electrodes 2724, one or more detector chambers 2788, one or more emitter chambers 2778, and a light barrier construct 2720.

The opaque frame 2726 can include one or more materials configured to prevent or block the transmission of light. In some aspects, the opaque frame 2726 may form a single integrated unit. In some aspects, the opaque frame 2726 may be formed of a continuous material. The light barrier construct 2720 can include one or more materials configured to prevent or block the transmission of light. In some aspects, the light barrier construct 2720 may form a single integrated unit. In some aspects, the light barrier construct 2720 may be formed of a continuous material. In some aspects, the light barrier construct 2720 and the opaque frame 2726 may form a single integrated unit. In some aspects, the light barrier construct 2720 and the opaque frame 2726 may be separably connected.

The light barrier construct 2720 may include one or more light barriers, such as light barriers 2720a, 2720b, 2720c, 2720d, which are provided as non-limiting examples. In some aspects, light barriers may be also be referred to as light blocks herein. The light barriers may form one or more portions of the light barrier construct 2720. The light barrier construct 2720 (or light barrier portions thereof) may prevent light from passing therethrough. The light barrier construct 2720 may include spaces between various light barriers which may define one or more chambers (e.g., detector chambers 2788, emitter chambers 2778). In some aspects, the one or more chambers (e.g., detector chambers 2788, emitter chambers 2778) may be enclosed by the light barrier construct 2720 or light barrier portions thereof, a surface of a substrate (e.g., PCB), and a lens or cover. In some aspects, light may only enter the chambers through the lens or cover.

An example of a light barrier is provided with reference to example light barrier 2720a. Light barrier 2720a forms a portion of light barrier construct 2720. Light barrier 2720a may prevent (e.g., block) light from passing therethrough between adjacent chambers. For example, light barrier 2720a may prevent light from passing through the light barrier construct 2720 between an emitter chamber 2778 and a detector chamber 2788. Light barrier 2720a, or portions thereof, may include a width 2771. In some aspects, width 2771 may be less than about 1.85mm. In some aspects, width 2771 may be less than about 1.9mm. In some aspects, width 2771 may be less than about 1.95mm. In some aspects, width 2771 may be about 1.88mm. In some aspects, the width 2771 may be less (e.g., smaller) than length 2779. In some aspects, width 2771 may be less than about 55% of length 2779. In some aspects, width 2771 may be less than about 60% of length 2779. In some aspects, width 2771 may be less than about 65% of length 2779. In some aspects, width 2771 may be about 58.9% of length 2779.

Another example of a light barrier is provided with reference to example light barrier 2720b. Light barrier 2720b forms a portion of light barrier construct 2720. Light barrier 2720b may prevent (e.g., block) light from passing therethrough between adjacent chambers. For example, light barrier 2720b may prevent light from passing through the light barrier construct 2720 between an emitter chamber 2778 and a detector chamber 2788. Light barrier 2720b, or portions thereof, may include a width 2772. In some aspects, width 2772 may be less than about 1.35mm. In some aspects, width 2772 may be less than about 1.40mm. In some aspects, width 2772 may be less than about 1.45mm. In some aspects, width 2772 may be about 1.37mm. In some aspects, the width 2772 may be substantially similar to width 2771. In some aspects, the width 2772 may be less (e.g., smaller) than width 2771. In some aspects, width 2772 may be less than about 70% of width 2771. In some aspects, width 2772 may be less than about 75% of width 2771. In some aspects, width 2772 may be less than about 80% of width 2771. In some aspects, width 2772 may be about 72.9% of width 2771.

Another example of a light barrier is provided with reference to example light barrier 2720c. Light barrier 2720c forms a portion of light barrier construct 2720. Light barrier 2720c may prevent (e.g., block) light from passing therethrough between adjacent chambers. For example, light barrier 2720c may prevent light from passing through the light barrier construct 2720 between adjacent detector chamber 2788.

Another example of a light barrier is provided with reference to example light barrier 2720d. Light barrier 2720d forms a portion of light barrier construct 2720. Light barrier 2720d may prevent (e.g., block) light from passing therethrough between adjacent chambers. For example, light barrier 2720d may prevent light from passing through the light barrier construct 2720 between adjacent emitter chambers 2778. In some aspects, light barrier 2720d may have a width 2775 separating adjacent emitter chambers of less than about 1.30mm. In some aspects, width 2775 may be less than about 1.25mm. In some aspects, width 2775 may be less than about 1.20mm. In some aspects, width 2775 may be about 1.20mm. In some aspects, width 2775 may be substantially similar to width 2772. In some aspects, width 2775 may be less (e.g., smaller) than width 2772. In some aspects, width 2775 may be less than about 95% of width 2772. In some aspects, width 2775 may be less than about 90% of width 2772. In some aspects, width 2775 may be less than about 85% of width 2772. In some aspects, width 2775 may be about 87.6% of width 2772.

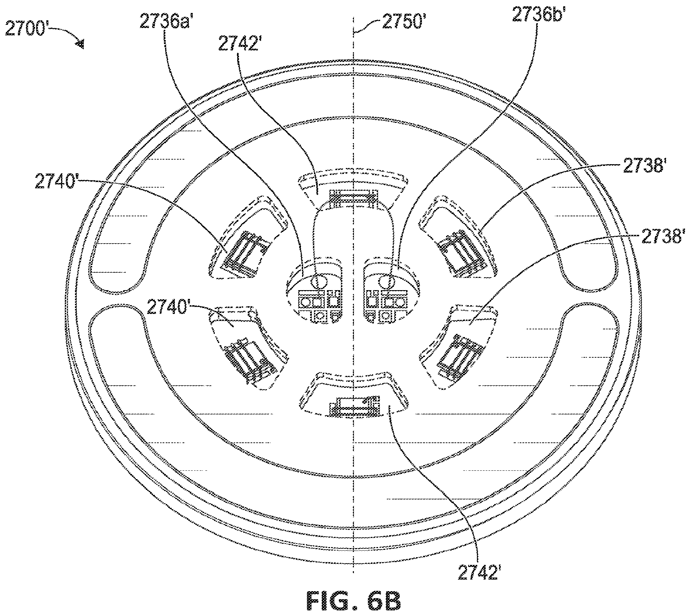

The emitter chambers 2778 are positioned within a central region of the sensor or module 2700. The emitter chambers 2778 may be positioned adjacent to one another across a centerline of the sensor or module 2700 as described in greater detail with reference to Figure 6B, for example. The emitter chambers 2778 may be positioned adjacent the center point C₁. Each of the emitter chambers 2778 may be a similar size and/or shape. The emitter chambers 2778 may be separated, at least in part, by light barrier 2720d of the light barrier construct 2720. In some aspects, as shown in this example, the light barrier 2720d may form an entire distance between emitter chambers 2778. For example, emitter chambers 2778 may be separated by only the light barrier 2720d such that other components (e.g., detectors, detector chambers, etc.) are not positioned between the emitter chambers 2778.

A portion of the emitter chambers 2778 may extend a length 2779 away from center point C₁. In some aspects, length 2779 may be less than about 3.15mm. In some aspects, length 2779 may be less than about 3.20mm. In some aspects, length 2779 may be less than about 3.25mm. In some aspects, length 2779 may be about 3.19mm. In some aspects, the length 2779 may be greater (e.g., larger) than a width of a light barrier separating an emitter chamber from a detector chamber such as width 2771. In some aspects, length 2779 may be greater than about 165% of width 2771. In some aspects, length 2779 may be greater than about 170% of width 2771. In some aspects, length 2779 may be greater than about 175% of width 2771. In some aspects, length 2779 may be about 169.7% of width 2771.

As shown in this example aspect, the detector chambers 2788 are arranged in a substantially circular pattern. Each of the detector chambers 2788 houses a detector 2706 positioned on a substrate (e.g., PCB) in a substantially circular or annular pattern. The detectors 2706 may be positioned in a central region of each of the respective detector chambers 2788. The detector chambers 2788 are arranged along a ring defined by ring L₁. In some aspects, such as shown in this example aspect, detectors 2706 of respective detector chambers 2788 may also be arranged along a same ring along which the detector chambers 2788 are arranged (such as in aspects where detectors are positioned in a central region of respective chambers). The ring L₁ may intersect a central region of the detector chambers 2788. In this example aspect, the ring L₁ encloses an entirety of the emitter chambers 2778 such that the emitter chambers 2778 are positioned within an interior region (e.g., a central region) of the ring L₁ defined by the detector chambers 2788. In some aspects, each of the detector chambers 2788 (and corresponding detectors 2706 within respective detector chambers 2788) may be positioned at a substantially similar or same distance away from the center point C₁ (e.g., center of sensor or module 2700). In some aspects, the detectors 2706 may be rectangular including longer sides and shorter sides. The detectors 2706 may be positioned on a substrate of the sensor or module 2700 such that a long side of each detector is orthogonal to a radius extending away from center point C₁ (e.g., radius r₁, radius r₂, radius r₃). Advantageously, orienting the detectors 2706 on the sensor or module 2700 in an annular arrangement with a long side of the detectors 2706 orthogonal the center point C₁ may improve an accuracy of physiological measurements by ensuring that light from emitters travels along a known path length from emitters to the detectors 2706 and may also reduce processing requirements of the sensor or module 2700 by reducing the amount of variables (e.g., number of light path lengths) required to process in order to determine physiological data.

The electrodes 2724 can include a reference electrode and a negative electrode (and/or a positive electrode). In some aspects, a wearable device such as a watch incorporating the sensor or module 2700 can include another electrode (e.g., a positive electrode) located on the housing of the wearable device configured to make contact with the wearer's skin. In some configurations, a surface of the electrodes 2724 may be flush with a surface of the opaque frame 2726.

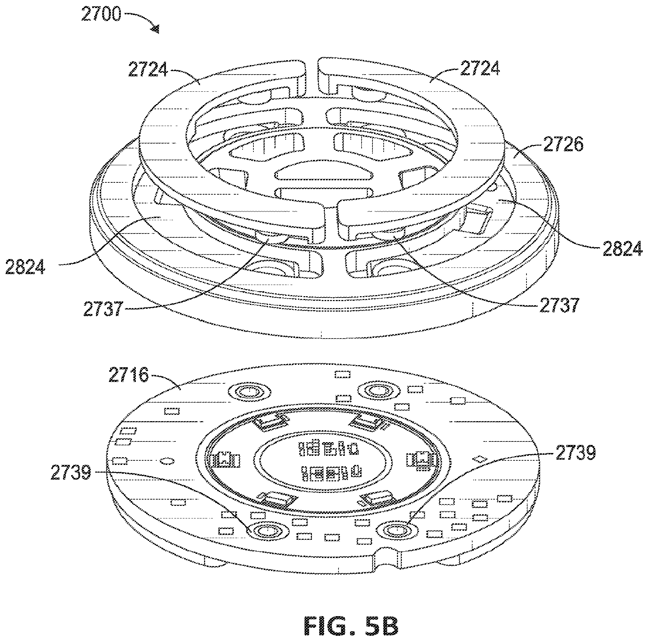

The electrodes 2724 are positioned within or along a portion of the opaque frame 2726 such as shown in Figure 5B for example. In some aspects, the electrodes 2724 can be substantially semicircular. In some aspects, the electrodes 2724 can be substantially semiannular. In the example aspect shown, each of the electrodes 2724 forms a substantial half annulus. Advantageously, an annular shaped electrode may improve contact with the skin of a wearer (e.g., by contacting a diverse area of skin) while simultaneously reducing the amount of surface area of the electrode. In some aspects, each of the electrodes 2724 may be a similar size and/or shape. In some aspects, the electrodes 2724 may be various sizes and/or shapes. In this example aspect, the electrodes 2724 are positioned within the sensor or module 2700 (e.g., within the opaque frame 2726) along ring defined by L₂. In various aspects described herein, the ring L₂ may include various radii which may advantageously provide improved contact between the electrodes 2724 and the skin of a wearer of the device.

The opaque frame 2726 includes one or more gaps (e.g., g₁, g₂) between electrodes 2724. The gaps g₁, g₂, (or other portions of the opaque frame 2726) may electrically insulate each of the electrodes 2724 from one another. Each of the electrodes 2724 includes substantially straight edge along a portion of respective gaps g₁, g₂. In some aspects, the gaps g₁, g₂, may be a similar or a same size. In some aspects, the gaps g₁, g₂, may be a different size than each other. In some aspects, the gaps g₁, g₂, may be less than about 1.6mm. In some aspects, the gaps g₁, g₂, may be less than about 1.65mm. In some aspects, the gaps g₁, g₂, may be less than about 1.7mm. In some aspects, the gaps g₁, g₂, may be about 1.62mm. As discussed above, in some implementations the frame 2726 includes recesses 2824 sized and/or shaped to receive the electrodes 2724. In some implementations, each of such recesses 2824 includes first and second ends, the first ends of the recesses 2824 are separated from one another by gap g₁, and the second ends of the recesses 2824 are separated from one another by gap g₂.

The ring L₁ may be concentric with an outer perimeter of the sensor or module 2700. The ring L₂ may be concentric with an outer perimeter of the sensor or module 2700. The ring L₂ may be concentric with a ring defined by positions of the detector chambers 2788 such as ring L₁. Center point C₁ may define a geometric center of ring L₁. Center point C₁ may define a geometric center of ring L₂. Center point C₁ may define a geometric center of an outer perimeter of the sensor or module 2700. In some aspects, such as shown in Figure 5A, each of L₁, L₂, and an outer perimeter of the sensor or module 2700 are concentric with each other and share a same geometric center shown as C₁.

The ring L₁ may include a radius r₁. In some aspects, radius r₁ may be less than about 6.25mm. In some aspects, radius r₁ may be less than about 6.50mm. In some aspects, radius r₁ may be less than about 6.75mm. In some aspects, radius r₁ may be about 6.34mm. In some aspects, the radius r₁ may be less (e.g., smaller) than radius r₂. In some aspects, radius r₁ may be less than about 55% of r₂. In some aspects, radius r₁ may be less than about 60% of r₂. In some aspects, radius r₁ may be less than about 65% of r₂. In some aspects, radius r₁ may be about 59% of r₂. In some aspects, the radius r₁ may be less (e.g., smaller) than radius r₃. In some aspects, radius r₁ may be less than about 40% of r₃. In some aspects, radius r₁ may be less than about 45% of r₃. In some aspects, radius r₁ may be less than about 50% of r₃. In some aspects, radius r₁ may be about 41.7% of r₃.

The ring L₂ may include a radius r₂. In some aspects, radius r₂ may be less than about 10.5mm. In some aspects, radius r₂ may be less than about 10.75mm. In some aspects, radius r₂ may be less than about 11.0mm. In some aspects, radius r₂ may be about 10.73mm. In some aspects, the radius r₂ may be less (e.g., smaller) than radius r₃. In some aspects, radius r₂ may be less than about 65% of r₃. In some aspects, radius r₂ may be less than about 70% of r₃. In some aspects, radius r₂ may be less than about 75% of r₃. In some aspects, radius r₂ may be about 70.6% of r₃.

In some aspects, the sensor or module 2700 (e.g., an outer perimeter of the sensor or module 2700) may include a radius r₃. In some aspects, radius r₃ may be less than about 14.5mm. In some aspects, radius r₃ may be less than about 15.0mm. In some aspects, radius r₃ may be less than about 15.50mm. In some aspects, radius r₃ may be less than about 16.0mm. In some aspects, radius r₃ may be about 15.19mm.

Figure 5B illustrates an additional example aspect of an optional electrocardiogram (ECG) sensor. The electrocardiogram (ECG) sensor may include a plurality of electrodes 2724 configured to make contact with the wearer's skin. The plurality of electrodes 2724 may be located on the sensor or module 2700. As disclosed herein, the wearable device incorporating the module can include another electrode located on the housing of the wearable device configured to make contact with the wearer's skin.

Figure 5B is an exploded perspective view of an example aspects of a sensor or module 2700. As shown in Figure 5B, the opaque frame 2726 can include recesses (which may also be referred to as "indentations") having the shape and size to accommodate the electrodes 2724 or other components with a suitable shape and size. For example, in some implementations, frame 2726 includes recesses 2824. Recesses 2824 can be sized and/or shaped to receive electrodes 2724. In some implementations, recesses 2824 have a depth (for example, measured from a plane of the frame 2726) that is substantially equal to a thickness of the electrodes 2724. In some implementations, recesses 2824 have a size and/or shape that matches a size and/or shape of the electrodes 2724. For example, in some implementations in which the electrodes have a semi-annular shape (such as that illustrated in at least Figures 5A-5B), the recesses 2824 can have a semi-annular shape.

A front side of the electrodes 2724 can have one or more posts 2737 extending past openings in the opaque frame 2726 into corresponding openings on the substrate 2716. The posts 2737 of the electrodes 2724 can establish an electrical connection with the corresponding openings of the substrate 2716. A plurality of screws (or other types of fasteners) can extend into the corresponding openings of the substrate 2716 from the front side of the substrate 2716 to secure the electrodes 2724 to the sensor or module 2700 by threadedly mating or otherwise with the posts 2737. When a wearer puts the wearable device incorporating the sensor or module 2700 onto the wearer's wrist, the electrodes 2724 can make contact with the wearer's skin.

With continued reference to Figure 5B, the substrate 2716 can include a printed circuit board (PCB). The substrate 2716 can include a conductive liquid adhesive 2739. The conductive liquid adhesive 2739 may be provided on the copper of the substrate 2716. The conductive liquid adhesive 2739 may facilitate conductive electrical connection between the electrodes 2724 and the substrate 2716.

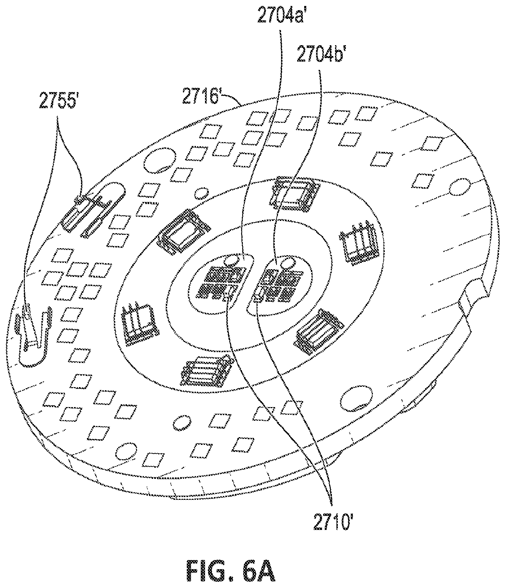

With continued reference to Figure 5B, one or more spring contacts (such as spring contacts 2755' shown in Figure 6A) may be located between the electrodes 2724 and the substrate 2716. The shape, size, and/or number of the spring contacts can vary. The spring contacts can establish an electrical connection between the electrodes 2724 and the substrate 2716. The spring contacts can be biased toward the electrodes 2724 to ensure a firm electrical connection between the spring contacts and the electrodes 2724 and the substrate 2716.

Figure 6A illustrates another example arrangement of an optical sensor, including emitters, detectors, and thermistors, on a sensor or module processor substrate 2716'. As shown in Figure 6A, each of the first and second groups of emitters 2704a', 2704b' can include five emitters (or optionally a different number of emitters as required or desired). Each of the emitters of the first and second groups of emitters 2704a', 2704b' may comprise an LED and can be configured to emit light at various wavelengths such as any of the wavelengths discussed herein, for example, a first wavelength of about 525 nm to about 650 nm (such as about 525 nm or about 580 nm or about 645 nm), a second wavelength from about 620 nm to about 660 nm (such as about 625 nm), a third wavelength from about 650 nm to about 670 nm (such as about 660 nm), a fourth wavelength from about 900 nm to about 910 nm, and a fifth wavelength at about 970 nm. As shown in Figure 6A, the substrate 2716' can include spring contacts 2755' for facilitating physical and/or electrical connection between the substrate 2716' and electrodes (e.g., electrodes 2724 shown in Figure 5B, for example).

Figures 6B-6C illustrate an example physiological parameter measurement sensor or module 2700' and example light paths between emitters and detectors of the module 2700'.

Figure 6B illustrates an example arrangement of emitter and detector chambers of the sensor or module 2700'. As shown, the sensor or module 2700' can include a first emitter chamber 2736a' enclosing a first emitter group comprising one or more emitters, a second emitter chamber 2736b' enclosing a second emitter group comprising one or more emitters, one or more first detector chambers 2740', one or more second detector chambers 2742', and one or more third detector chambers 2738'. In some aspects, each detector chamber may enclose one detector.

The first emitter group of the first emitter chamber 2736a' may comprise the same number and type of emitters as the second emitter group of the second emitter chamber 2736b'. In other words, each emitter of the first emitter group may correspond to an emitter of the same type (e.g., same wavelength) of the second emitter group. The emitters of the first emitter group may be arranged in a configuration that mirrors the emitters of the second emitter group across a centerline 2750' of the sensor or module 2700' as shown in Figure 6B. For example, each emitter of the first group of emitters may be located a distance away from a centerline 2750' of the sensor or module 2700' that is a same distance that a corresponding emitter of the second group of emitters is located away from the centerline 2750' of the sensor or module 2700'. For example, the first and second emitter groups may each include an emitter that emits light of a first wavelength and that are positioned at locations that are mirror images of each other across a centerline 2750' of the sensor or module 2700'. Additionally, the first and second emitter groups may each include an emitter that emits light of a second wavelength and that are positioned at locations that are mirror images of each other across a centerline 2750' of the sensor or module 2700'. Each of the emitters of the first emitter group may correspond to an emitter of the second emitter group located at a mirror image position, and vice versa.

The one or more second detector chambers 2742' may be bisected by a centerline 2750' of the sensor or module 2700'. Each of the detectors of the respective one or more second detector chambers 2742' may be bisected by a centerline 2750' of the sensor or module 2700'. In other words, the one or more second detector chambers 2742' and the respective detectors and the sensor or module 2700' may each share a same (e.g., parallel) centerline 2750'. The sensor or module 2700' may be oriented (e.g., rotated) with respect to the tissue of a wearer in any orientation. In an example implementation where the sensor or module 2700' is worn on a wrist of a user, the sensor or module 2700' may be rotated in any direction with respect to the wrist or forearm of the wearer. In one example configuration, the sensor or module 2700' may be oriented with respect to the forearm (or other body part) of a wearer such that the centerline 2750' of the sensor or module is perpendicular to a line extending along a length of the forearm of the wearer (e.g., from the elbow to the wrist). Advantageously, such a configuration may improve physiological measurements by facilitating light emitted from the emitter chambers and detected at the detector chambers (e.g., light travelling from emitter chamber 2736a' to detector chamber 2738') to penetrate into soft tissue of the wearer (e.g., blood vessels) rather than other tissues such as bone. In another example configuration, the sensor or module 2700' may be oriented with respect to the forearm (or other body part) of a wearer such that the centerline 2750' of the sensor or module is parallel to a line extending along a length of the forearm of the wearer (e.g., from the elbow to the wrist). Advantageously, such a configuration may improve physiological measurements by facilitating light emitted from the emitter chambers and detected at the detector chambers (e.g., light travelling from emitter chamber 2736a' to detector chamber 2742') to penetrate into soft tissue of the wearer (e.g., blood vessels) rather than other tissues such as bone.

As shown in Figure 6B, emitters of the first and second emitter groups that correspond to each other (e.g., emit the same wavelength and mirror each other) may each emit light that travels along respective paths to the detectors of the one or more second detector chambers 2742'. The respective paths of light from the corresponding emitters may be of equal length. This may be because the corresponding emitters are each positioned an equal distance away from a detector of a chamber 2742'. The corresponding emitters may each be an equal distance away from a detector of a chamber 2742' because they are positioned at mirror images of each other across a centerline 2750' of the sensor or module 2700' that bisects the one or more second detector chambers 2472' and respective detectors.

The one or more second detector chambers 2742' and their respective detectors may be used, at least in part, for calibration, for example to characterize the emitters, by providing known information such as a known ratio. For example, information corresponding to a wavelength detected at a detector of a chamber 2742' from an emitter of the first group of emitters may be similar or the same as information corresponding to that wavelength detected at the detector of the chamber 2742' from an emitter of the second group of emitters and a comparison (e.g., subtracting, dividing, etc.) of the information resulting from the first and second groups of emitters may yield a known number such as zero or one because the corresponding emitters from the first and second emitter groups may be an equal distance from the detector of chamber 2742' and light emitted therefrom may travel a same distance to the detector of chamber 2742'. As an example of normalization, ratios of wavelengths detected at detectors of chambers 2738', 2740' may be normalized (e.g., divided by) ratios of wavelengths detected at detectors of chambers 2742'. In instances where the information resulting from detection of light from the first and second groups of emitters is not the same or is substantially different (e.g., as a result of emission intensity variations or other such discrepancies) the information may be adjusted or normalized (e.g., calibrated) to account for such differences. This normalization or on-board calibration or characterization of the emitters may improve accuracy of the physiological measurements and provide for continuous calibration or normalization during measurements. In some aspects, a processor may be configured to calibrate or normalize the physiological parameter measurement of the sensor continuously. In some aspects, a processor may be configured to calibrate or normalize the physiological parameter measurement of the sensor while the optical physiological sensor measures physiological parameters of the wearer.

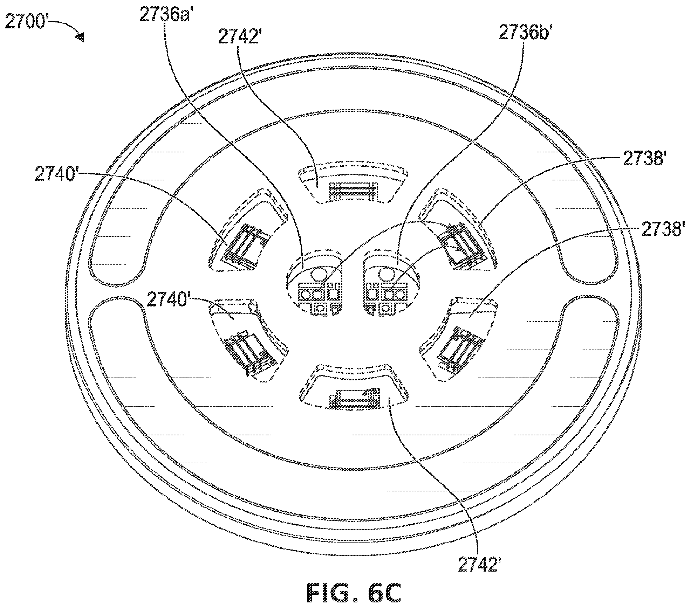

Figure 6C illustrates an example arrangement of emitter and detector chambers of the sensor or module 2700'. As shown, the sensor or module 2700' can include a first emitter chamber 2736a', a second emitter chamber 2736b', one or more first detector chambers 2740', one or more second detector chambers 2742', and one or more third detector chambers 2738', for example as discussed elsewhere herein.

The first and second emitter chambers 2736a', 2736b' may be located at non-equal distances away from each of the chambers of the one or more detector chambers 2738', 2740'. Thus, with respect to each detector chamber of the chambers 2738', 2740', the first and second emitter chamber 2736a', 2736b', may each be a "near" or "far" emitter chamber. In other words, each detector of the detector chambers 2738', 2740' may detect light, of any given wavelength, from both a "near" emitter and a "far" emitter, with the near and far emitters being included in either the first or second emitter group, respectively.

As an example, as shown in Figure 6C, light of a given wavelength may travel along a path from an emitter in the first emitter group to the detector of detector chamber 2738' and light of the same wavelength may travel along a path from an emitter in the second emitter group to the same detector. The light from the first emitter group may travel along a longer path than light from the second emitter group before reaching the detector of chamber 2738'. Thus, for any detector of detector chambers 2738' or 2740', the detector may receive light of a given wavelength from both a near (e.g., proximal) emitter and a far (e.g., distal) emitter. This may not be the case for detectors of chambers 2742' because the first and second emitter groups may each be located a same distance away from any given detector of detector chambers 2742', as described herein.

For convenience, the terms "proximal" and "distal" may be used herein to describe structures relative to any of the detector chambers or their respective detectors. For example, an emitter may be proximal to a detector chamber of the first detector chambers and distal to a detector of the second detector chambers. The term "distal" refers to one or more emitters that are farther away from a detector chamber than at least some of the other emitters. The term "proximal" refers to one or more emitters that are closer to a detector chamber than at least some of the other emitters. The term "proximal emitter" may be used interchangeably with "near emitter" and the term "distal emitter" may be used interchangeably with "far emitter".

A single emitter may be both proximal to one detector and distal to another detector. For example, an emitter may be a proximal emitter relative to a detector of the first detector chambers and may be a distal emitter relative to a detector of the second detector chambers.

Light of a given wavelength that is detected at a detector may provide different information depending on the length of the path it has travelled from the emitter (e.g., along a long path from a distal emitter or along a short path from a proximal emitter). For example, light that has travelled along a long path from a distal emitter may penetrate deeper into the tissue of a wearer of the device and may provide information pertaining to pulsatile blood flow or constituents. The use of a proximal and distal emitter for each wavelength may improve accuracy of the measurement, for example information pertaining to light that has travelled along a long path from a distal emitter may be normalized by (e.g., divided by) information pertaining to light that has travelled along a short path from a proximal emitter.

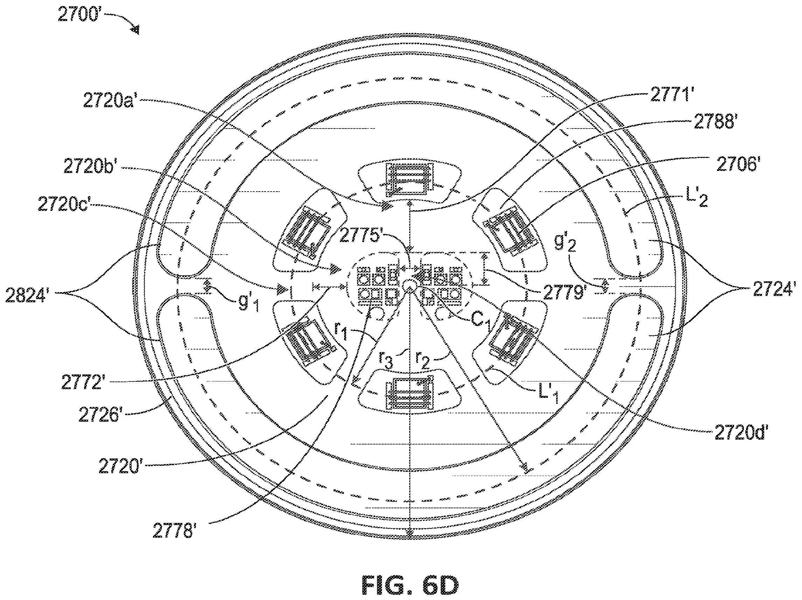

Figures 6D-6G illustrate an example physiological parameter measurement sensor or module 2700' and example light barriers or light blocks between emitter and detector chambers of the module 2700'.

Figure 6D is a front view of an example aspect of a sensor or module 2700'. The sensor or module 2700' includes an opaque frame 2726', one or more electrodes 2724', one or more detector chambers 2788', one or more emitter chambers 2778', and a light barrier construct 2720'.

The opaque frame 2726' can include one or more materials configured to prevent or block the transmission of light. In some aspects, the opaque frame 2726' may form a single integrated unit. In some aspects, the opaque frame 2726' may be formed of a continuous material. The light barrier construct 2720' can include one or more materials configured to prevent or block the transmission of light. In some aspects, the light barrier construct 2720' may form a single integrated unit. In some aspects, the light barrier construct 2720' may be formed of a continuous material. In some aspects, the light barrier construct 2720' and the opaque frame 2726' may form a single integrated unit. In some aspects, the light barrier construct 2720' and the opaque frame 2726' may be separably connected.

The light barrier construct 2720' may include one or more light barriers, such as light barriers 2720a', 2720b', 2720c', 2720d', which are provided as non-limiting examples. In some aspects, light barriers may be also be referred to as light blocks herein. The light barriers may form one or more portions of the light barrier construct 2720'. The light barrier construct 2720' (or light barrier portions thereof) may prevent light from passing therethrough. The light barrier construct 2720' may include spaces between various light barriers which may define one or more chambers (e.g., detector chambers 2788', emitter chambers 2778'). In some aspects, the one or more chambers (e.g., detector chambers 2788', emitter chambers 2778') may be enclosed by the light barrier construct 2720' or light barrier portions thereof, a surface of a substrate (e.g., PCB), and a lens or cover. In some aspects, light may only enter the chambers through the lens or cover.

An example of a light barrier is provided with reference to example light barrier 2720a'. Light barrier 2720a' forms a portion of light barrier construct 2720'. Light barrier 2720a' may prevent (e.g., block) light from passing therethrough between adjacent chambers. For example, light barrier 2720a' may prevent light from passing through the light barrier construct 2720' between an emitter chamber 2778' and a detector chamber 2788'. Light barrier 2720a', or portions thereof, may include a width 2771'. In some aspects, width 2771' may be less than about 3.30mm. In some aspects, width 2771' may be less than about 3.25mm. In some aspects, width 2771' may be less than about 3.20mm. In some aspects, width 2771' may be about 3.24mm. In some aspects, the width 2771' may be greater (e.g., larger) than length 2779'. In some aspects, width 2771' may be less than about 165% of length 2779'. In some aspects, width 2771' may be less than about 160% of length 2779'. In some aspects, width 2771' may be less than about 155% of length 2779'. In some aspects, width 2771' may be about 160% of length 2779'. Advantageously, a greater width 2771' (e.g., a wider light barrier separating the emitter chambers 2778' and detector chambers 2788') may cause light emitted from the emitter chambers 2778' to travel a greater distance before reaching the detector chambers 2788'. Light that travels a greater distance may penetrate deeper into the tissue of the wearer which may improve accuracy of a physiological measurement.

Another example of a light barrier is provided with reference to example light barrier 2720b'. Light barrier 2720b' forms a portion of light barrier construct 2720'. Light barrier 2720b' may prevent (e.g., block) light from passing therethrough between adjacent chambers. For example, light barrier 2720b' may prevent light from passing through the light barrier construct 2720' between an emitter chamber 2778' and a detector chamber 2788'. Light barrier 2720b', or portions thereof, may include a width 2772'. In some aspects, width 2772' may be less than about 1.65mm. In some aspects, width 2772' may be less than about 1.60mm. In some aspects, width 2772' may be less than about 1.55mm. In some aspects, width 2772' may be about 1.59mm. In some aspects, the width 2772' may be less (e.g., smaller) than width 2771'. In some aspects, width 2772' may be less than about 60% of width 2771'. In some aspects, width 2772' may be less than about 55% of width 2771'. In some aspects, width 2772' may be less than about 50% of width 2771'. In some aspects, width 2772' may be about 49% of width 2771'. Advantageously, a greater width 2772' may cause light emitted from the emitter chambers 2778' to travel a greater distance before reaching the detector chambers 2788'. Light that travels a greater distance may penetrate deeper into the tissue of the wearer which may improve accuracy of a physiological measurement

Another example of a light barrier is provided with reference to example light barrier 2720c'. Light barrier 2720c' forms a portion of light barrier construct 2720'. Light barrier 2720c' may prevent (e.g., block) light from passing therethrough between adjacent chambers. For example, light barrier 2720c' may prevent light from passing through the light barrier construct 2720' between adjacent detector chamber 2788'.

Another example of a light barrier is provided with reference to example light barrier 2720d'. Light barrier 2720d' forms a portion of light barrier construct 2720'. Light barrier 2720d' may prevent (e.g., block) light from passing therethrough between adjacent chambers. For example, light barrier 2720d' may prevent light from passing through the light barrier construct 2720' between adjacent emitter chambers 2778'. In some aspects, light barrier 2720d' may have a width 2775' separating adjacent emitter chambers of less than about 1.40mm. In some aspects, width 2775' may be less than about 1.35mm. In some aspects, width 2775' may be less than about 1.30mm. In some aspects, width 2775' may be about 1.28mm. In some aspects, width 2775' may be less (e.g., smaller) than width 2771'. In some aspects, width 2775' may be less than about 50% of width 2771'. In some aspects, width 2775' may be less than about 45% of width 2771'. In some aspects, width 2775' may be less than about 40% of width 2771'. In some aspects, width 2775' may be less than about 35% of width 2771'. In some aspects, width 2775' may be about 39.5% of width 2771'.

The emitter chambers 2778' are positioned within a central region of the sensor or module 2700'. The emitter chambers 2778' may be positioned adjacent to one another across a centerline of the sensor or module 2700' as described in greater detail with reference to Figure 6B, for example. The emitter chambers 2778' may be positioned adjacent the center point C'₁. Each of the emitter chambers 2778' may be a similar size and/or shape. The emitter chambers 2778' may be separated, at least in part, by light barrier 2720d' of the light barrier construct 2720'. In some aspects, as shown in this example, the light barrier 2720d' may form an entire distance between emitter chambers 2778'. For example, emitter chambers 2778' may be separated by only the light barrier 2720d' such that other components (e.g., detectors, detector chambers, etc.) are not positioned between the emitter chambers 2778'.

A portion of the emitter chambers 2778' may extend a length 2779' away from center point C'₁. In some aspects, length 2779' may be less than about 2.15mm. In some aspects, length 2779' may be less than about 2.10mm. In some aspects, length 2779' may be less than about 2.05mm. In some aspects, length 2779' may be less than about 2.0mm. In some aspects, length 2779' may be about 2.02mm. In some aspects, the length 2779' may be less (e.g., smaller) than a width of a light barrier separating an emitter chamber from a detector chamber such as width 2771'. In some aspects, length 2779' may be less than about 70% of width 2771'. In some aspects, length 2779' may be less than about 65% of width 2771'. In some aspects, length 2779' may be less than about 60% of width 2771'. In some aspects, length 2779' may be about 62.3% of width 2771'.

As shown in this example aspect, the detector chambers 2788' are arranged in a substantially circular pattern. Each of the detector chambers 2788' houses a detector 2706' positioned on a substrate (e.g., PCB) in a substantially circular or annular pattern. The detectors 2706' may be positioned in a central region of each of the respective detector chambers 2788'. The detector chambers 2788' are arranged along a ring defined by ring L'₁. In some aspects, such as shown in this example aspect, detectors 2706' of respective detector chambers 2788' may also be arranged along a same ring along which the detector chambers 2788' are arranged (such as in aspects where detectors are positioned in a central region of respective chambers). The ring L'₁ may intersect a central region of the detector chambers 2788'. In this example aspect, the ring L'₁ encloses an entirety of the emitter chambers 2778' such that the emitter chambers 2778' are positioned within an interior region (e.g., a central region) of the ring L'₁ defined by the detector chambers 2788'. In some aspects, each of the detector chambers 2788' (and corresponding detectors 2706' within respective detector chambers 2788') may be positioned at a substantially similar or same distance away from the center point C'₁ (e.g., center of sensor or module 2700'). In some aspects, the detectors 2706' may be rectangular including longer sides and shorter sides. The detectors 2706' may be positioned on a substrate of the sensor or module 2700' such that a long side of each detector is orthogonal to a radius extending away from center point C'₁ (e.g., radius r'₁, radius r'₂, radius r'₃). Advantageously, orienting the detectors 2706' on the sensor or module 2700' in an annular arrangement with a long side of the detectors 2706' orthogonal the center point C'₁ may improve an accuracy of physiological measurements by ensuring that light from emitters travels along a known path length from emitters to the detectors 2706' and may also reduce processing requirements of the sensor or module 2700' by reducing the amount of variables (e.g., number of light path lengths) required to process in order to determine physiological data.

The electrodes 2724' can include a reference electrode and a negative electrode (and/or a positive electrode). In some aspects, a wearable device such as a watch incorporating the sensor or module 2700' can include another electrode (e.g., a positive electrode) located on the housing of the wearable device configured to make contact with the wearer's skin. In some configurations, a surface of the electrodes 2724' may be flush with a surface of the opaque frame 2726'.

The electrodes 2724' are positioned within or along a portion of the opaque frame 2726' such as shown in Figure 6D for example. In some aspects, the electrodes 2724' can be substantially semicircular. In some aspects, the electrodes 2724' can be substantially semi-annular. In the example aspect shown, each of the electrodes 2724' forms a substantial half annulus. Advantageously, an annular shaped electrode may improve contact with the skin of a wearer (e.g., by contacting a diverse area of skin) while simultaneously reducing the amount of surface area of the electrode. In some aspects, each of the electrodes 2724' may be a similar size and/or shape. In some aspects, the electrodes 2724' may be various sizes and/or shapes. In this example aspect, the electrodes 2724' are positioned within the sensor or module 2700' (e.g., within the opaque frame 2726') along ring defined by L'₂. In various aspects described herein, the ring L'₂ may include various radii which may advantageously provide improved contact between the electrodes 2724' and the skin of a wearer of the device. In some implementations, frame 2726' includes recesses 2824' that are sized and/or shaped to accommodate the electrodes 2724'. In some implementations, recesses 2824' have a depth (for example, measured from a plane of the frame 2726') that is substantially equal to a thickness of the electrodes 2724'. In some implementations, recesses 2824' have a size and/or shape that matches a size and/or shape of the electrodes 2724'. For example, in some implementations in which the electrodes have a semi-annular shape, the recesses 2824' can have a semi-annular shape.

The opaque frame 2726' includes one or more gaps (e.g., g'₁, g'₂) between electrodes 2724'. The gaps g'₁, g'₂, (or other portions of the opaque frame 2726') may electrically insulate each of the electrodes 2724' from one another. Each of the electrodes 2724' includes a curved edge along a portion of respective gaps g'₁, g'₂. In some aspects, the gaps g'₁, g'₂, may be a similar or a same size. In some aspects, the gaps g'₁, g'₂, may be a different size than each other. In some aspects, the gaps g'₁, g'₂, may be less than about 0.6mm. In some aspects, the gaps g'₁, g'₂, may be less than about 0.65mm. In some aspects, the gaps g'₁, g'₂, may be less than about 0.7mm. In some aspects, the gaps g'₁, g'₂, may be about 0.62mm. As discussed above, in some implementations the frame 2726' includes recesses 2824' sized and/or shaped to receive the electrodes 2724'. In some implementations, each of such recesses 2824' includes first and second ends, the first ends of the recesses 2824' are separated from one another by gap g'₁, and the second ends of the recesses 2824' are separated from one another by gap g'₂ (see Figure 6D). In some implementations, such as that illustrated in at least Figure 6D, ends of the recesses 2824' and/or ends of electrodes 2724' have a rounded shape.

The ring L'₁ may be concentric with an outer perimeter of the sensor or module 2700'. The ring L'₂ may be concentric with an outer perimeter of the sensor or module 2700'. The ring L'₂ may be concentric with a ring defined by positions of the detector chambers 2788' such as ring L'₁. Center point C'₁ may define a geometric center of ring L'₁. Center point C'₁ may define a geometric center of ring L'₂. Center point C'₁ may define a geometric center of an outer perimeter of the sensor or module 2700'. In some aspects, such as shown in Figure 6D, each of L'₁, L'₂, and an outer perimeter of the sensor or module 2700' are concentric with each other and share a same geometric center shown as C'₁.

The ring L'₁ may include a radius r'₁. In some aspects, radius r'₁ may be less than about 6.5mm. In some aspects, radius r'₁ may be less than about 6.45mm. In some aspects, radius r'₁ may be less than about 6.40mm. In some aspects, radius r'₁ may be about 6.40mm. In some aspects, the radius r'₁ may be less (e.g., smaller) than radius r'₂. In some aspects, radius r'₁ may be less than about 60% of r'₂. In some aspects, radius r'₁ may be less than about 55% of r'₂. In some aspects, radius r'₁ may be less than about 50% of r'₂. In some aspects, radius r'₁ may be about 50.9% of r'₂. In some aspects, the radius r'₁ may be less (e.g., smaller) than radius r'₃. In some aspects, radius r'₁ may be less than about 40% of r'₃. In some aspects, radius r'₁ may be less than about 45% of r'₃. In some aspects, radius r'₁ may be less than about 50% of r'₃. In some aspects, radius r'₁ may be about 42% of r'₃.

The ring L'₂ may include a radius r'₂. In some aspects, radius r'₂ may be less than about 13mm. In some aspects, radius r'₂ may be less than about 12.75mm. In some aspects, radius r'₂ may be less than about 12.5mm. In some aspects, radius r'₂ may be about 12.59mm. In some aspects, the radius r'₂ may be less (e.g., smaller) than radius r'₃. In some aspects, radius r'₂ may be less than about 80% of r'₃. In some aspects, radius r'₂ may be less than about 85% of r'₃. In some aspects, radius r'₂ may be less than about 90% of r'₃. In some aspects, radius r'₂ may be about 82.7% of r'₃.

In some aspects, the sensor or module 2700' (e.g., an outer perimeter of the sensor or module 2700') may include a radius r'₃. In some aspects, radius r'₃ may be less than about 15mm. In some aspects, radius r'₃ may be less than about 15.0mm. In some aspects, radius r'₃ may be less than about 15.25mm. In some aspects, radius r'₃ may be less than about 15.5mm. In some aspects, radius r'₃ may be about 15.22mm.

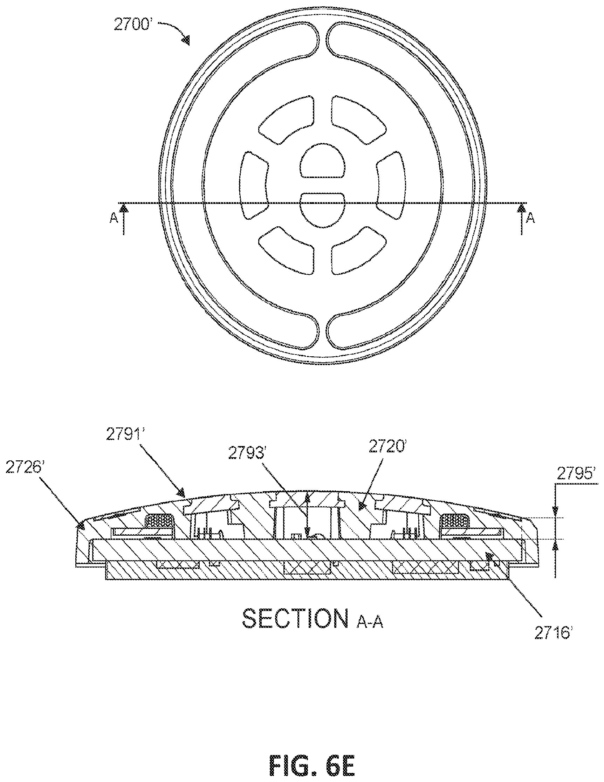

Figure 6E is a side cutaway view of an example aspect of a sensor or module 2700'. The sensor or module 2700' includes a barrier construct 2720', an outer surface 2791', and a substrate 2716'. The outer surface 2791' may include light barrier construct portions, lens portions, opaque frame portions, and/or electrode portions. The outer surface 2791' of the sensor or module 2700' may face and/or contact the skin of a wearer and may include a generally convex curvature shape. When the sensor or module 2700' is worn by the wearer, the outer surface 2791' (at least a portion of which may be comprise electrodes) can be pressed onto the skin of the wearer and the skin or tissue of the wearer can conform around the convex curvature. The contact between the outer surface 2791' and the tissue of the wearer can leave negligible or no air gaps between the tissue and the outer surface 2791' which can ensure maximal and/or continual contact between the users' skin and sensors, such as electrodes. A central region of the sensor or module 2700' may have a height 2793'. For example, the height of the light barrier construct 2720' at a central region of the sensor or module 2700' may correspond to height 2793'. The height 2793' may be a maximum distance the outer surface 2791' extends perpendicularly away from the substrate 2716' (e.g., toward the skin of a wearer). An outer region (e.g., along a perimeter of the substrate 2716') of the sensor or module 2700' may have a height 2795'. For example, the height of the light barrier construct 2720' and/or opaque frame 2726' at an outer region of the sensor or module 2700' may correspond to height 2795'. The height 2795' may be a minimum distance the outer surface 2791' extends perpendicularly away from the substrate 2716' (e.g., toward the skin of a wearer).

In some aspects, height 2793' may be less than about 2.95mm. In some aspects, height 2793' may be less than about 2.90mm. In some aspects, height 2793' may be less than about 2.85mm. In some aspects, height 2793' may be less than about 2.80mm. In some aspects, height 2793' may be about 2.85mm. In some aspects, height 2793' may be less than about 2.70mm. In some aspects, height 2793' may be less than about 2.65mm. In some aspects, height 2793' may be less than about 2.60mm. In some aspects, height 2793' may be less than about 2.55mm. In some aspects, height 2793' may be about 2.58mm.

In some aspects, height 2795' may be less than about 1.40mm. In some aspects, height 2795' may be less than about 1.35mm. In some aspects, height 2795' may be less than about 1.30mm. In some aspects, height 2795' may be less than about 1.25mm. In some aspects, height 2795' may be about 1.29mm. In some aspects, height 2795' may be less than about 1.90mm. In some aspects, height 2795' may be less than about 1.85mm. In some aspects, height 2795' may be less than about 1.80mm. In some aspects, height 2795' may be less than about 1.75mm. In some aspects, height 2795' may be about 1.78mm.

In some aspects, the height 2793' may be greater (e.g., larger) than height 2795'. In some aspects, height 2793' may be less than about 230% of height 2795'. In some aspects, height 2793' may be less than about 225% of height 2795'. In some aspects, height 2793' may be less than about 220% of height 2795'. In some aspects, height 2793' may be less than about 215% of height 2795'. In some aspects, height 2793' may be about 221% of height 2795'. In some aspects, height 2793' may be less than about 155% of height 2795'. In some aspects, height 2793'may be less than about 150% of height 2795'. In some aspects, height 2793' may be less than about 145% of height 2795'. In some aspects, height 2793'may be less than about 140% of height 2795'. In some aspects, height 2793' may be about 145% of height 2795'.

Advantageously, a greater height 2793' (and/or greater ratio of height 2793' to 2795') (for example, a taller light barrier at a central region of the sensor or module 2700 may cause light emitted from emitter chambers to travel a greater distance before reaching the detector chambers. Light that travels a greater distance may penetrate deeper into the tissue of the wearer which may improve accuracy of a physiological measurement. A smaller height 2793' (and/or smaller ratio of height 2793' to 2795') may reduce discomfort to the wearer wearing the wearable device 10 or may reduce obstruction to blood flow of the wearer by reducing the amount of pressure the wearable device places on the wearer. The height 2793' and/or height 2795' may be selected to balance the above-mentioned considerations such as increasing the depth which light penetrates into the tissue and reducing discomfort or blood flow obstruction of the wearer.

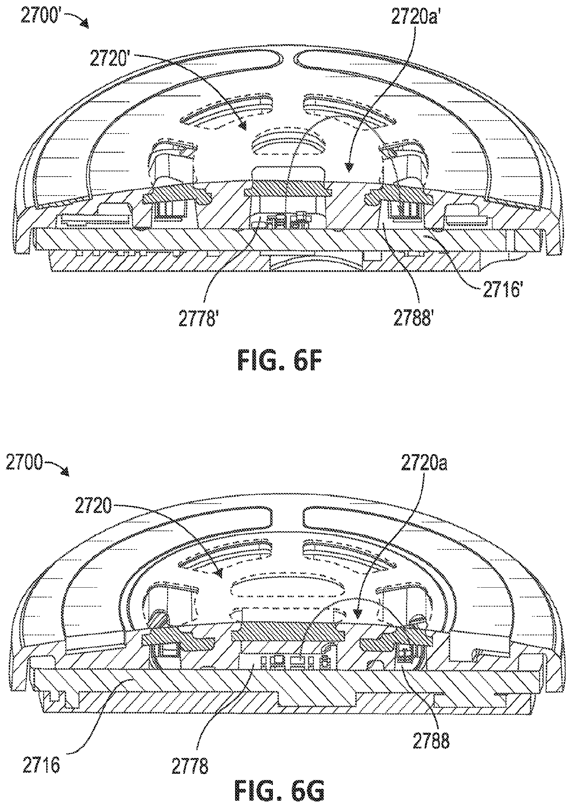

Figure 6F and Figure 6G illustrate two example aspects of a sensor or module 2700' with different light barrier construct configurations. Figures 6F and 6G also show an example light path from an emitter chamber to a detector chamber. The light barrier construct 2720' (or portions thereof) shown in the example aspect of Figure 6F may be taller (e.g., extending away from a surface of the substrate 2716) and/or wider than the light barrier construct 2720 (or portions thereof) shown in the example aspect of Figure 6G. The greater height and/or width of the light barrier construct 2720' in the aspect of Figure 6F may cause the light emitted from an emitter chamber 2778' to travel a greater distance before reaching a detector chamber and thus penetrate deeper into the tissue of the wearer than in the aspect of Figure 6G. Thus, adjusting the height and/or width of the light barrier construct may affect the path the light travels from the emitter chamber to the detector chamber which may affect an accuracy of a physiological measurement. The height and/or width of the light barrier construct may be adjusted, according to various aspects, as required or desired.

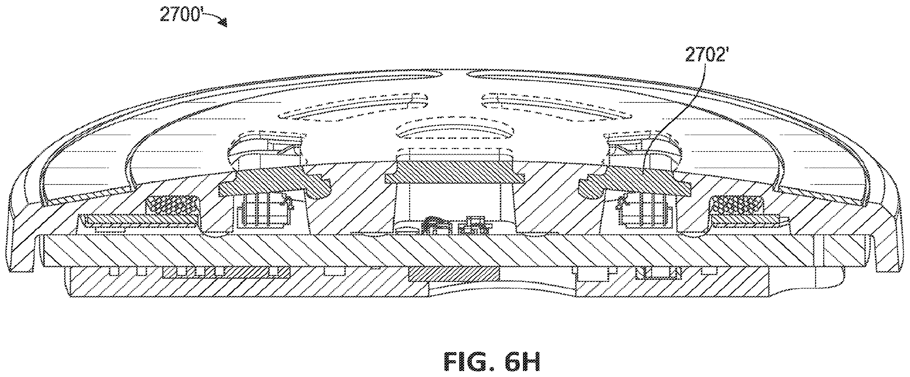

Figure 6H illustrates a cutaway side view of an example sensor or module 2700' showing light transmissive lens(es) or cover(s) 2702' and light diffusing material. The light diffusing materials can be included in one or more of the emitter or detector chambers to improve distribution of emitted light and/or detected light. The diffusing materials or encapsulant, can include, for example, microspheres or glass microspheres. The encapsulant can eliminate air gaps between the surface of the light transmissive cover 2702' and the emitters and/or the detectors. The encapsulant can be included around the emitters to more evenly spread the emitted light, causing the emitted light to appear to be emitted from an entire emitter chamber rather than from a point source (that is, a single LED emitter) if the encapsulant were absent. The light transmissive lens(es) or cover(s) 2702' may include polycarbonate.

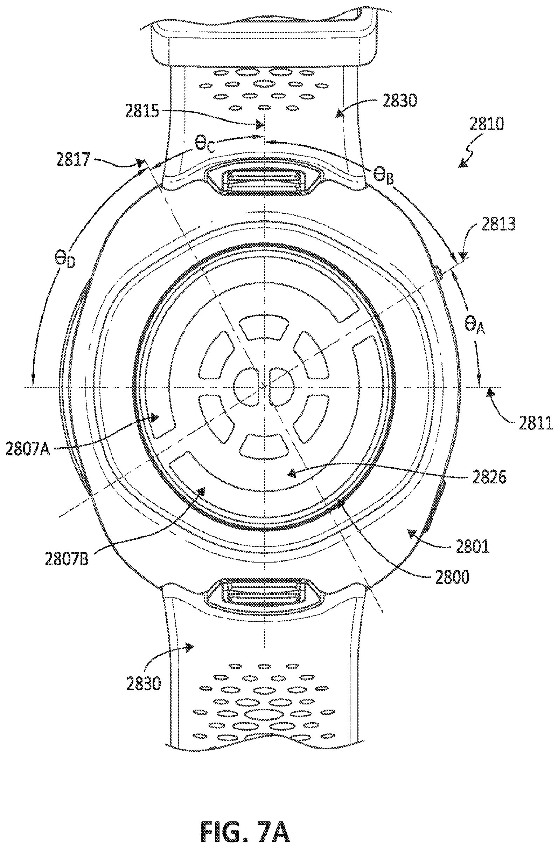

Figure 7A illustrates an example wearable device 2810. The wearable device 2810 can include similar structural and/or operational features as any of the other example wearable devices shown and/or described herein such as wearable device 10. The wearable device 2810 can include strap(s) 2830, a device housing 2801, and a sensor or module 2800. The sensor or module 2800 can include similar structural and/or operational features as any of the other example sensor or modules shown and/or described herein such as sensor or module 100, sensor or module 2700, and/or sensor or module 2700'. The sensor or module 2800 can include a frame 2826 and electrodes 2807A, 2807B. Electrodes 2807A, 2807B can include similar structural and/or operational features as any of the other example electrodes shown and/or described herein such as electrodes 124/125, electrodes 2724, and/or electrodes 2724'. Electrodes 2807A, 2807B (and/or any of the other example electrodes shown and/or described herein) may be ECG electrodes. The electrodes 2807 may be positioned within or along a portion of the frame 2826. A surface of the electrodes 2807 may be flush with a surface of the frame 2826.

The sensor or module 2800 may be disposed within a portion of the device housing 2801. The sensor or module 2800 may face a surface of the user's skin and may contact the user's skin when the wearable device 2810 is worn by the user. The sensor or module 2800 may protrude a distance away from the device housing 2801 which may facilitate contact between the sensor or module 2800 and the user's skin which may improve physiological measurements.

Electrode 2807A may be a positive electrode. The electrode 2807A may be a negative electrode. The electrode 2807A may be a reference electrode. Electrode 2807B may be a positive electrode. The electrode 2807B may be a negative electrode. The electrode 2807B may be a reference electrode. In some implementations, the electrode 2807A may be a positive or negative electrode and the electrode 2807B may be a positive or a negative electrode. In some implementations, the electrode 2807A may be either positive or negative electrode and the electrode 2807B may be a reference electrode. In some implementations, the electrode 2807B may be either positive or negative electrode and the electrode 2807A may be a reference electrode. The device 2810 can include another electrode (e.g., a third electrode), such as electrode 2807C which may be positioned on a portion of housing 2801. Electrode 2807C may be positioned on any portion of housing 2801 such as a top, bottom, left side, right side, etc. In some implementations, the electrode 2807C may comprise a portion of the housing 2801 extending around an entire perimeter of the display 2812. A user may selectively contact electrode 2807C, which may be on portion of the wearable device 2810 that is opposite the electrodes 2807A, 2807B to effectuate an ECG measurement. An electrically insulating material 127 can separate the electrode 2807C from the remainder of the housing 2801 and/or from other electrodes on a physiological sensor module. When the wearer wants to make a measurement the wearer can press on or touch the electrode 2807C using the wearer's finger or another part of the wearer's body such that the wearer's skin makes contact with the electrode 2807C.

Example axes are illustrated as superimposed on the example wearable device 2810 shown in Figure 7A. Axis 2811 may be parallel with a line extending along the length of a forearm of a user when the wearable device 2810 is worn by the user. For example, axis 2811 may be substantially parallel with a line extending from a user's elbow to a user's wrist when the wearable device 2810 is worn by the user. Axis 2811 may be orthogonal to a line extending along a length of the strap(s) 2830. Axis 2815 may be orthogonal to a line extending along the length of a forearm of a user when the wearable device 2810 is worn by the user. For example, axis 2815 may be substantially orthogonal with a line extending from a user's elbow to a user's wrist when the wearable device 2810 is worn by the user. Axis 2815 may be parallel with a line extending along a length of the strap(s) 2830. Axis 2811 and axis 2815 may be orthogonal to each other.

Axis 2811 may bisect the sensor or module 2800 and/or the wearable device 2810. For example, a center of mass of the sensor or module 2800 and/or the wearable device 2810 may lie on the axis 2811. Axis 2815 may bisect the sensor or module 2800 and/or the wearable device 2810. For example, a center of mass of the sensor or module 2800 and/or the wearable device 2810 may lie on the axis 2815.

Electrode 2807A may be symmetrical with electrode 2807B about axis 2813. Axis 2813 may not intersect electrode 2807A. Axis 2813 may not intersect electrode 2807B. Electrode 2807A may be symmetrical with itself about axis 2817. Electrode 2807B may be symmetrical with itself about axis 2817. Axis 2817 may intersect electrode 2807A. For example, axis 2817 may bisect electrode 2807A. Axis 2817 may intersect electrode 2807B. For example, axis 2817 may bisect electrode 2807B. Axis 2813 and axis 2817 may be orthogonal to each other.

Electrode 2807A may be annular. Electrode 2807A may be semi-annular. Electrode 2807A may form a substantially half-annulus. Electrode 2807B may be annular. Electrode 2807B may be semi-annular. Electrode 2807B may form a substantially half-annulus. In some implementations, electrode 2807A may be a same shape and/or size as electrode 2807B. In some implementations, electrode 2807A may be a different shape and/or size than electrode 2807B. Electrode 2807A may be a mirror image of electrode 2807B across axis 2813. An entirety of electrode 2807A may be located on a portion of the wearable device 2810 that is opposite, about axis 2813, a portion of the wearable device 2810 on which an entirety of electrode 2807B is located. Electrode 2807A may contact a different location of a user's skin than electrode 2807A. For example, electrode 2807A may contact a portion of a user's skin that is on an opposite side of axis 2813 from a portion of the user's skin that electrode 2807B contacts. Advantageously, this may improve an measurement, at least because electrodes 2807A and 2807B contact different portions of the user's skin, which may improve signal-to-noise ratio, signal artifact detection, or the like such as by providing a portion of the skin to measure a reference signal that is non-redundant of portions of the skin used to measure a positive or negative signal.

Axis 2811 may intersect electrode 2807A. Axis 2811 may not bisect electrode 2807A. Axis 2815 may intersect electrode 2807A. Axis 2815 may not bisect electrode 2807A. Axis 2811 may intersect electrode 2807B. Axis 2811 may not bisect electrode 2807B. Axis 2815 may intersect electrode 2807B. Axis 2815 may not bisect electrode 2807B.

The center of mass of electrode 2807A may lie on axis 2817. The center of mass of electrode 2807A may not lie on axis 2811 or axis 2815. The center of mass of electrode 2807B may lie on axis 2817. The center of mass of electrode 2807B may not lie on axis 2811 or axis 2815. The center of mass of electrode 2807A may be displaced from the center of mass of electrode 2807B. The center of mass of electrode 2807A and/or electrode 2807B may be displaced from a center of mass of the sensor or module 2800 and/or the wearable device 2810. The center of mass of electrode 2807A may be located at a mirror image of the center of mass of electrode 2807B across the axis 2813.

Axis 2813 may be rotated from axis 2811 by angle ΘA. Angle ΘA may be between 0 degrees and 90 degrees. In some implementations, angle ΘA may be less than about 10 degrees, less than about 20 degrees, less than about 30 degrees, less than about 40 degrees, less than about 50 degrees, less than about 60 degrees, less than about 70 degrees, less than about 80 degrees, or less than about 90 degrees. In some implementations, angle ΘA may be about 45 degrees.

Axis 2815 may be rotated from axis 2813 by angle ΘB. Angle ΘB may be between 0 degrees and 90 degrees. In some implementations, angle ΘB may be less than about 10 degrees, less than about 20 degrees, less than about 30 degrees, less than about 40 degrees, less than about 50 degrees, less than about 60 degrees, less than about 70 degrees, less than about 80 degrees, or less than about 90 degrees. In some implementations, angle ΘB may be about 45 degrees.

Axis 2817 may be rotated from axis 2815 by angle ΘC. Angle ΘC may be between 0 degrees and 90 degrees. In some implementations, angle ΘC may be less than about 10 degrees, less than about 20 degrees, less than about 30 degrees, less than about 40 degrees, less than about 50 degrees, less than about 60 degrees, less than about 70 degrees, less than about 80 degrees, or less than about 90 degrees. In some implementations, angle ΘC may be about 45 degrees.

Axis 2817 may be rotated from axis 2811 by angle ΘD. Angle ΘD may be between 0 degrees and 90 degrees. In some implementations, angle ΘD may be less than about 10 degrees, less than about 20 degrees, less than about 30 degrees, less than about 40 degrees, less than about 50 degrees, less than about 60 degrees, less than about 70 degrees, less than about 80 degrees, or less than about 90 degrees. In some implementations, angle ΘD may be about 45 degrees.

In some implementations, angle ΘB may be greater than angle ΘA. In some implementations, angle ΘD may be greater than angle ΘC. In some implementations, angle ΘD may include the same number of degrees as angle ΘB. In some implementations, angle ΘC may include the same number of degrees as angle ΘA. In some implementations, angle ΘA plus angle ΘB may equal 90 degrees. In some implementations, angle ΘC plus angle ΘD may equal 90 degrees. Axis 2813 may intersect axis 2811 and/or axis 2815. For example, axis 2813 may not be parallel with axis 2811 and/or axis 2815. Axis 2817 may intersect axis 2811 and/or axis 2815. For example, axis 2817 may not be parallel with axis 2811 and/or axis 2815.

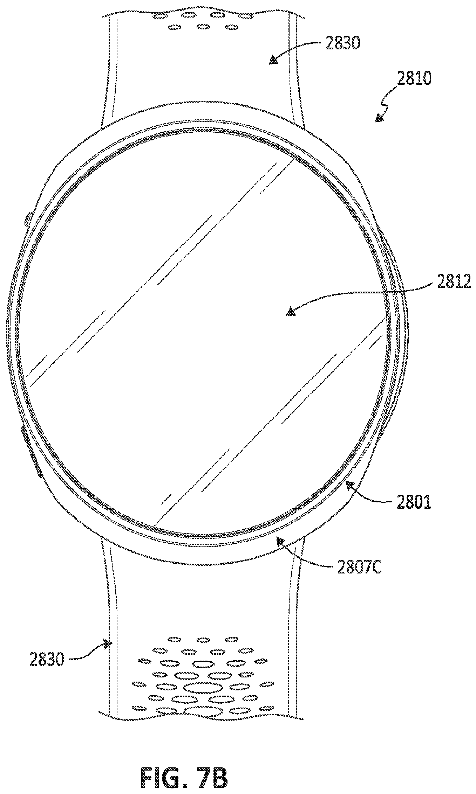

The wearable device 2810 may rotate about axis 2811 or axis 2815 such as when a user presses on a side of the wearable device wearable device 2810 that is opposite the sensor or module 2800 such as shown in Figure 7B. For example, a user may press on electrode 2807C to effectuate an ECG measurement which may cause the wearable device 2810 to rotate about axis 2811 and/or axis 2815. In some implementations, a user may commonly contact a portion of the electrode 2807C that is adjacent to the strap(s) 2830 which may cause the wearable device 2810 to rotate about axis 2811. In some implementations, a user may commonly contact a portion of the electrode 2807C that is between the straps strap(s) 2830, such as a side of the wearable device 2810, which may cause the wearable device 2810 to rotate about axis 2815. In some implementations, the wearable device 2810 may be more likely to rotate about axis 2811 or axis 2815 than any other axis within a same plane. For example, the wearable device 2810 may have a lower moment of inertia about axis 2811 or axis 2815 than any other axis within a same plane. For example, the device 2810 may be more susceptible to tilting, pivoting, rotating, etc. about axis 2811 or 2815 than other axes within a same plane. This may be, in part, because a user may more commonly contact portions of the electrode 2807C along axis 2811 and/or axis 2815, and/or because portions of the electrode 2807C may lie along axis 2811 and/or axis 2815.

In some implementations, the wearable device 2810 may be less likely to rotate about axis 2813 or axis 2817 than axis 2811 or axis 2815. In some implementations, the wearable device 2810 may be less likely to rotate about axis 2813 or axis 2817 than any other axis within a same plane. This may be, in part, because a user may less commonly contact portions of the electrode 2807C that are not disposed along axis 2811 and/or axis 2815, and/or because portions of the electrode 2807C may not lie along axis 2811 and/or axis 2815. Moreover, this may be, in part, because the wearable device 2810 may have a higher moment of inertia about axis 2813 or axis 2817 than any other axis within the same plane. For example, the device 2810 may be more resistant to tilting, pivoting, rotating, etc. about axis 2813 or 2817 than other axes within a same plane. For example, the strap(s) 2830 may prevent the device 2810 from tilting, pivoting, and/or rotating along either axis 2813 or axis 2817. For example, rotation about axis 2813 or axis 2817 would require a force large enough to cause the strap(s) 2830 to twist whereas rotation about axis 2811 or axis 2815 would not require the strap(s) 2830 to twist. Moreover, rotation about axis 2811 or axis 2815 may require the strap(s) 2830 to twist more (thus requiring a larger torsion force) than rotation about any other axis within the same plane. Accordingly, regardless of where a user may press on the device housing 2801, such as to contact the electrode 2807C, and/or regardless of where electrode 2807C is located on the device housing 2801, the strap(s) 2830 may prevent rotation of the wearable device 2810 along axis 2813 and/or axis 2817.

The electrodes 2807A, 2807B may intersect each of the axes about which rotation is most common. For example, electrodes 2807A may intersect axis 2811 and axis 2815. Accordingly, at least a portion of electrode 2807A is likely to maintain contact with a skin of the user, such as near axis 2811 and/or 2815, when the wearable device 2810 is rotating at least because rotation about either axis 2811 or axis 2815 would not cause these axes to separate from the skin of the user during their respective rotations. As another example, electrodes 2807B may intersect axis 2811 and axis 2815. Accordingly, at least a portion of electrode 2807B is likely to maintain contact with a skin of the user for at least the reasons provided with respect to electrode 2807A.

Figure 7B illustrates an additional view of example wearable device 2810 which may be an opposite side of wearable device 2810 as shown in Figure 7A. The wearable device 2810 can include strap(s) 2830, a display screen 2812, a device housing 2801, and electrode 2807C. The electrode 2807C may be disposed within or along a surface of the device housing 2801. The device housing 2801 may include the electrode 2807C. The electrode 2807C may be integrated with the device housing 2801. A portion of the device housing 2801 may function as the electrode 2807C. For example, the device housing 2801 may include conductive material configured to measure electrical activity detected at a skin of a user.

The electrode 2807C may be a positive electrode. The electrode 2807C may be a negative electrode. The electrode 2807C may be a reference electrode. In some implementations, a user may contact the electrode 2807C with a portion of their body that is different from the portion of the body on which they are wearing the wearable device 2810. For example, the user may wear the wearable device 2810 on their left wrist and may contact the electrode 2807C with a finger of their right hand. In some implementations, the electrode 2807C may measure a positive or negative electrical signal on a first portion of the user's body and either electrode 2807A or electrode 2807B may measure an opposite signal of electrode 2807C (whether positive or negative) on another portion of the user's body and either electrode 2807A or electrode 2807B may measure a reference signal. The portions of the user' body may be on opposite sides of the user's heart. For example, the user's heart may be in between the left and right hands of the user.

The electrode 2807C may extend around a perimeter or a periphery of the wearable device 2810 or device housing 2801. The electrode 2807C may be adjacent to the display screen 2812. The electrode 2807C may encompass a portion of the display screen 2812. The electrode 2807C may surround the display screen 2812. The electrode 2807C may encompass an entirety of the display screen 2812. For example, the electrode 2807C may contiguously circumscribe the display screen 2812. In some implementations, the electrode 2807C may form a closed loop along which a user may contact any point to effectuate an measurement. In some implementations, the wearable device 2810 may include multiple electrodes 2807C that comprise multiple discrete sections of the device housing 2801 and which all measure a same electrode signal such as all positive, all negative, or all reference. For example, the wearable device 2810 may include electrodes 2807C disposed within a top, bottom, left, and/or right portion of the device housing 2801.

The electrode 2807C may be disposed on an upper surface of the device housing 2801. For example, the electrode 2807C, or portion thereof, may be parallel or substantially parallel with display screen 2812. Electrode 2807C may be disposed on a side surface of the device housing 2801.

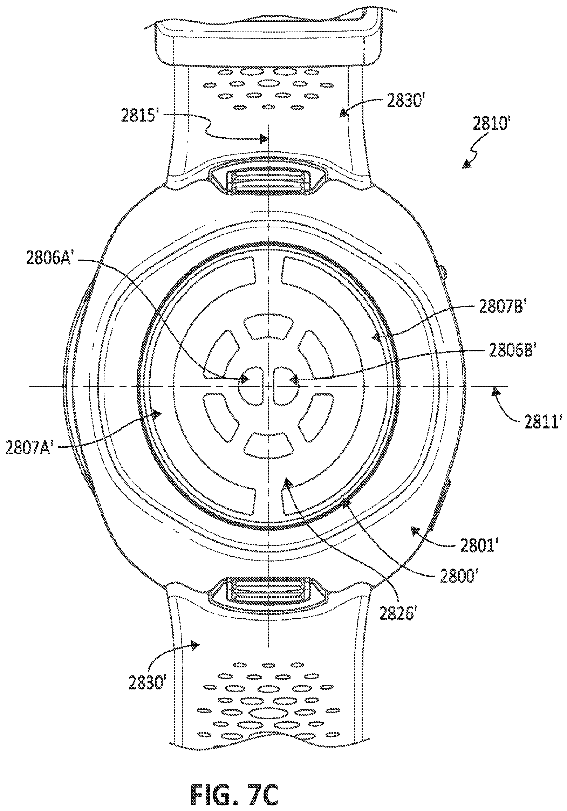

Figure 7C illustrates an example wearable device 2810'. The wearable device 2810' can include similar structural and/or operational features as any of the other example wearable devices shown and/or described herein such as wearable device 2810. Wearable device 2810' can include a device housing 2801', strap(s) 2830', and a sensor or module 2800'. The sensor or module 2800' can include a frame 2826', electrode 2807A', electrode 2807B', emitter chamber 2806A', and emitter chamber 2806B'. Emitter chamber 2806A' can enclose a first group of emitters situated on a substrate, such as a PCB, of the sensor or module 2800'. Emitter chamber 2806B' can enclose a second group of emitters situated on a substrate, such as a PCB, of the sensor or module 2800'.

Example axes 2815' and 2811' are illustrated as superimposed on the example wearable device 2810' shown in Figure 7C. Axes 2815' and 2811' may include similar features as axes 2815 and 2811, respectively, shown and/or discussed with respect to Figure 7A. Axis 2815' may bisect the wearable device 2810'. Axis 2815' may bisect the sensor or module 2800'. Axis 2815' may not intersect electrode 2807A'. Axis 2815' may not intersect electrode 2807B'. Axis 2815' may not intersect emitter chamber 2806A'. Axis 2815' may not intersect emitter chamber 2806B'. electrode 2807A' and electrode 2807B' may be symmetrical across axis 2815'. For example, electrode 2807A' and electrode 2807B' may be mirror images of each other across axis 2815'. Emitter chamber 2806A' and emitter chamber 2806B' may be symmetrical across axis 2815'. For example, emitter chamber 2806A' and emitter chamber 2806B' may be mirror images of each across axis 2815'.

Axis 2811' may intersect electrode 2807A'. Axis 2811' may bisect electrode 2807A'. For example, electrode 2807A' may be symmetrical with itself across axis 2811'. Axis 2811' may intersect electrode 2807B'. Axis 2811' may bisect electrode 2807B'. For example, electrode 2807B' may be symmetrical with itself across axis 2811'.

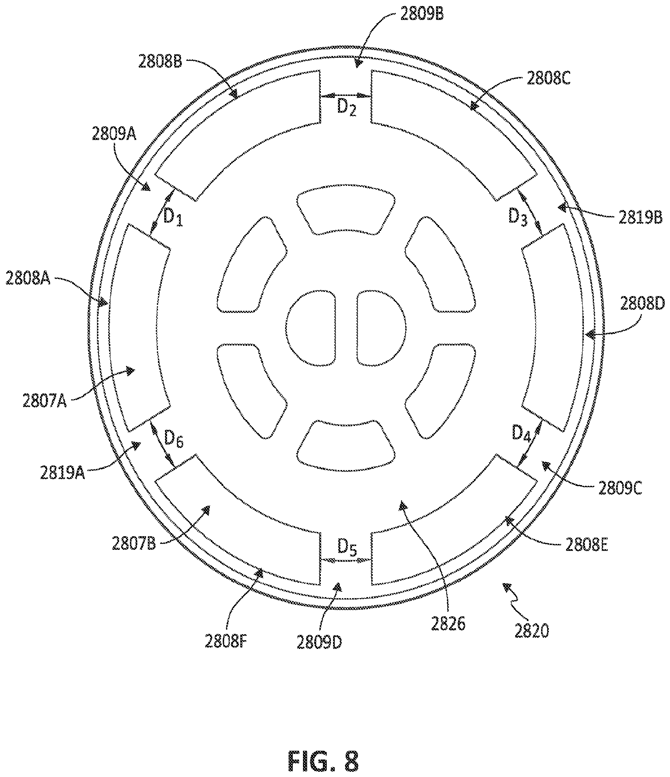

Figure 8 illustrates a front view of an example sensor or module 2820. The sensor or module 2820 can include similar structural and/or operational features as any of the other example sensor or modules shown and/or described herein. The sensor or module 2820 can include electrode 2807A, electrode 2807B, and a frame 2826.

The frame 2826 can include receptacles 2808A-2808F. The receptacles 2808A-2808F may be sized to receive a portion of electrode 2807A and/or electrode 2807B. For example, receptacles 2808A-2808C may be sized to receive portions of electrode 2807A such that portions of electrode 2807A are exposed through receptacles 2808A-2808C. As another example, receptacles 2808D-2808F may be sized to receive portions of electrode 2807B such that portions of electrode 2807B are exposed through receptacles 2808D-2808F. In some implementations, a majority of a surface area of a surface of electrode 2807A may be exposed through receptacles 2808A-2808C. In some implementations, a majority of a surface area of a surface of electrode 2807B may be exposed through receptacles 2808D-2808F. The portions of electrode 2807A that are exposed through receptacles 2808A-2808C may be flush with portions of the frame 2826 that surround the receptacles 2808A-2808C. The portions of electrode 2807B that are exposed through receptacles 2808D-2808F may be flush with portions of the frame 2826 that surround the receptacles 2808D-2808F.

The receptacles 2808A-2808F may form an annulus. Receptacles 2808A-2808C may form a semi-annulus or a substantially half annulus. Receptacles 2808D-2808F may form a semi-annulus or a substantially half annulus.

In the example implementation shown in Figure 8, the frame 2826 includes six receptacles. In some implementations, the frame can include one receptacle, two receptacles, three receptacles, four receptacles, five receptacles, or more than six receptacles. In some implementations, the frame 2826 can include a same number of receptacles as electrodes such as one receptacle per electrode. In some implementations, frame 2826 can include a different number of receptacles than electrodes.

The frame 2826 can include cover portions 2809A-2809D. Cover portions 2809A-2809B may secure electrode 2807A within the sensor or module 2820. Cover portions 2809A-2809B may secure electrode 2807B within the sensor or module 2820. Cover portion 2809A can have a width D₁. Cover portion 2809B can have a width D₂. Cover portion 2809C can have a width D₄. Cover portion 2809D can have a width D₅. In the example implementation shown in Figure 8, the frame 2826 includes four cover portions. In some implementations, the frame can include one cover portion, two cover portions, three cover portions, or more than four cover portions. The frame 2826 can include twice as many cover portions as electrodes. The frame 2826 can include two cover portions per electrode. As another example, the frame can include one cover portion per electrode. As another example, the frame can include three cover portions per electrode. Any number of cover portions per electrode is contemplated. In some implementations, the frame 2826 may include a different number of cover portions for one electrode than for another electrode.

The frame 2826 can include partitions 2819A and 2819B. Partitions 2819A, 2819B may separate electrode 2807A from electrode 2807B. For example, partitions 2819A, 2819B may electrically insulate electrode 2807A from electrode 2807B.

In some implementations, D₁=D₂. In some implementations, D₄=D₅. In some implementations, D₁=D₂=D₄=D₅. In some implementations, D₃=D₆. In some implementations, D₁=D₂=D₃=D₄=D₅=D₆. In some implementations, one or more of D₁, D₂, D₃, D₄, D₅, D₆ has a different length than one or more of D₁, D₂, D₃, D₄, D₅, D₆. In some implementations, cover portions 2809A-2809D and partitions 2819A, 2819B are equally spaced apart from one another on an annulus around a periphery of the frame 2826 or sensor or module 2820. D₁ can have a length of less than 7mm, less than 6mm, less than 5mm, less than 4mm, less than 3.5mm, less than 3mm, less than 2.5mm, etc. by way of non-limiting examples.

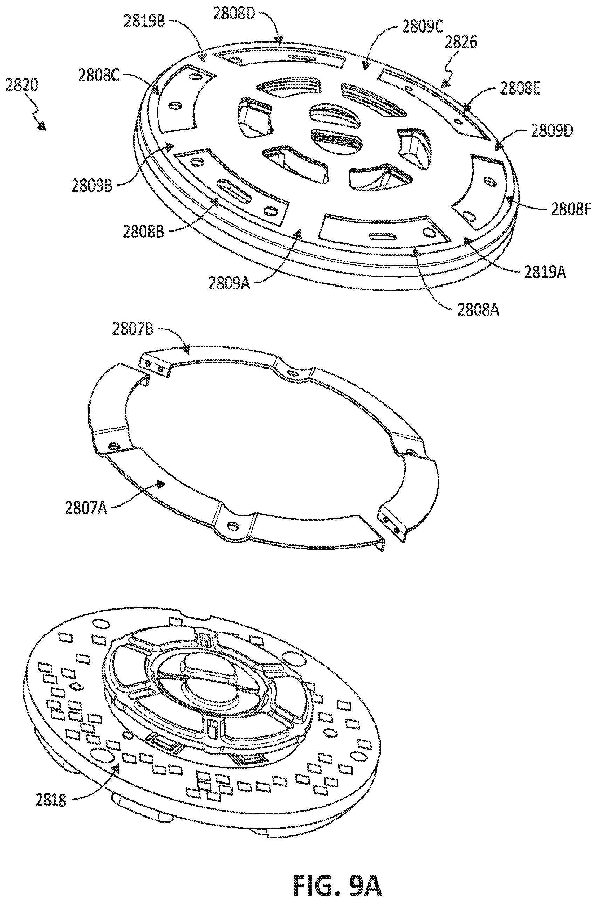

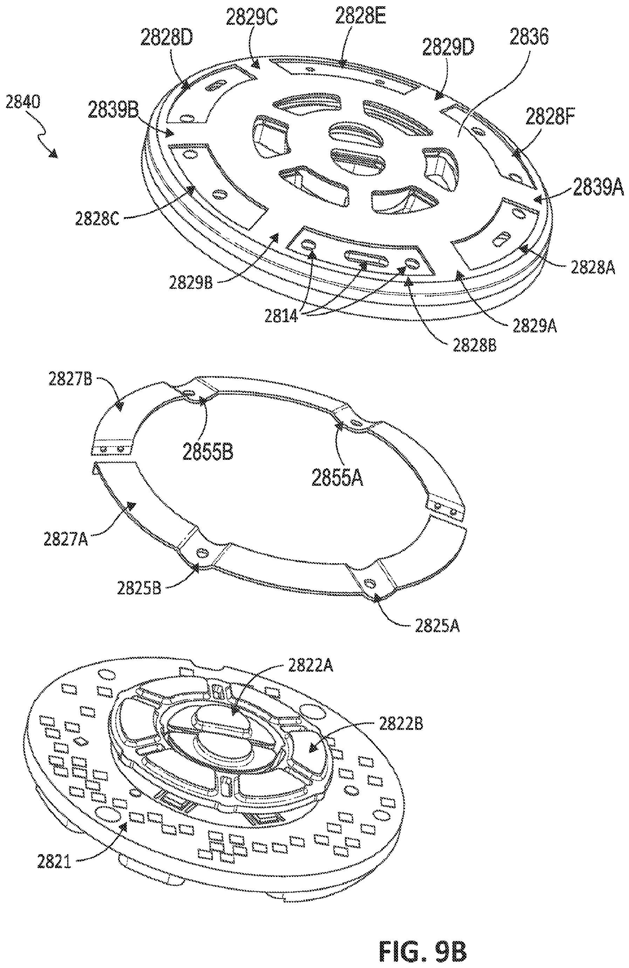

Figure 9A is a perspective exploded view of an example sensor or module 2820. The sensor or module 2820 can include a substrate 2818. The substrate 2818 may include a printed circuit board (PCB). The sensor or module 2820 can include electrode 2807A and electrode 2807B. The sensor or module 2820 can include frame 2826. Frame 2826 can include receptacles 2808A-2808C. Portions of electrode 2807A may be exposed through receptacles 2808A-2808C. For example, portions of electrode 2807A may contact the skin of a user through receptacles 2808A-2808C. Frame 2826 can include receptacles 2808D-2808F. Portions of electrode 2807B may be exposed through receptacles 2808D-2808F. For example, portions of electrode 2807B may contact the skin of a user through receptacles 2808D-2808F. In some implementations, electrode 2807A and electrode 2807B may be oriented differently with respect to other components of the sensor or module 2820 such as the substrate 2818 and/or the frame 2826. For example, electrode 2807A and electrode 2807B may be oriented as shown and/or described with respect to Figure 9B. As another example, electrode 2807A and electrode 2807B may be oriented as shown and/or described with respect to Figure 5A or Figure 6D, for example.

Figure 9B is a perspective exploded view of an example sensor or module 2840. The sensor or module 2840 can include a substrate 2821. The substrate 2821 may include a printed circuit board (PCB). One or emitters may be disposed on the substrate 2821. One or more detectors may be disposed on the substrate 2821. The sensor or module 2840 can include light transmissive covers 2822A, 2822B. Light transmissive cover 2822A may cover one or more emitters situated on the substrate 2821. Light transmissive cover 2822B may cover one or more detectors situated on the substrate 2821. In some implementations, light transmissive covers 2822A, 2822B may form a single body. In some implementations, light transmissive cover 2822A may be a separate, distinct component than light transmissive cover 2822B.

The sensor or module 2840 can include electrode 2827A and electrode 2827B. The sensor or module 2840 can include frame 2836. Frame 2836 can include receptacles 2828A-2828F, which may be apertures in the frame 2836. Portions of electrode 2827A may be exposed through receptacles 2828A-2828C. Portions of electrode 2827B may be exposed through receptacles 2828D-2828F. For example, portions of electrode 2827A and/or 2827B may contact the skin of a user through receptacles 2828A-2828F and can conduct electrical signals originating from the user via contact with the skin of the user. Frame 2836 can include one receptacle per electrode or more than one receptacle per electrode.

Any of the example receptacles, such as receptacles 2828A-2828F may include one or more openings. For example, receptacle 2828B may include openings 2814. Openings 2814 may connect an outer surface of the frame 2836 with an inner surface of the frame 2836 and/or an interior region of the sensor or module 2840. Openings 2814 may facilitate an electrical connection between electrode 2827A and substrate 2821. For example, an electrically conductive material may be disposed through openings 2814 and may contact the electrode 2827A and the substrate 2821. Openings 2814 may be circular. Openings 2814 may be elongate. Any of the example receptacles 2828A-2828F may include the same or a different number of openings, such as zero, one, two, three, four, or more than four openings.

Frame 2836 can include cover portions 2829A-2829D. Electrode 2827A may include recess portions 2825A, 2825B. The recess portions 2825A, 2825B may form a non-uniform surface with other adjacent portions of electrode 2827A. The recess portions 2825A, 2825B may not be exposed. For example, the recess portions 2825A, 2825B may not contact the skin of a user. Cover portions 2829A, 2829B may cover recess portions 2825A, 2825B, respectively. Cover portions 2829A, 2829B may be configured to receive recess portions 2825A, 2825B, respectively. Cover portions 2829A, 2829B may secure electrode 2827A to the sensor or module 2840. Frame 2836 may include similar cover portions configured to secure electrode 2827B. Electrode 2827B may include recess portions 2855A-2855B. Cover portions 2829C-2829D may cover the recess portions 2855A-2855B.

Frame 2836 can include partitions 2839A-2839B. Partitions 2839A-2839B may separate electrode 2827A from electrode 2827B. Partitions 2839A-2839B may electrically insulate electrode 2827A from electrode 2827B. At least a portion of partitions 2839A-2839B may cover at least a portion of electrodes 2827A-2827B, such as an end portion of the electrodes 2827A-2827B.

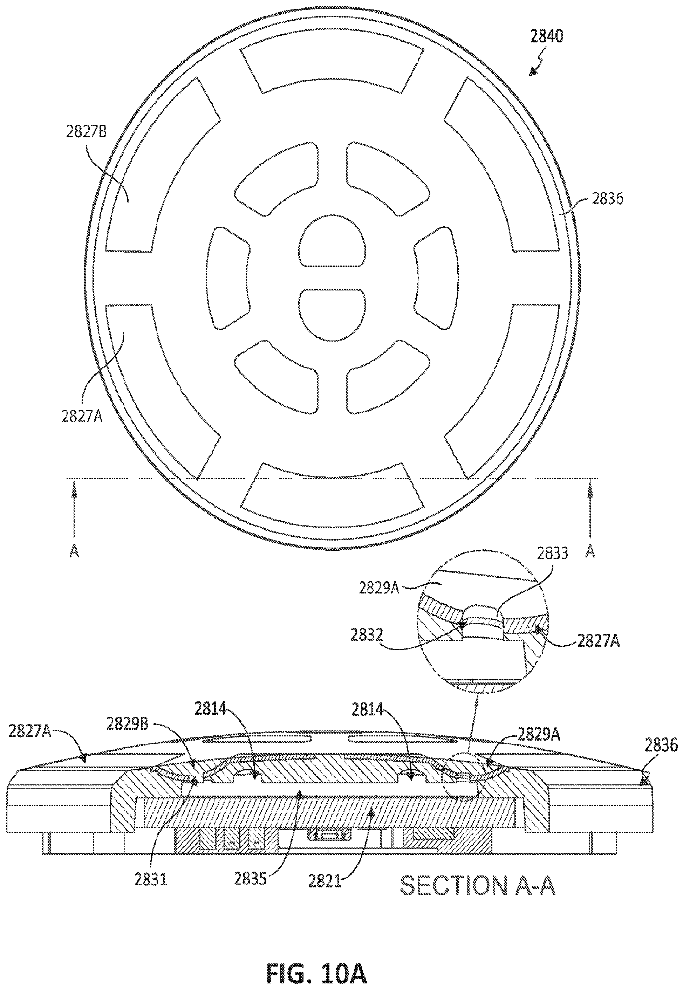

Figure 10A is a side cutaway view of an example sensor or module including a frame 2836, an electrode 2827A, and a substrate 2821. Electrode 2827A may be disposed within the frame 2836 and/or secured to the frame 2836. The frame 2836 can include cover portions 2829A, 2829B. Cover portions 2829A, 2829B can be configured to cover portions of electrode 2827A to secure the electrode 2827A to the frame 2836.

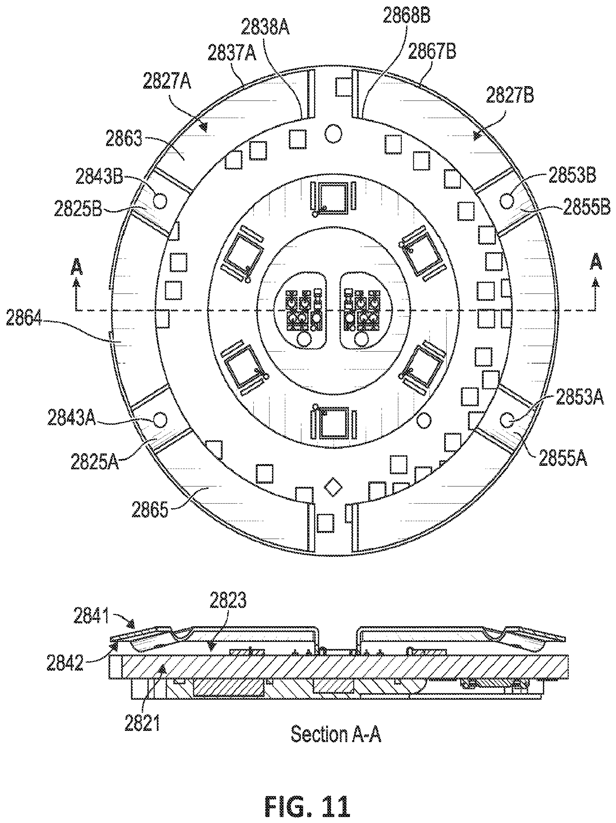

Cover portion 2829B can include post 2831. Post 2831 may be sized and/or shaped to penetrate an opening of an electrode 2827A, such as through-hole 2843A shown and/or described with reference to Figure 11. Post 2831 may insulate adjacent portions of an electrode 2827A from an interior region 2835 of frame 2836. Post 2831 may be configured to prevent substantial movement of the electrode 2827A.

Frame 2836 can include shaft 2833. Shaft 2833 may extend into cover portion 2829A. Shaft 2833 may extend through a portion of the frame 2836 beneath the cover portion 2829A. Shaft 2833 may receive an electrically conductive material which may contact the electrode 2827A via the opening 2832 and which may contact the substrate 2821 via the interior region 2835. A through-hole in the electrode 2827A may surround the shaft 2833.

The shaft 2833 can include opening 2832. Opening 2832 may expose adjacent portions of electrode 2827A to an interior region 2835 of frame 2836. The interior region 2835 of frame 2836 may be a space between the frame 2836 and/or electrode 2827A and the substrate 2821. In some implementations, electrode 2827A may be electrically connected to the substrate 2821 via opening 2832. For example, an electrically conductive material disposed in the interior region 2835 of frame 2836 may contact electrode 2827A via opening 2832 and may also contact the substrate 2821.

The frame 2836 can include openings 2814. Openings 2814 may expose electrode 2827A to the interior region 2835. Openings 2814 may be configured to facilitate a physical and/or electrical connection between the substrate 2821 and the electrode 2827A. For example, an electrically conductive material may be disposed through openings 2814 and may contact the electrode 2827A and the substrate 2821.

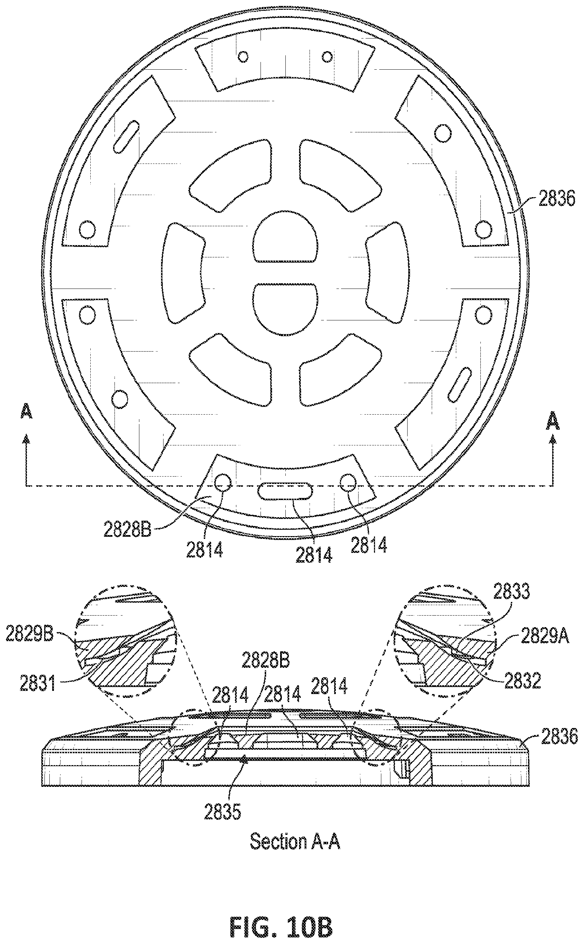

Figure 10B is a cutaway view of the frame 2836, including a cutaway view of the receptacle 2828B including the openings 2814. The openings 2814 may be continuous with the interior region 2835. A substrate, such as substrate 2821 shown in Figure 10A, may be positioned within the frame beneath and/or adjacent to the interior region 2835. The openings 2814 may expose the interior region 2835 to an exterior of the frame 2836. The openings 2814 may expose the interior region 2835 to the receptacle 2828B or to an electrode positioned within the receptacle 2828B. An electrically conductive material may fill the interior region 2835, in whole or in part, including the openings 2814. For example, an electrically conductive material may electrically couple a substrate positioned adjacent to the interior region 2835 with an electrode positioned within the receptacle 2828B via openings 2814. Receptacle 2828B may have any number of openings 2814 which may have any size and/or shape.

Post 2831 may be positioned adjacent to the cover portion 2829B. Post 2831 may extend through a through-hole of an electrode held by the frame 2836. Post 2831 may extend from the cover portion 2829B through an electrode to a portion of the frame 2836 adjacent to the cover portion. Post 2831 may comprise a solid interior. Post 2831 may comprise a hollow interior. Post 2831 may comprise a material that is continuous with adjacent portions of the frame 2836.

Shaft 2833 may be positioned adjacent to the cover portion 2829A. Shaft 2833 may extend, at least partially, through a through-hole of an electrode held by the frame 2836. Shaft 2833 may extend from the cover portion 2829A. Opening 2832 may expose a portion of an electrode positioned under cover portion 2829A to the interior region 2835 of the frame 2836. An electrically conductive material may contact a portion of an electrode via the opening 2832 and may contact a substrate positioned within the frame 2836 adjacent to the interior region 2835. An electrode positioned under the cover portion 2829A may be in electrical communication via the opening 2832 with a substrate positioned within the frame 2836. Shaft 2833 may comprise a hollow interior which may receive a portion of an electrically conductive material in contact with an electrode and with a substrate. In some implementations, shaft 2833 may comprise a solid interior.

Figure 11 is a cutaway view of example electrodes 2827A, 2827B and substrate 2821 of a sensor or module. Electrode 2827B may include any of the features of electrode 2827A shown in any of the figures and/or described anywhere herein, and vice versa. Electrode 2827B may be symmetrical to electrode 2827A. Electrode 2827B may be a mirror image of electrode 2827A. Electrode 2827A can include through-holes 2843A, 2843B. Electrode 2827B can include through-holes 2853A, 2853B. Through-holes 2843A, 2843B may be disposed within recess portions 2825A, 2825B. Through-holes 2853A, 2853B may be disposed within recess portions 2855A, 2855B. In some implementations, electrode 2827A may not include through-holes 2843A, 2843B and/or recess portions 2825A, 2825B. In some implementations, electrode 2827B may not include through-holes 2853A, 2853B and/or recess portions 2855A, 2855B.

Electrode 2827A may include an outer surface 2841. Portions of the outer surface 2841 may be exposed and may contact the skin of a user. In some implementations, less than all portions of the outer surface 2841 are exposed and/or contact the skin of the user. For example, portions of the outer surface 2841 within the recess portions 2825A, 2825B, may not be exposed and/or may not contact the skin of the user. The outer surface 2841 may not be uniform, flat, level, and/or planar. The outer surface 2841 may include irregularities, such as recess portions 2825A, 2825B.

Through-holes 2843A, 2843B may be disposed within a plane that is substantially parallel to a plane in which portions of the outer surface adjacent to the opening are disposed. Through-holes 2843A, 2843B may be disposed within a plane that is substantially parallel to a plane created by the skin of the user in proximity to the through-holes 2843A, 2843B.

Electrode 2827A may include an inner surface 2842. Inner surface 2842 may be substantially parallel and/or substantially planar with outer surface 2841. In some implementations, inner surface 2842 may be non-parallel and/or non-planar with outer surface 2841.

The outer surface 2841 and/or the inner surface 2842 may be non-planar. For example, the outer surface 2841 may substantially form a portion of a substantially conical or spherical surface. As another example, the outer surface 2841 may be flush with a substantially conical or convex surface of a frame of a sensor or module. As another example, the outer surface 2841 may be non-parallel with a substantially planar surface 2823 of a substrate 2821 of a sensor or module. As another example, a cross section of the outer surface 2841 and/or inner surface 2842 may be angled with respect to a surface 2823 of substrate 2821 of a sensor or module. The surface 2823 may be the surface of the substrate 2821 on which emitters and/or detectors are located.

In some implementations, electrode 2827A and electrode 2827B may be oriented differently with respect to the substrate 2821. For example, electrode 2827A and electrode 2827B may be oriented as shown and/or described with respect to Figure 9A.

The through-hole 2843B may be positioned within recess portion 2825B. The through-hole 2843B may extend through the electrode 2827A. The through-hole 2843B may be a through-hole or via. The through-hole 2843B may be circular, as shown. In some implementations, the through-hole 2843B may comprise another shape such as rectangular or triangular. A center of the through-hole 2843B may be positioned equidistant between the outer edge 2837A and the inner edge 2838A. The through-hole 2843B may have a diameter of less than 2.0mm, less than 1.5mm, less than 1.0mm, less than 0.5mm, etc., by way of non-limiting examples. Through-holes 2843A, 2853A, or 2853B may have any of the features shown and/or described with respect to through-hole 2843B. A center of the through-hole 2843B may be positioned equidistant between the through-hole 2843A and an end of the electrode 2827A. A center of the through-hole 2843A may be positioned equidistant between the through-hole 2843B and another end of the electrode 2827A.

The electrode 2827A may include portion 2863, portion 2864, and portion 2865. The portion 2863 may be adjacent to the recess portion 2825B. The portion 2863 may be between the recess portion 2825B and an end of the electrode 2827A. The portion 2864 may be between the recess portions 2825B and 2825A. The portion 2865 may be adjacent to the recess portion 2825A. the portion 2865 may be between the recess portion 2825A and an end of the electrode 2827A. Portions 2863, 2864, and/or 2865, or surfaces thereof, may be exposed to an exterior of the frame 2836 and may contact the skin of a user. The portion 2863 may be a similar size as portion 2864 and/or portion 2865.

The electrode 2827A may include an outer edge 2837A. The outer edge 2837A may be substantially circular from a top view, as shown in Figure 11. Portions of the outer edge 2837A may define at least a portion of a circle. For example, portions of outer edge 2837A extending along electrode portions 2863, 2864, and/or 2865 may define a portion of one or more circles. In some implementations, portions of outer edge 2837A extending along portion 2863, portion 2864, and portion 2865 may define different portions of a same circle. The outer edge 2837A, or portions thereof, may define a circle having a diameter of less than 50mm, less than 40mm, less than 35mm, less than 30mm, less than 25mm, less than 20mm, etc., by way of non-limiting examples. The electrode 2827B may have an outer edge 2867B. The outer edge 2867B, or portions thereof, may define at least portions of one or more circles, which circles may be coincident with one or more circles defined by outer edge 2837A.

The electrode 2827A may include an inner edge 2838A. The inner edge 2838A may be substantially circular from a top view, as shown in Figure 11. Portions of the inner edge 2838A may define at least a portion of a circle. For example, portions of inner edge 2838A extending along electrode portions 2863, 2864, and/or 2865 may define a portion of one or more circles. The inner edge 2838A, or portions thereof, may define a circle having a diameter of less than less than 45mm, less than 40mm, less than 35mm, less than 30mm, less than 25mm, less than 20mm, less than 15mm, etc., by way of non-limiting examples. The electrode 2827B may have an inner edge 2868B. The inner edge 2868B, or portions thereof, may define at least portions of one or more circles, which circles may be coincident with one or more circles defined by inner edge 2838A. The inner edge 2838A, or portions thereof, may be parallel with the outer edge 2837A. In some implementations, the inner edge 2838A, or portions thereof, may be non-parallel with the outer edge 2837A. The inner edge 2868A, or portions thereof, may be parallel with the outer edge 2867A. In some implementations, the inner edge 2868A, or portions thereof, may be non-parallel with the outer edge 2867A. In some implementations, electrode 2827A may be shaped and/or sized differently than electrode 2827B.

Although electrodes 2827A, 2827B are shown in Figure 11 as being circular, annular, or semi-annular, electrodes 2827A, 2827B, or any of the other example electrodes shown and/or described herein be shaped differently. For example, any of the electrodes shown and/or described herein may be rectangular, semi-circular, half-circle, triangular, U-shaped, or the like. As another example, outer edge 2837A and/or inner edge 2838A may define a non-circular curve. For example, the outer edge 2837A and/or inner edge 2838A may comprise one or more angles, when viewed from a top view.

The electrode 2827A may have a width between outer edge 2837A and inner edge 2838A of less than 5mm, less than 4mm, less than 3.5mm, less than 3mm, less than 2.5mm, etc. by way of non-limiting examples.

The electrode 2827A may have a thickness between outer surface 2841 and inner surface 2842 of less than 0.3mm, less than 0.25mm, less than 0.2mm, less than 0.15mm, etc., by way of non-limiting examples.

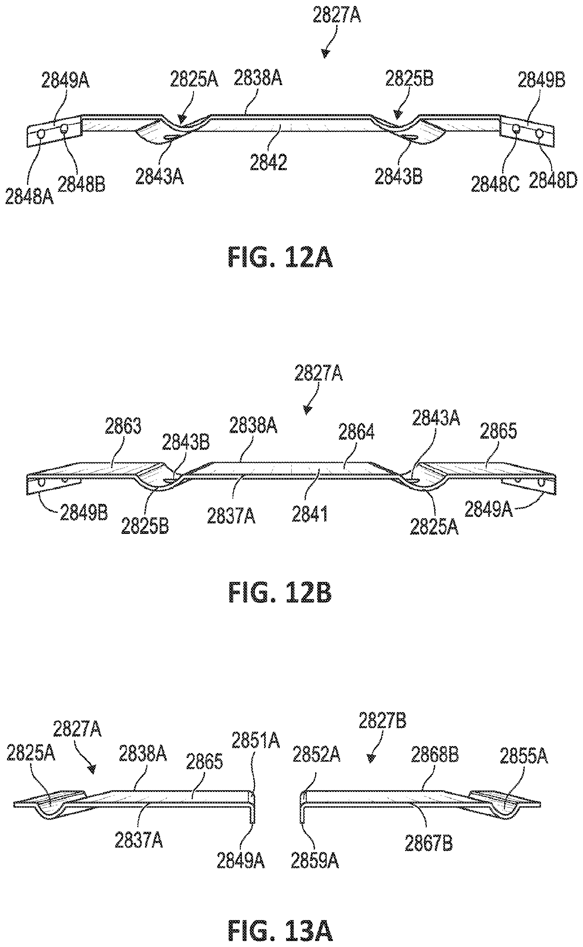

Figure 12A is a side view of electrode 2827A. Electrode 2827A can include recess portions 2825A, 2825B. Recess portions 2825A, 2825B may be curved. Recess portions 2825A, 2825B may be curved with respect to adjacent portions of the electrode 2827A. Recess portions 2825A, 2825B may have a different curvature than other portions of the electrode 2827A, such as portions that are adjacent to the recess portions 2825A, 2825B. Recess portions 2825A, 2825B may interrupt a continuity of other portions of the electrode 2827A. Recess portions 2825A, 2825B may be non-uniform with respect to other portions of the electrode 2827A, such as portions that are adjacent to the recess portions 2825A, 2825B. Recess portions 2825A, 2825B may be disposed between adjacent portions of the electrode 2827A that are configured to contact the skin of a user. The recess portions 2825A, 2825B may not contact the skin of the user.

Electrode 2827A can include end portions 2849A, 2849B. End portions 2849A, 2849B may be angled with respect to adjacent portions of the electrode 2827A. For example, end portions 2849A, 2849B may be orthogonal to adjacent portions of the electrode 2847. End portions 2849A, 2849B can include openings such as through-holes 2848A-2848D. End portion 2849A can include one opening, two openings, three openings, or more than three openings. End portion 2849B can include one opening, two openings, three openings, or more than three openings. End portion 2849A may include a same number of openings as end portion 2849B. End portion 2849A may include a different number of openings than end portion 2849B. In some implementations, end portion 2849A and/or end portion 2849B may not include any openings. Through-holes 2848A-2848D may be configured to receive a portion of a frame of a sensor or module. Through-holes 2848A-2848D may be configured to secure the electrode 2827A to the frame of the sensor or module. End portions 2849A, 2849B may be configured to prevent the electrode 2827A from moving with respect to a frame of the sensor or module.

The electrode 2827A may include an inner edge 2838A that extends along the electrode 2827A. The inner edge 2838A may continuously extend along the electrode 2827A such as from end portion 2849A to end portion 2849B along recess portion 2825A and recess portion 2825B. In some implementations, the inner edge 2838A may be curved, beveled, chamfered, etc.

Figure 12B is another side view of electrode 2827A. The electrode 2827A may include outer surface 2841 that extends along the electrode 2827A. The outer surface 2841 may be disposed between outer edge 2837A and inner edge 2838A. The outer surface 2841 may extend along the portion 2863, the recess portion 2825B, the portion 2864, the recess portion 2825A, and the portion 2865. Portions of the outer surface 2841 may be exposed to an exterior and may contact the skin of the user. For example, portion of outer surface 2841 extending along portion 2863, portion 2864, and/or portion 2865 may contact the skin of the user. Portions of the outer surface 2841 may be inhibited from contacting the skin of the user, at least because they are separated from the skin of the user by a distance and/or are covered by portions of the frame 2836 such that they are not exposed and/or are blocked from contacting the skin of the user. For example, portions of outer surface 2841 extending along recess portion 2825B and/or recess portion 2825A may be recessed a distance from the skin of the user and/or occluded by the frame 2836 from contacting the skin of the user.

The outer edge 2837A may extend along a length of the electrode 2827A. The outer edge 2837A may continuously extend along the electrode 2827A along one or more of end portion 2849B, portion 2863, recess portion 2825B, portion 2864, recess portion 2825A, portion 2865, and/or end portion 2849A. For example, recess portion 2825B may share a continuous edge with portion 2863 and portion 2864. In some implementations, the outer edge 2837A may be curved, beveled, chamfered, etc.

Figure 13A is a side view of electrode 2827A and electrode 2827B. Electrode 2827A and electrode 2827B may be shown in Figure 13A positioned relative to one another as they would be if they were positioned within frame 2836 in a sensor module. The end portion 2849A may be positioned at an end of electrode 2827A. The end portion 2849A may be adjacent to the portion 2865. The inner edge 2838A and/or the outer edge 2837A may extend along the end portion 2849A. The end transition 2851A may extend from the portion 2865 to the end portion 2849A. The end transition 2851A may be curved, beveled, chamfered, or may be a sharp edge, etc. The end portion 2849A may extend from an adjacent portion of the electrode 2827A (e.g., portion 2865) at an angle, such as a 90-degree angle. For example, the outer edge 2837A may comprise an angle (e.g., a bend or a curve) between portion 2865 and end portion 2849A. As shown, the outer edge 2837A may comprise a 90-degree bend between portion 2865 and end portion 2849A. The inner edge 2838A may also comprise an angled bend between portion 2865 and end portion 2849A. In some implementations, the end portion 2849A may extend from the adjacent portion 2865 of the electrode 2827A at less than 90 degrees, or in some implementations, at greater than 90 degrees. The electrode 2827B may include end portion 2859A and end transition 2852A which may include similar features as shown and/or described with respect to electrode 2827A. As shown, end portion 2849A may be parallel to end portion 2859A.

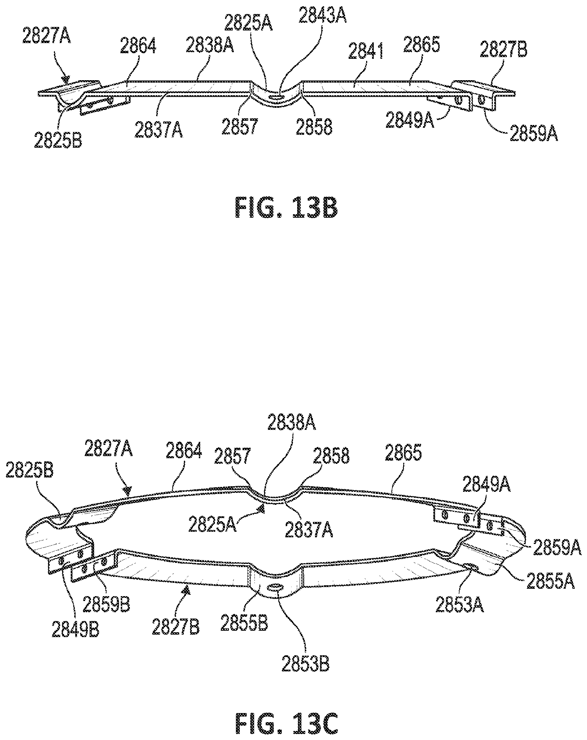

Figure 13B is another side view of electrode 2827A and electrode 2827B. Electrode 2827A and electrode 2827B may be shown in Figure 13B positioned relative to one another as they would be within frame 2836. The electrode 2827A can include recess transitions 2857 and 2858. The recess portion 2825A may be positioned between the recess transitions 2857 and 2858. The recess transition 2857 may form a portion of the outer surface 2841. The recess transition 2857 may be positioned between the portion 2864 and the recess portion 2825A. The recess transition 2857 may be curved, beveled, chamfered, or may be a sharp edge, etc. The recess transition 2858 may form a portion of the outer surface 2841. The recess transition 2858 may be positioned between the portion 2865 and the recess portion 2825A. The recess transition 2858 may be curved, beveled, chamfered, or may be a sharp edge, etc. The inner edge 2838A and/or the outer edge 2837A may continuously extend from portion 2864 to recess portion 2825A along recess transition 2857.

Recess portion 2825A may be substantially cylindrical. A portion of outer surface 2841 extending along recess portion 2825A may form a portion of a cylinder.

Figure 13C is a perspective view of electrode 2827A and electrode 2827B. Electrode 2827A and electrode 2827B may be shown in Figure 13B positioned relative to one another as they would be within frame 2836. Inner edge 2838A may extend along recess portion 2825A between portion 2864 and portion 2865. A portion of inner edge 2838A extending along recess portion 2825A may be substantially semi-circular. For example, a portion of the inner edge 2838A extending along the recess portion 2825A may form a portion of a circle. A portion of outer edge 2837A extending along the recess portion 2825A may be substantially semi-circular and may, for example, form a portion of a circle. In some implementations, the inner edge 2838A and/or the outer edge 2837A may define a portion of a non-circular curve. A curve (e.g., circle) defined, at least in part, by the portion of the inner edge 2838A extending along the recess portion 2825A may intersect a circle defined, in part, by the portion of the inner edge 2838A extending along the portion 2864 or portion 2865. A curve (e.g., circle) defined, at least in part, by the portion of the outer edge 2837A extending along the recess portion 2825A may intersect a circle defined, in part, by the portion of the outer edge 2837A extending along the portion 2864 or portion 2865.

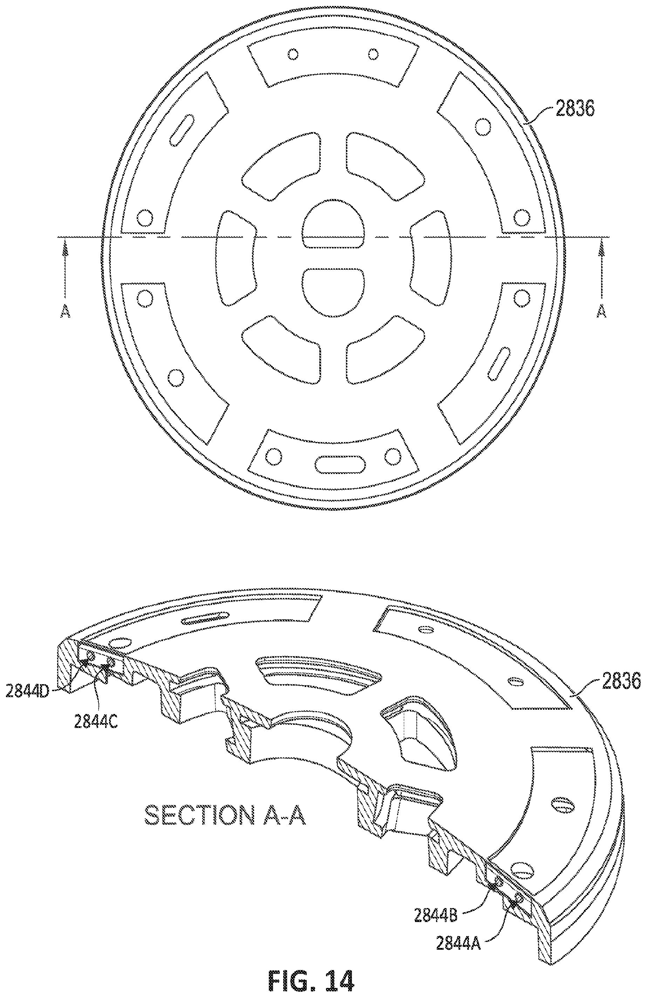

Figure 14 is a perspective cutaway view of an example frame 2836 of a sensor or module. Frame 2836 can include protrusions 2844A-2844D. The protrusions 2844A-2844D may be configured to secure to a portion of an electrode, such as an end portion. For example, the protrusions 2844A-2844D may fit inside an opening of an electrode such as through-holes 2848A-2848D shown and/or described with respect to Figure 12A. The protrusions 2844A-2844D may secure an electrode to the frame 2836. The protrusions 2844A-2844D may prevent an electrode from moving relative to the frame 2836. In some implementations, the frame 2836 may include less than four protrusions or more than four protrusions. The protrusions 2844A-2844D may be cylindrical. The protrusions 2844A-2844D be rectangular parallelepipeds.

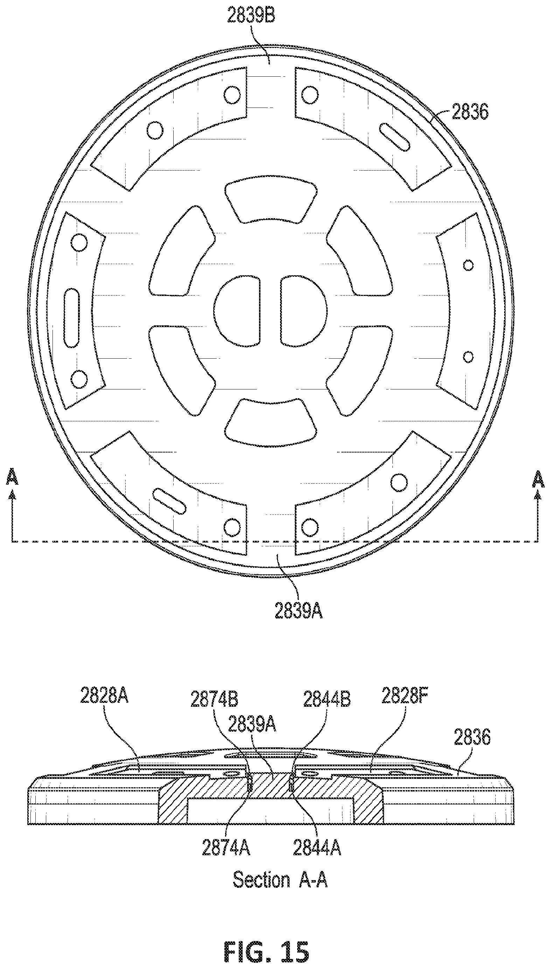

Figure 15 is a cutaway view of the frame 2836 of a sensor or module. The frame 2836 includes partition 2839A and partition 2839B. Partition 2839A may be positioned between receptacle 2828A and receptacle 2828F. Partition 2839A can be positioned between an electrode that is positioned within receptacle 2828A and an electrode that is positioned within receptacle 2828F. The partition 2839A may electrically insulate an electrode positioned within receptacle 2828A from an electrode that is positioned within receptacle 2828F. The partition 2839A may cover at least a portion of one or more electrodes, such as an end portion of an electrode. The frame 2836 can include protrusions 2844A-2844B and 2874A-2874B extending away from partition 2839A and which may extend through an electrode to secure an electrode within the frame 2836. The protrusions 2874A, 2874B may be positioned on a side of the partition 2839A that is opposite the protrusions 2844A, 2844B. As shown a portion of an electrode that is positioned within receptacle 2828A (or an electrode positioned within receptacle 2828F) may extend into the frame 2836 and be enclosed within the frame 2836 adjacent to the partition 2839A.

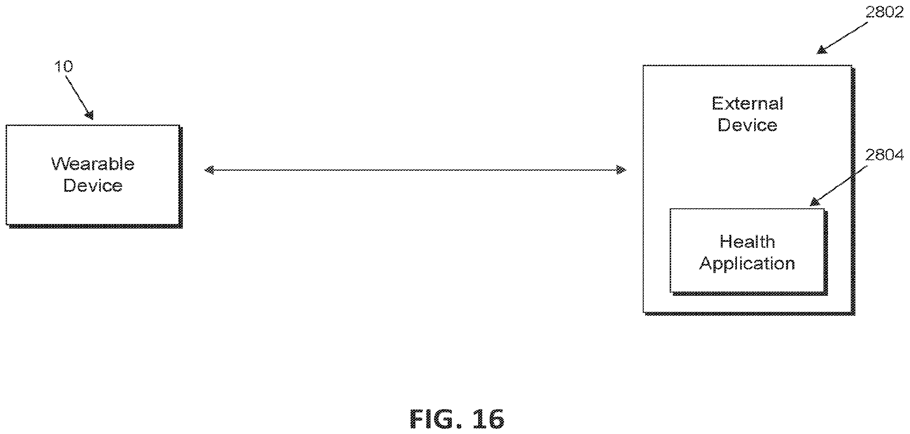

As discussed herein and as shown in Figure 2, the wearable device 10 can be in communication, for example wirelessly, to an external device. Figure 16 shows a block diagram illustrating an example aspect of the wearable device 10 in communication with an external device 2802. The communication may be wireless, such as, but not limited to, Bluetooth and/or near-field communication (NFC) wireless communication. As shown in Figure 2, the wearable device 10 may be in communication with any number and/or types of external devices 2802 which may include a patient monitor 202 mobile communication device 204 (for example, a smartphone), a computer 206 (which can be a laptop or a desktop), a tablet 208, a nurses' station system 201, glasses such as smart glasses configured to display images on a surface of the glasses and/or the like. The external device 2802 may include a health application 2804. "External device" and "computing device" may be used interchangeably herein.

A user may operate the external device 2802 as described herein. A wearer may wear the wearable device 10. In some implementations, the user of the external device 2802 and the wearer of the wearable device 10 are different people. In some implementations, the user of the external device 2802 and the wearer of the wearable device 10 are the same person. The terms "user" and "wearer" and "patient" may be used interchangeably herein and may all refer to a person wearing the wearable device 10 and/or a person using the health application 2804 and their uses in any of the given examples are not meant to be limiting of the present disclosure.

The wearable device 10 may communicate information such as physiological data of the wearer/user to the external device 2802. The external device 2802 may display the physiological parameters received from the wearable device 10, as described herein.

The external device 2802 may control operation of the wearable device 10, for example via a wireless connection as described herein. For example, the external device 2802 may cause the wearable device 10 to start or stop taking measurements of a wearer's physiological parameters. In some aspects, the wearable device 10 may continuously measure and communicate a wearer's physiological parameters to the external device 2802. In some aspects, the external device 2802 may continuously display the wearer's physiological parameters received from the wearable device 10. In some aspects, the wearable device 10 may measure and communicate physiological parameters to an external device 2802 for a finite amount of time, such as 1 minute, upon receiving user input at the external device 2802 communicated to the wearable device 10.

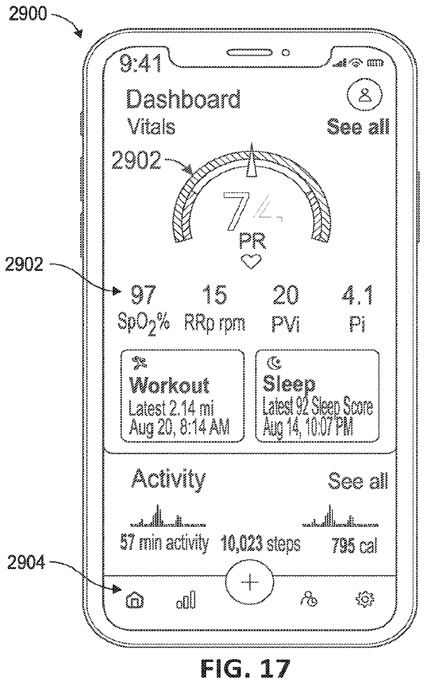

Figure 17 illustrates an example interactive graphical user interface of a health application 2804, according to some aspects of the present disclosure. In various aspects, aspects of the user interfaces may be rearranged from what is shown and described below, and/or particular aspects may or may not be included. The health application 2804 can execute on the external device 2802 to present the graphical user interface of Figure 17. As described herein, the health application 2804 can receive a respective client configuration package that causes the presentation of the graphical user interface of Figure 17. The graphical user interface of Figure 17 may have similar user interface elements and/or capabilities.

Figure 17 illustrates an example dashboard user interface 2900 of the health application 2804. The dashboard user interface 2900 can display current physiological parameters 2902 of a wearer such as pulse rate, SpO₂, RRp, PVi, Pi and the like. In addition to the presentation of current wearer physiological parameter(s) 2902, the dashboard user interface 2900 can present indicator(s) associated with one or more of the physiological parameters 2902 that visually indicate a status of the parameters 2902 and various status ranges for each parameter 2902. The indicator(s) may be color coded or otherwise show a severity or status of a physiological parameter 2902. The dashboard user interface 2900 may additionally display a history of wearer statistics/information such as workout history information, sleep information, activity levels, steps taken, and/or calories burned.

The dashboard user interface 2900 may additionally display one or more navigation selectors 2904 configured for selection by a user. The one or more navigation selectors 2904 may include a home navigation selector, an activity navigation selector, a workout navigation selector, a vitals navigation selector, a sleep navigation selector, a history navigation selector, a share navigation selector and/or a settings navigation selector. Selection of the navigation selectors 2904 may cause the health application 2804 to display any of the graphical user interfaces described herein associated with the selected navigation selector 2904. The navigation selectors 2904 may be displayed in any of the graphical user interfaces described herein.

### Additional Considerations

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain aspects, the terms "generally parallel" and "substantially parallel" refer to a value, amount, or characteristic that departs from exactly parallel by less than or equal to 10 degrees, 5 degrees, 3 degrees, or 1 degree. As another example, in certain aspects, the terms "generally perpendicular" and "substantially perpendicular" refer to a value, amount, or characteristic that departs from exactly perpendicular by less than or equal to 10 degrees, 5 degrees, 3 degrees, or 1 degree.

Many other variations than those described herein will be apparent from this disclosure. For example, certain acts, events, or functions of any of the algorithms described herein can be performed in a different sequence, can be added, merged, or left out altogether (for example, not all described acts or events are necessary for the practice of the algorithms). Moreover, acts or events can be performed concurrently, for example, through multi-threaded processing, interrupt processing, or multiple processors or processor cores or on other parallel architectures, rather than sequentially. In addition, different tasks or processes can be performed by different machines and/or computing systems that can function together.

It is to be understood that not necessarily all such advantages can be achieved in accordance with any particular example of the examples disclosed herein. Thus, the examples disclosed herein can be embodied or carried out in a manner that achieves or optimizes one advantage or group of advantages as taught herein without necessarily achieving other advantages as may be taught or suggested herein.

The various illustrative logical blocks, modules, and algorithm steps described in connection with the examples disclosed herein can be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative logical blocks and modules described in connection with the examples disclosed herein can be implemented or performed by a machine, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can include electrical circuitry or digital logic circuitry configured to process computer-executable instructions. In another example, a processor can include an FPGA or other programmable device that performs logic operations without processing computer-executable instructions. A processor can also be implemented as a combination of computing devices, for example, a combination of a DSP and a microprocessor, a plurality of microprocessors, one or more microprocessors in conjunction with a DSP core, or any other such configuration. A computing environment can include any type of computer system, including, but not limited to, a computer system based on a microprocessor, a mainframe computer, a digital signal processor, a portable computing device, a device controller, or a computational engine within an appliance, to name a few.

The steps of a method, process, or algorithm described in connection with the examples disclosed herein can be embodied directly in hardware, in a software module stored in one or more memory devices and executed by one or more processors, or in a combination of the two. A software module can reside in RAM memory, flash memory, ROM memory, EPROM memory, EEPROM memory, registers, hard disk, a removable disk, a CD-ROM, or any other form of non-transitory computer-readable storage medium, media, or physical computer storage known in the art. An example storage medium can be coupled to the processor such that the processor can read information from, and write information to, the storage medium. In the alternative, the storage medium can be integral to the processor. The storage medium can be volatile or nonvolatile. The processor and the storage medium can reside in an ASIC.

The apparatuses and methods described herein may be implemented by one or more computer programs executed by one or more processors. The computer programs include processor-executable instructions that are stored on a non-transitory tangible computer readable medium. The computer programs may also include stored data. Non-limiting examples of the non-transitory tangible computer readable medium are nonvolatile memory, magnetic storage, and optical storage.

The term "substantially" when used in conjunction with the term "real-time" forms a phrase that will be readily understood by a person of ordinary skill in the art. For example, it is readily understood that such language will include speeds in which no or little delay occurs.

Conditional language used herein, such as, among others, "can," "might," "may," "for example," and the like, unless specifically stated otherwise, or otherwise understood within the context as used, is generally intended to convey that certain examples include, while other examples do not include, certain features, elements and/or states. Thus, such conditional language is not generally intended to imply that features, elements and/or states are in any way required for one or more examples or that one or more examples necessarily include logic for deciding, with or without author input or prompting, whether these features, elements and/or states are included or are to be performed in any particular example. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Disjunctive language such as the phrase "at least one of X, Y, or Z," unless specifically stated otherwise, is otherwise understood with the context as used in general to present that an item, term, etc., may be either X, Y, or Z, or any combination thereof (for example, X, Y, and/or Z). Thus, such disjunctive language is not generally intended to, and should not, imply that certain examples require at least one of X, at least one of Y, or at least one of Z to each be present.

Unless otherwise explicitly stated, articles such as "a" or "an" should generally be interpreted to include one or more described items. Accordingly, phrases such as "a device configured to" are intended to include one or more recited devices. Such one or more recited devices can also be collectively configured to carry out the stated recitations. For example, "a processor configured to carry out recitations A, B and C" can include a first processor configured to carry out recitation A working in conjunction with a second processor configured to carry out recitations B and C.

While the above detailed description has shown, described, and pointed out novel features as applied to various examples, it will be understood that various omissions, substitutions, and changes in the form and details of the devices or algorithms illustrated can be made without departing from the disclosure. The invention is defined in the appended claims.

## Claims

1. A wearable device (110A, 110B, 110C, 350, 2810, 2810', 10) configured to perform physiological measurements, the wearable device (110A, 110B, 110C, 350, 2810, 2810', 10) comprising:
a frame (2826, 2826', 2836);
an electrode (354, 355, 124, 125, 2807A, 2807B, 2807A', 2807B', 2827A, 2827B) secured to the frame (2826, 2826', 2836) and configured to conduct electrical signals originating from a user of the wearable device (110A, 110B, 110C, 350, 2810, 2810', 10), the electrode (354, 355, 124, 125, 2807A, 2807B, 2807A', 2807B', 2827A, 2827B) comprising:
a first portion (2863, 2865) having a surface (2841) configured to contact a skin of the user;
a second portion (2864) having a surface (2841) configured to contact the skin of the user;
a third portion (2825A, 2825B, 2855A, 2855B) disposed between the first portion (2863, 2865) and the second portion (2864), the third portion (2825A, 2825B, 2855A, 2855B) comprising:
a surface (2841) that extends away from the surface (2841) of the first portion (2863, 2865) and the surface (2841) of the second portion (2864) and is separated from the skin of the user when the first portion (2863, 2865) or the second portion (2864) contacts the skin of the user; and
a through-hole (2843A, 2843B, 2853A, 2853B) extending through the electrode (354, 355, 124, 125, 2807A, 2807B, 2807A', 2807B', 2827A, 2827B) and configured to receive at least a portion (2831, 2833) of the frame (2826, 2826', 2836), wherein a cover portion (2809A, 2909B, 2809C, 2809D, 2829A, 2929B, 2829C, 2829D) of the frame (2826, 2826', 2836) occludes the third portion (2825A, 2825B, 2855A, 2855B) from contacting the skin of the user; and
an end portion (2849A, 2849B, 2859A, 2859B) adjacent to the first portion (2863, 2865) and extending away from the first portion (2863, 2865) at an angle; and
a substrate (2818, 2821) in electrical connection with the electrode (354, 355, 124, 125, 2807A, 2807B, 2807A', 2807B', 2827A, 2827B) and responsive to the electrical signals originating from the user.

2. The wearable device of the preceding claim, wherein the end portion (2849A, 2849B, 2859A, 2859B) is enclosed by the frame (2826, 2826', 2836).

3. The wearable device (110A, 110B, 110C, 350, 2810, 2810', 10) of any of the preceding claims, wherein the frame (2826, 2826', 2836) occludes the end portion (2849A, 2849B, 2859A, 2859B) from contacting the skin of the user.

4. The wearable device (110A, 110B, 110C, 350, 2810, 2810', 10) of any of the preceding claims, wherein a surface (2841) of the end portion (2849A, 2849B, 2859A, 2859B) does not contact the skin of the user.

5. The wearable device (110A, 110B, 110C, 350, 2810, 2810', 10) of any of the preceding claims, wherein the end portion (2849A, 2849B, 2859A, 2859B) is substantially orthogonal to the first portion (2863, 2865).

6. The wearable device (110A, 110B, 110C, 350, 2810, 2810', 10) of any of the preceding claims, wherein the end portion (2849A, 2849B, 2859A, 2859B) comprises a through-opening (2848A, 2848B, 2848C, 2848D) extending through the end portion (2849A, 2849B, 2859A, 2859B), the through-opening (2848A, 2848B, 2848C, 2848D) configured to receive a protrusion (2844A, 2844B, 2844C, 2844D) of the frame (2826, 2826', 2836) to secure the electrode (354, 355, 124, 125, 2807A, 2807B, 2807A', 2807B', 2827A, 2827B) to the frame (2826, 2826', 2836).

7. The wearable device (110A, 110B, 110C, 350, 2810, 2810', 10) of any of the preceding claims, further comprising a fourth portion (2825A, 2825B, 2855A, 2855B) comprising:
a surface (2841) that is continuous with the surface (2841) of the second portion (2864), wherein the surface (2841) of the fourth portion (2825A, 2825B, 2855A, 2855B extends away from the surface (2841) of the second portion (2864)and is separated from the skin of the user when the second portion (2864)contacts the skin of the user; and
a second through-hole (2843A, 2843B, 2853A, 2853B) extending through the electrode (354, 355, 124, 125, 2807A, 2807B, 2807A', 2807B', 2827A, 2827B) and configured to receive an electrically conductive material configured to conduct the electrical signals originating from the user to the substrate (2818, 2821).

8. The wearable device (110A, 110B, 110C, 350, 2810, 2810', 10) of the preceding claim, wherein a second cover portion (2809A, 2909B, 2809C, 2809D, 2829A, 2929B, 2829C, 2829D) of the frame (2826, 2826', 2836) occludes the fourth portion (2825A, 2825B, 2855A, 2855B) from contacting the skin of the user.

9. The wearable device (110A, 110B, 110C, 350, 2810, 2810', 10) of any of the preceding claims, further comprising:
a fifth portion (2863, 2865) comprising a surface (2841) that is continuous with the surface (2841) of the fourth portion (2825A, 2825B, 2855A, 2855B) and configured to contact the skin of the user; and
another end portion (2849A, 2849B, 2859A, 2859B) adjacent to the fifth portion (2863, 2865), the another end portion (2849A, 2849B, 2859A, 2859B) having a surface (2841) that is continuous with the surface (2841) of the fifth portion (2863, 2865), the another end portion (2849A, 2849B, 2859A, 2859B) extending from the fifth portion (2863, 2865) at an angle.

10. The wearable device (110A, 110B, 110C, 350, 2810, 2810', 10) of the preceding claim, wherein the another end portion (2849A, 2849B, 2859A, 2859B) comprises another through-opening (2848A, 2848B, 2848C, 2848D) extending through the another end portion (2849A, 2849B, 2859A, 2859B), the another through-opening (2848A, 2848B, 2848C, 2848D) configured to receive another protrusion (2844A, 2844B, 2844C, 2844D) of the frame (2826, 2826', 2836) to secure the electrode (354, 355, 124, 125, 2807A, 2807B, 2807A', 2807B', 2827A, 2827B) to the frame (2826, 2826', 2836).

11. The wearable device (110A, 110B, 110C, 350, 2810, 2810', 10) of any of the preceding claims, wherein the first portion (2863, 2865) is substantially semi-annular.

12. The wearable device (110A, 110B, 110C, 350, 2810, 2810', 10) of any of the preceding claims, wherein the first portion (2863, 2865) and the second portion (2864) form at least a portion of a semi-annulus.

13. The wearable device (110A, 110B, 110C, 350, 2810, 2810', 10) of any of the preceding claims, wherein the surface (2841) of the third portion (2825A, 2825B, 2855A, 2855B) is continuous with the surface (2841) of the first portion (2863, 2865) and the surface (2841) of the second portion (2864).

14. The wearable device (110A, 110B, 110C, 350, 2810, 2810', 10) of any of the preceding claims, wherein the end portion (2849A, 2849B, 2859A, 2859B) comprises a surface (2841) that is continuous with the surface (2841) of the first portion (2863, 2865).

15. The wearable device (110A, 110B, 110C, 350, 2810, 2810', 10) of any of the preceding claims, further comprising a hardware processor (348, 364, 14, 108, 1402, 1080, 1082) coupled to the substrate (2818, 2821) and configured to access the electrical signals conducted via the electrode (354, 355, 124, 125, 2807A, 2807B, 2807A', 2807B', 2827A, 2827B) and perform one or more electrocardiography techniques with the electrical signals conducted via the electrode (354, 355, 124, 125, 2807A, 2807B, 2807A', 2807B', 2827A, 2827B).

## Patentansprüche

1. Tragbare Vorrichtung (110A, 110B, 110C, 350, 2810, 2810', 10), die dafür konfiguriert ist, physiologische Messungen auszuführen, wobei die tragbare Vorrichtung (110A, 110B, 110C, 350, 2810, 2810', 10) aufweist:
einen Rahmen (2826, 2826', 2836);
eine Elektrode (354, 355, 124, 125, 2807A, 2807B, 2807A', 2807B', 2827A, 2827B), die am Rahmen (2826, 2826', 2836) befestigt und dafür konfiguriert ist, elektrische Signale, die von einem Benutzer der tragbaren Vorrichtung (110A, 110B, 110C, 350, 2810, 2810', 10) ausgehen, zu leiten, wobei die Elektrode (354, 355, 124, 125, 2807A, 2807B, 2807A', 2807B', 2827A, 2827B) aufweist:
einen ersten Abschnitt (2863, 2865) mit einer Oberfläche (2841), die dafür konfiguriert ist, mit Haut des Benutzers in Kontakt zu kommen;
einen zweiten Abschnitt (2864) mit einer Oberfläche (2841), die dafür konfiguriert ist, mit der Haut des Benutzers in Kontakt zu kommen;
einen zwischen dem ersten Abschnitt (2863, 2865) und dem zweiten Abschnitt (2864) angeordneten dritten Abschnitt (2825A, 2825B, 2855A, 2855B), wobei der dritte Abschnitt (2825A, 2825B, 2855A, 2855B) aufweist:
eine Oberfläche (2841), die sich von der Oberfläche (2841) des ersten Abschnitts (2863, 2865) und der Oberfläche (2841) des zweiten Abschnitts (2864) weg erstreckt und von der Haut des Benutzers getrennt ist, wenn der erste Abschnitt (2863, 2865) oder der zweite Abschnitt (2864) mit der Haut des Benutzers in Kontakt steht; und
ein Durchgangsloch (2843A, 2843B, 2853A, 2853B), das sich durch die Elektrode (354, 355, 124, 125, 2807A, 2807B, 2807A', 2807B', 2827A, 2827B) erstreckt und dafür konfiguriert ist, mindestens einen Abschnitt (2831, 2833) des Rahmens (2826, 2826', 2836) aufzunehmen, wobei ein Abdeckabschnitt (2809A, 2909B, 2809C, 2809D, 2829A, 2929B, 2829C, 2829D) des Rahmens (2826, 2826', 2836) den dritten Abschnitt (2825A, 2825B, 2855A, 2855B) daran hindert, mit der Haut des Benutzers in Kontakt zu kommen; und
einen Endabschnitt (2849A, 2849B, 2859A, 2859B), der dem ersten Abschnitt (2863, 2865) benachbart ist und sich in einem Winkel vom ersten Abschnitt (2863, 2865) weg erstreckt; und
ein Substrat (2818, 2821), das mit der Elektrode (354, 355, 124, 125, 2807A, 2807B, 2807A', 2807B', 2827A, 2827B) elektrisch verbunden ist und auf die vom Benutzer ausgehenden elektrischen Signale anspricht.

2. Tragbare Vorrichtung nach dem vorhergehenden Anspruch, wobei der Endabschnitt (2849A, 2849B, 2859A, 2859B) vom Rahmen (2826, 2826', 2836) umschlossen ist.

3. Tragbare Vorrichtung (110A, 110B, 110C, 350, 2810, 2810', 10) nach einem der vorhergehenden Ansprüche, wobei der Rahmen (2826, 2826', 2836) verhindert, dass der Endabschnitt (2849A, 2849B, 2859A, 2859B) mit der Haut des Benutzers in Kontakt kommt.

4. Tragbare Vorrichtung (110A, 110B, 110C, 350, 2810, 2810', 10) nach einem der vorhergehenden Ansprüche, wobei eine Oberfläche (2841) des Endabschnitts (2849A, 2849B, 2859A, 2859B) nicht mit der Haut des Benutzers in Kontakt kommt.

5. Tragbare Vorrichtung (110A, 110B, 110C, 350, 2810, 2810', 10) nach einem der vorhergehenden Ansprüche, wobei der Endabschnitt (2849A, 2849B, 2859A, 2859B) sich im Wesentlichen orthogonal zum ersten Abschnitt (2863, 2865) erstreckt.

6. Tragbare Vorrichtung (110A, 110B, 110C, 350, 2810, 2810', 10) nach einem der vorhergehenden Ansprüche, wobei der Endabschnitt (2849A, 2849B, 2859A, 2859B) eine Durchgangsöffnung (2848A, 2848B, 2848C, 2848D) aufweist, die sich durch den Endabschnitt (2849A, 2849B, 2859A, 2859B) erstreckt, wobei die Durchgangsöffnung (2848A, 2848B, 2848C, 2848D) dafür konfiguriert ist, einen Vorsprung (2844A, 2844B, 2844C, 2844D) des Rahmens (2826, 2826', 2836) aufzunehmen, um die Elektrode (354, 355, 124, 125, 2807A, 2807B, 2807A', 2807B', 2827A, 2827B) am Rahmen (2826, 2826', 2836) zu befestigen.

7. Tragbare Vorrichtung (110A, 110B, 110C, 350, 2810, 2810', 10) nach einem der vorhergehenden Ansprüche, ferner aufweisend einen vierten Abschnitt (2825A, 2825B, 2855A, 2855B), der aufweist:
eine Oberfläche (2841), die nahtlos an die Oberfläche (2841) des zweiten Abschnitts (2864) anschließt, wobei die Oberfläche (2841) des vierten Abschnitts (2825A, 2825B, 2855A, 2855B) sich von der Oberfläche (2841) des zweiten Abschnitts (2864) weg erstreckt und von der Haut des Benutzers getrennt ist, wenn der zweite Abschnitt (2864) mit der Haut des Benutzers in Kontakt steht; und
ein zweites Durchgangsloch (2843A, 2843B, 2853A, 2853B), das sich durch die Elektrode (354, 355, 124, 125, 2807A, 2807B, 2807A', 2807B', 2827A, 2827B) erstreckt und dafür konfiguriert ist, ein elektrisch leitfähiges Material aufzunehmen, das dafür konfiguriert ist, die vom Benutzer ausgehenden elektrischen Signale zum Substrat (2818, 2821) zu leiten.

8. Tragbare Vorrichtung (110A, 110B, 110C, 350, 2810, 2810', 10) nach dem vorhergehenden Anspruch, wobei ein zweiter Abdeckabschnitt (2809A, 2909B, 2809C, 2809D, 2829A, 2929B, 2829C, 2829D) des Rahmens (2826, 2826', 2836) verhindert, dass der vierte Abschnitt (2825A, 2825B, 2855A, 2855B) mit der Haut des Benutzers in Kontakt kommt.

9. Tragbare Vorrichtung (110A, 110B, 110C, 350, 2810, 2810', 10) nach einem der vorhergehenden Ansprüche, ferner aufweisend:
einen fünften Abschnitt (2863, 2865), der eine Oberfläche (2841) aufweist, die nahtlos an die Oberfläche (2841) des vierten Abschnitts (2825A, 2825B, 2855A, 2855B) anschließt und dafür konfiguriert ist, mit der Haut des Benutzers in Kontakt zu kommen; und
einen dem fünften Abschnitt (2863, 2865) benachbarten weiteren Endabschnitt (2849A, 2849B, 2859A, 2859B), wobei der weitere Endabschnitt (2849A, 2849B, 2859A, 2859B) eine Oberfläche (2841) aufweist, die nahtlos an die Oberfläche (2841) des fünften Abschnitts (2863, 2865) anschließt, wobei der weitere Endabschnitt (2849A, 2849B, 2859A, 2859B) sich unter einem Winkel vom fünften Abschnitt (2863, 2865) aus erstreckt.

10. Tragbare Vorrichtung (110A, 110B, 110C, 350, 2810, 2810', 10) nach dem vorhergehenden Anspruch, wobei der weitere Endabschnitt (2849A, 2849B, 2859A, 2859B) eine weitere Durchgangsöffnung (2848A, 2848B, 2848C, 2848D) aufweist, die sich durch den weiteren Endabschnitt (2849A, 2849B, 2859A, 2859B) erstreckt, wobei die weitere Durchgangsöffnung (2848A, 2848B, 2848C, 2848D) dafür konfiguriert ist, einen weiteren Vorsprung (2844A, 2844B, 2844C, 2844D) des Rahmens (2826, 2826', 2836) aufzunehmen, um die Elektrode (354, 355, 124, 125, 2807A, 2807B, 2807A', 2807B', 2827A, 2827B) am Rahmen (2826, 2826', 2836) zu befestigen.

11. Tragbare Vorrichtung (110A, 110B, 110C, 350, 2810, 2810', 10) nach einem der vorhergehenden Ansprüche, wobei der erste Abschnitt (2863, 2865) im Wesentlichen halbringförmig ist.

12. Tragbare Vorrichtung (110A, 110B, 110C, 350, 2810, 2810', 10) nach einem der vorhergehenden Ansprüche, wobei der erste Abschnitt (2863, 2865) und der zweite Abschnitt (2864) zumindest einen Teil eines Halbkreises bilden.

13. Tragbare Vorrichtung (110A, 110B, 110C, 350, 2810, 2810', 10) nach einem der vorhergehenden Ansprüche, wobei die Oberfläche (2841) des dritten Abschnitts (2825A, 2825B, 2855A, 2855B) nahtlos an die Oberfläche (2841) des ersten Abschnitts (2863, 2865) und die Oberfläche (2841) des zweiten Abschnitts (2864) anschließt.

14. Tragbare Vorrichtung (110A, 110B, 110C, 350, 2810, 2810', 10) nach einem der vorhergehenden Ansprüche, wobei der Endabschnitt (2849A, 2849B, 2859A, 2859B) eine Oberfläche (2841) aufweist, die nahtlos an die Oberfläche (2841) des ersten Abschnitts (2863, 2865) anschließt.

15. Tragbare Vorrichtung (110A, 110B, 110C, 350, 2810, 2810', 10) nach einem der vorhergehenden Ansprüche, ferner aufweisend einen Hardware-Prozessor (348, 364, 14, 108, 1402, 1080, 1082), der mit dem Substrat (2818, 2821) verbunden und dafür konfiguriert ist, auf die über die Elektrode (354, 355, 124, 125, 2807A, 2807B, 2807A', 2807B', 2827A, 2827B) geleiteten elektrischen Signale zuzugreifen und eine oder mehrere Elektrokardiographietechniken bezüglich den über die Elektrode (354, 355, 124, 125, 2807A, 2807B, 2807A', 2807B', 2827A, 2827B) geleiteten elektrischen Signalen auszuführen.

## Revendications

1. Dispositif vestimentaire (110A, 110B, 110C, 350, 2810, 2810', 10) configuré pour réaliser des mesures physiologiques, le dispositif vestimentaire (110A, 110B, 110C, 350, 2810, 2810', 10) comprenant :
un cadre (2826, 2826', 2836) ;
une électrode (354, 355, 124, 125, 2807A, 2807B, 2807A', 2807B', 2827A, 2827B) fixée au cadre (2826, 2826', 2836) et configurée pour conduire des signaux électriques provenant d'un utilisateur du dispositif vestimentaire (110A, 110B, 110C, 350, 2810, 2810', 10), l'électrode (354, 355, 124, 125, 2807A, 2807B, 2807A', 2807B', 2827A, 2827B) comprenant :
une première partie (2863, 2865) ayant une surface (2841) configurée pour entrer en contact avec une peau de l'utilisateur ;
une deuxième partie (2864) ayant une surface (2841) configurée pour entrer en contact avec la peau de l'utilisateur ;
une troisième partie (2825A, 2825B, 2855A, 2855B) disposée entre la première partie (2863, 2865) et la deuxième partie (2864), la troisième partie (2825A, 2825B, 2855A, 2855B) comprenant :
une surface (2841) qui s'étend dans le sens opposé à la surface (2841) de la première partie (2863, 2865) et à la surface (2841) de la deuxième partie (2864) et est séparée de la peau de l'utilisateur lorsque la première partie (2863, 2865) ou la deuxième partie (2864) entre en contact avec la peau de l'utilisateur ; et
un orifice traversant (2843A, 2843B, 2853A, 2853B) s'étendant à travers l'électrode (354, 355, 124, 125, 2807A, 2807B, 2807A', 2807B', 2827A, 2827B) et configuré pour recevoir au moins une partie (2831, 2833) du cadre (2826, 2826', 2836), dans lequel une partie de couverture (2809A, 2909B, 2809C, 2809D, 2829A, 2929B, 2829C, 2829D) du cadre (2826, 2826', 2836) empêche la troisième partie (2825A, 2825B, 2855A, 2855B) d'entrer en contact avec la peau de l'utilisateur ; et
une partie d'extrémité (2849A, 2849B, 2859A, 2859B) adjacente à la première partie (2863, 2865) et s'étendant dans le sens opposé à la première partie (2863, 2865) avec un angle ; et
un substrat (2818, 2821) en connexion électrique avec l'électrode (354, 355, 124, 125, 2807A, 2807B, 2807A', 2807B', 2827A, 2827B) et sensible aux signaux électriques provenant de l'utilisateur.

2. Dispositif vestimentaire selon la revendication précédente, dans lequel la partie d'extrémité (2849A, 2849B, 2859A, 2859B) est renfermée par le cadre (2826, 2826', 2836).

3. Dispositif vestimentaire (110A, 110B, 110C, 350, 2810, 2810', 10) selon l'une quelconque des revendications précédentes, dans lequel le cadre (2826, 2826', 2836) empêche la partie d'extrémité (2849A, 2849B, 2859A, 2859B) d'entrer en contact avec la peau de l'utilisateur.

4. Dispositif vestimentaire (110A, 110B, 110C, 350, 2810, 2810', 10) selon l'une quelconque des revendications précédentes, dans lequel une surface (2841) de la partie d'extrémité (2849A, 2849B, 2859A, 2859B) n'entre pas en contact avec la peau de l'utilisateur.

5. Dispositif vestimentaire (110A, 110B, 110C, 350, 2810, 2810', 10) selon l'une quelconque des revendications précédentes, dans lequel la partie d'extrémité (2849A, 2849B, 2859A, 2859B) est essentiellement orthogonale à la première partie (2863, 2865).

6. Dispositif vestimentaire (110A, 110B, 110C, 350, 2810, 2810', 10) selon l'une quelconque des revendications précédentes, dans lequel la partie d'extrémité (2849A, 2849B, 2859A, 2859B) comprend une ouverture traversante (2848A, 2848B, 2848C, 2848D) s'étendant à travers la partie d'extrémité (2849A, 2849B, 2859A, 2859B), l'ouverture traversante (2848A, 2848B, 2848C, 2848D) étant configurée pour recevoir une saillie (2844A, 2844B, 2844C, 2844D) du cadre (2826, 2826', 2836) pour fixer l'électrode (354, 355, 124, 125, 2807A, 2807B, 2807A', 2807B', 2827A, 2827B) au cadre (2826, 2826', 2836).

7. Dispositif vestimentaire (110A, 110B, 110C, 350, 2810, 2810', 10) selon l'une quelconque des revendications précédentes, comprenant en outre une quatrième partie (2825A, 2825B, 2855A, 2855B) comprenant :
une surface (2841) qui est continue vis-à-vis de la surface (2841) de la deuxième partie (2864), dans lequel la surface (2841) de la quatrième partie (2825A, 2825B, 2855A, 2855B) s'étend dans le sens opposé à la surface (2841) de la deuxième partie (2864) et est séparée de la peau de l'utilisateur lorsque la deuxième partie (2864) entre en contact avec la peau de l'utilisateur ; et
un second orifice traversant (2843A, 2843B, 2853A, 2853B) s'étendant à travers l'électrode (354, 355, 124, 125, 2807A, 2807B, 2807A', 2807B', 2827A, 2827B) et configuré pour recevoir un matériau électriquement conducteur configuré pour conduire les signaux électriques provenant de l'utilisateur au substrat (2818, 2821).

8. Dispositif vestimentaire (110A, 110B, 110C, 350, 2810, 2810', 10) selon la revendication précédente, dans lequel une seconde partie de couverture (2809A, 2909B, 2809C, 2809D, 2829A, 2929B, 2829C, 2829D) du cadre (2826, 2826', 2836) empêche la quatrième partie (2825A, 2825B, 2855A, 2855B) d'entrer en contact avec la peau de l'utilisateur.

9. Dispositif vestimentaire (110A, 110B, 110C, 350, 2810, 2810', 10) selon l'une quelconque des revendications précédentes, comprenant en outre :
une cinquième partie (2863, 2865) comprenant une surface (2841) qui est continue vis-à-vis de la surface (2841) de la quatrième partie (2825A, 2825B, 2855A, 2855B) et configurée pour entrer en contact avec la peau de l'utilisateur ; et
une autre partie d'extrémité (2849A, 2849B, 2859A, 2859B) adjacente à la cinquième partie (2863, 2865), l'autre partie d'extrémité (2849A, 2849B, 2859A, 2859B) ayant une surface (2841) qui est continue vis-à-vis de la surface (2841) de la cinquième partie (2863, 2865), l'autre partie d'extrémité (2849A, 2849B, 2859A, 2859B) s'étendant depuis la cinquième partie (2863, 2865) avec un angle.

10. Dispositif vestimentaire (110A, 110B, 110C, 350, 2810, 2810', 10) selon la revendication précédente, dans lequel l'autre partie d'extrémité (2849A, 2849B, 2859A, 2859B) comprend une autre ouverture traversante (2848A, 2848B, 2848C, 2848D) s'étendant à travers l'autre partie d'extrémité (2849A, 2849B, 2859A, 2859B), l'autre ouverture traversante (2848A, 2848B, 2848C, 2848D) étant configurée pour recevoir une autre saillie (2844A, 2844B, 2844C, 2844D) du cadre (2826, 2826', 2836) pour fixer l'électrode (354, 355, 124, 125, 2807A, 2807B, 2807A', 2807B', 2827A, 2827B) au cadre (2826, 2826', 2836).

11. Dispositif vestimentaire (110A, 110B, 110C, 350, 2810, 2810', 10) selon l'une quelconque des revendications précédentes, dans lequel la première partie (2863, 2865) est essentiellement semi-annulaire.

12. Dispositif vestimentaire (110A, 110B, 110C, 350, 2810, 2810', 10) selon l'une quelconque des revendications précédentes, dans lequel la première partie (2863, 2865) et la deuxième partie (2864) forment au moins une partie d'un semi-anneau.

13. Dispositif vestimentaire (110A, 110B, 110C, 350, 2810, 2810', 10) selon l'une quelconque des revendications précédentes, dans lequel la surface (2841) de la troisième partie (2825A, 2825B, 2855A, 2855B) est continue vis-à-vis de la surface (2841) de la première partie (2863, 2865) et de la surface (2841) de la deuxième partie (2864).

14. Dispositif vestimentaire (110A, 110B, 110C, 350, 2810, 2810', 10) selon l'une quelconque des revendications précédentes, dans lequel la partie d'extrémité (2849A, 2849B, 2859A, 2859B) comprend une surface (2841) qui est continue vis-à-vis de la surface (2841) de la première partie (2863, 2865).

15. Dispositif vestimentaire (110A, 110B, 110C, 350, 2810, 2810', 10) selon l'une quelconque des revendications précédentes, comprenant en outre un processeur matériel (348, 364, 14, 108, 1402, 1080, 1082) couplé au substrat (2818, 2821) et configuré pour accéder aux signaux électriques conduits par l'intermédiaire de l'électrode (354, 355, 124, 125, 2807A, 2807B, 2807A', 2807B', 2827A, 2827B) et réaliser une ou plusieurs techniques d'électrocardiographie avec les signaux électriques conduits par l'intermédiaire de l'électrode (354, 355, 124, 125, 2807A, 2807B, 2807A', 2807B', 2827A, 2827B).
